(19) Europäisches Patentamt
European Patent Office
Office européen des brevets

(11) EP 4 324 822 A1

(12) EUROPEAN PATENT APPLICATION
published in accordance with Art. 153(4) EPC

(43) Date of publication:
21.02.2024 Bulletin 2024/08

(21) Application number: 22788126.5

(22) Date of filing: 08.04.2022

(51) International Patent Classification (IPC):
*C07C 309/06* (2006.01) *C07C 309/12* (2006.01)
*C07C 309/17* (2006.01) *C07C 309/40* (2006.01)
*C07C 309/42* (2006.01) *C07C 311/53* (2006.01)
*C07C 381/12* (2006.01) *C07D 309/12* (2006.01)
*C09K 3/00* (2006.01) *C07D 493/18* (2006.01)
*G03F 7/004* (2006.01) *G03F 7/038* (2006.01)
*G03F 7/039* (2006.01) *G03F 7/20* (2006.01)
*C07D 307/00* (2006.01) *C07D 307/12* (2006.01)

(52) Cooperative Patent Classification (CPC):
C07C 309/06; C07C 309/12; C07C 309/17; C07C 309/40; C07C 309/42; C07C 311/53; C07C 381/12; C07D 307/00; C07D 307/12; C07D 309/12; C07D 493/18; C09K 3/00; G03F 7/004; G03F 7/038; G03F 7/039; (Cont.)

(86) International application number:
PCT/JP2022/017419

(87) International publication number:
WO 2022/220201 (20.10.2022 Gazette 2022/42)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 16.04.2021 JP 2021070036

(71) Applicant: FUJIFILM Corporation
Tokyo 106-8620 (JP)

(72) Inventors:
- BEKKI Yosuke
  Haibara-gun, Shizuoka 421-0396 (JP)
- KOJIMA Masafumi
  Haibara-gun, Shizuoka 421-0396 (JP)
- GOTO Akiyoshi
  Haibara-gun, Shizuoka 421-0396 (JP)
- FUJIMAKI Nishiki
  Haibara-gun, Shizuoka 421-0396 (JP)

(74) Representative: HGF
HGF Limited
1 City Walk
Leeds LS11 9DX (GB)

# (54) ACTIVE LIGHT-SENSITIVE OR RADIATION-SENSITIVE RESIN COMPOSITION, RESIST FILM, PATTERN FORMING METHOD, METHOD FOR PRODUCING ELECTRONIC DEVICE, AND COMPOUND

(57) Provided are an actinic ray-sensitive or radiation-sensitive resin composition including a compound represented by general formula (S1) and an acid decomposable resin, a resist film formed using the actinic ray-sensitive or radiation-sensitive resin composition, a pattern forming method, and an electronic device manufacturing method. Also provided are an actinic ray-sensitive or radiation-sensitive resin composition that includes the compound represented by general formula (S1) and allows patterns with good shapes to be obtained when the composition is used for pattern formation, a resist film formed using the actinic ray-sensitive or radiation-sensitive resin composition, a pattern forming method, and an electronic device manufacturing method. Also provided is a compound that can be used for the actinic ray-sensitive or radiation-sensitive resin composition.

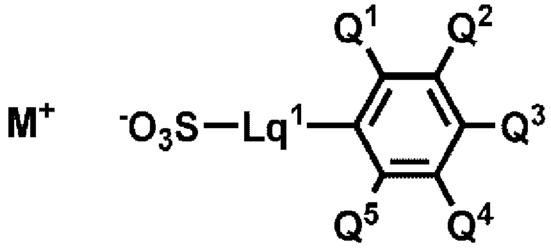

(S1)

$Q^1$ to $Q^5$ each independently represent a hydrogen atom or a substituent, provided that at least one of $Q^1$, $Q^2$, $Q^3$, $Q^4$, or $Q^5$ represents a substituent including an aryloxy group represented by general formula (QR1). $Lq^1$ represents a single bond or a divalent linking group. $M^+$ represents a cation.

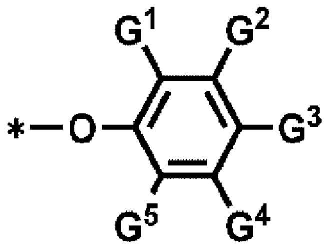

(QR1)

$G^1$ to $G^5$ each independently represent a hydrogen atom or a substituent, provided that at least one of $G^1$, $G^2$, $G^3$, $G^4$, or $G^5$ represents a substituent including an ester group. * represents a bonding position.

(52) Cooperative Patent Classification (CPC): (Cont.)
**G03F 7/20**

## Description

## BACKGROUND OF THE INVENTION

1. Field of the Invention

The present invention relates to an actinic ray-sensitive or radiation-sensitive resin composition, a resist film, a pattern forming method, a method for manufacturing an electronic device, and a compound.

2. Description of the Related Art

Conventional manufacturing processes for semiconductor devices such as ICs (Integrated Circuits) or LSI (Large Scale Integration) circuits involve lithographic microfabrication using an actinic ray-sensitive or radiation-sensitive resin composition (typically, a resist composition). In recent years, the degree of integration of integrated circuits has increased, and there is a growing demand for ultra-fine pattern formation in the submicron or quarter-micron range. Accordingly, the wavelength of exposure light tends to be shortened. Specifically, the g-line is replaced by the i-line and then by KrF excimer laser light. At present, exposure devices using an ArF excimer laser with a wavelength of 193 nm as the light source are being developed. In addition, techniques for further increasing resolving power are under development. Specifically, the so-called immersion method is under development, in which the space between a projection lens and a specimen is filled with a high-refractive index liquid (hereinafter referred to as "immersion liquid").

At present, in addition to the use of excimer laser light, lithography using electron beams (EB), X-rays, extreme ultraviolet rays (EUV), etc. is being developed. This leads to the development of resist compositions that are effectively sensitive to various types of actinic rays and radiation.

Various compounds are known as photoacid generators used for actinic ray-sensitive or radiation-sensitive resin compositions. For example, JP2013-160955A and JP2004-109976A describe compounds that decompose upon irradiation with actinic rays or radiation to generate acids having specific structures.

## SUMMARY OF THE INVENTION

However, the inventors have conducted studies and found that, when actinic ray-sensitive or radiation-sensitive resin compositions including the photoacid generators described in JP2013-160955A and JP2004-109976A are used to form, in particular, ultrafine patterns (having a line width of, for example, 20 nm or less), there is room for further improvement in the shapes of the patterns obtained.

It is an object of the invention to provide an actinic ray-sensitive or radiation-sensitive resin composition that allows patterns with good shapes to be obtained when the composition is used for pattern formation and to provide a resist film formed using the actinic ray-sensitive or radiation-sensitive resin composition, a pattern forming method, a method for manufacturing an electronic device, and a compound that can be used for the actinic ray-sensitive or radiation-sensitive resin composition.

The inventors have conducted extensive studies and found that the above object can be achieved by the following.

[1] An actinic ray-sensitive or radiation-sensitive resin composition including: a compound represented by the following general formula (S1); and an acid decomposable resin.

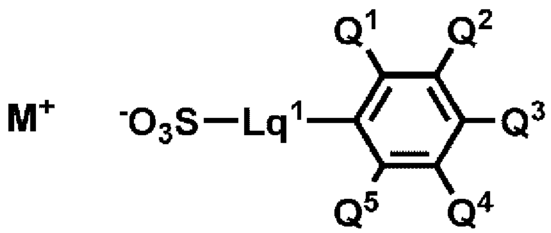

(S1)

In general formula (S1), $Q^1$, $Q^2$, $Q^3$, $Q^4$, and $Q^5$ each independently represent a hydrogen atom or a substituent, provided that at least one of $Q^1$, $Q^2$, $Q^3$, $Q^4$, or $Q^5$ represents a substituent including an aryloxy group represented by general formula (QR1) below. $Lq^1$ represents a single bond or a divalent linking group. $M^+$ represents an organic cation.

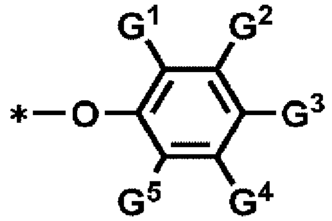

(QR1)

In general formula (QR1), $G^1$, $G^2$, $G^3$, $G^4$, and $G^5$ each independently represent a hydrogen atom or a substituent, provided that at least one of $G^1$, $G^2$, $G^3$, $G^4$, or $G^5$ represents a substituent including an ester group. * represents a bonding position.

[2] The actinic ray-sensitive or radiation-sensitive resin composition according to [1], wherein at least one of $Q^1$, $Q^2$, $Q^3$, $Q^4$, or $Q^5$ in general formula (S1) above represents an electron-withdrawing group.

[3] The actinic ray-sensitive or radiation-sensitive resin composition according to [1] or [2], wherein at least one of $Q^1$, $Q^2$, $Q^3$, $Q^4$, or $Q^5$ in general formula (S1) above represents a fluorine atom or a monovalent fluorinated hydrocarbon group.

[4] The actinic ray-sensitive or radiation-sensitive resin composition according to any one of [1] to [3], wherein $Lq^1$ in general formula (S1) above represents a single bond.

[5] The actinic ray-sensitive or radiation-sensitive resin composition according to any one of [1] to [4], wherein at least one of $Q^1$, $Q^2$, $Q^3$, $Q^4$, or $Q^5$ in general formula (S1) above represents the aryloxy group represented by general formula (QR1) above.

[6] The actinic ray-sensitive or radiation-sensitive resin composition according to [5], wherein $Q^3$ in general formula (S1) above represents the aryloxy group represented by general formula (QR1) above.

[7] The actinic ray-sensitive or radiation-sensitive resin composition according to any one of [1] to [6], wherein at least two selected from the group consisting of $G^1$, $G^2$, $G^3$, $G^4$, and $G^5$ in general formula (QR1) each represent the substituent including an ester group.

[8] The actinic ray-sensitive or radiation-sensitive resin composition according to any one of [1] to [7], wherein the substituent including an ester group is a group represented by general formula (GR1) or (GR2) below.

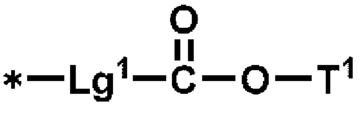

(GR1)

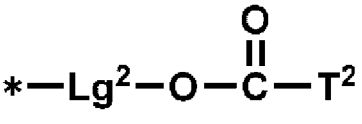

(GR2)

In general formulas (GR1) and (GR2), $Lg^1$ and $Lg^2$ each independently represent a single bond or a divalent linking group. $T^1$ and $T^2$ each independently represent an organic group. * represents a bonding position to a benzene ring in general formula (QR1).

[9] The actinic ray-sensitive or radiation-sensitive resin composition according to [8], wherein $Lg^1$ in general formula (GR1) above and $Lg^2$ in general formula (GR2) above each represent a single bond.

[10] The actinic ray-sensitive or radiation-sensitive resin composition according to [8] or [9], wherein $T^1$ in general formula (GR1) above and $T^2$ in general formula (GR2) above each independently represent an organic group having 1 to 20 carbon atoms.

[11] The actinic ray-sensitive or radiation-sensitive resin composition according to any one of [8] to [10], wherein $T^1$ in general formula (GR1) above and $T^2$ in general formula (GR2) above each independently represent a chain aliphatic group optionally including a heteroatom or a cyclic aliphatic group optionally including a heteroatom.

[12] A resist film formed using the actinic ray-sensitive or radiation-sensitive resin composition according to any one of [1] to [11].

[13] A pattern forming method using the resist film according to [12].

[14] A method for manufacturing an electronic device, the method including the pattern forming method according to [13].

[15] A compound represented by the following general formula (S1).

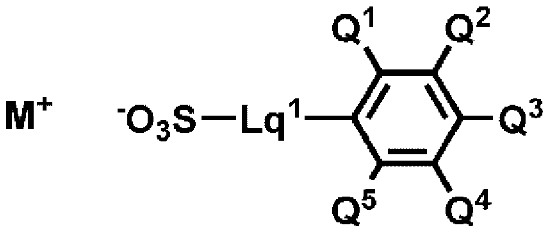

(S1)

In general formula (S1), $Q^1$, $Q^2$, $Q^3$, $Q^4$, and $Q^5$ each independently represent a hydrogen atom or a substituent, provided that at least one of $Q^1$, $Q^2$, $Q^3$, $Q^4$, or $Q^5$ represents a substituent including an aryloxy group represented by general formula (QR1) below. $Lq^1$ represents a single bond or a divalent linking group. $M^+$ represents an organic cation.

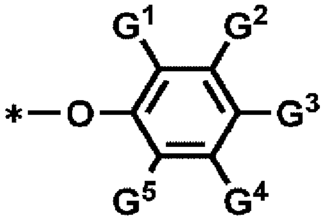

(QR1)

In general formula (QR1), $G^1$, $G^2$, $G^3$, $G^4$, and $G^5$ each independently represent a hydrogen atom or a substituent, provided that at least one of $G^1$, $G^2$, $G^3$, $G^4$, or $G^5$ represents a substituent including an ester group. * represents a bonding position.

The present invention can provide an actinic ray-sensitive or radiation-sensitive resin composition that allows patterns with good shapes to be obtained when the composition is used for pattern formation and to provide a resist film formed using the actinic ray-sensitive or radiation-sensitive resin composition, a pattern forming method, a method for manufacturing an electronic device, and a compound that can be used for the actinic ray-sensitive or radiation-sensitive resin composition.

## DESCRIPTION OF THE PREFERRED EMBODIMENTS

The present invention will next be described in detail.

Description of structural requirements described below may be made on the basis of representative embodiments of the present invention. However, the invention is not limited to theses embodiments.

As for notations of groups (atomic groups) in the present specification, a notation that is not specified as substituted and unsubstituted is intended to encompass groups having no substituent and groups having a substituent, so long as the notation does not depart from the spirit of the invention. For example, an "alkyl group" is intended to encompass not only an alkyl group having no substituent (an unsubstituted alkyl group) but also an alkyl group having a substituent (a substituted alkyl group). In the present specification, an "organic group" is a group including at least one carbon atom.

Preferably, the substituent is a monovalent substituent, unless otherwise specified.

In the present specification, "actinic rays" or "radiation" means, for example, an emission line spectrum of a

mercury lamp, far-ultraviolet rays typified by an excimer laser light, extreme ultraviolet light (EUV light), X-rays, an electron beam (EB), etc.

In the present specification, "light" means actinic rays or radiation.

In the present specification, "exposure to light" is intended to encompass not only exposure to an emission line spectrum of a mercury lamp, far-ultraviolet rays typified by an excimer laser light, extreme ultraviolet light, X-rays, EUV light, etc. but also image drawing using an electron beam or a particle beam such as an ion beam.

In the present specification, "to" is used to mean that numerical values before and after the "to" are used as the lower limit and the upper limit.

In the present specification, no limitation is imposed on the bonding direction of a divalent group, unless otherwise specified. For example, when Y in a compound represented by formula "X-Y-Z" is -COO-, Y may be -CO-O- or may be -O-CO-. This compound may be "X-CO-O-Z" or may be "X-O-CO-Z."

In the present specification, (meth)acrylate is intended to refer to acrylate and methacrylate, and (meth)acrylic is intended to refer to acrylic and methacrylic.

In the present specification, the weight average molecular weight (Mw), number average molecular weight (Mn), and dispersity (hereinafter may be referred to also as the "molecular weight distribution") (Mw/Mn) of a compound are defined as polystyrene-equivalent values determined by GPC (Gel Permeation Chromatography) measurement (solvent: tetrahydrofuran, flow rate (injection amount of a sample): 10 μL, columns: TSK gel Multipore HXL-M manufactured by TOSOH Corporation, column temperature: 40°C, flow velocity: 1.0 mL/minute, detector: differential refractive index detector) using a GPC apparatus (HLC-8120GPC manufactured by TOSOH Corporation).

In the present specification, the acid dissociation constant (pKa) is the pKa in an aqueous solution and is specifically a value determined by computation using the following software package 1 based on a Hammett substituent constant and a database of known literature values.

Software package 1: Advanced Chemistry Development (ACD/Labs) Software V 8.14 for Solaris (1994-2007 ACD/Labs).

The pKa can also be determined by a molecular orbital calculation method. In one specific example of this method, $H^+$ dissociation free energy in an aqueous solution is computed based on a thermodynamic cycle to compute the pKa. As for the method for computing the $H^+$ dissociation free energy, the density functional theory (DFT), for example, can be used for the computation. Various other methods have been reported in literature etc., but the computation method is not limited thereto. There are a plurality of software applications capable of performing the DFT, and one example is Gaussian 16.

In the present specification, the pKa is a value determined by computation using the software package 1 based on the Hammett substituent constant and the database of known literature values as described above. When the pKa cannot be computed using this method, a value obtained using Gaussian 16 based on the DFT (density functional theory) is used.

In the present specification, the pKa is a "value in an aqueous solution" as described above. When the pKa in an aqueous solution cannot be computed, the "pKa in a dimethyl sulfoxide (DMSO) solution" is used.

"Solids" are components forming a film (typically a resist film) formed using the actinic ray-sensitive or radiation-sensitive resin composition, and a solvent is not included. Any component included in the film is considered as a solid component even when the component is in a liquid form.

The actinic ray-sensitive or radiation-sensitive resin composition of the present invention (hereinafter may be referred to also as "the composition of the invention") will next be described in detail.

The actinic ray-sensitive or radiation-sensitive resin composition of the present invention is typically a resist composition and may be a positive-type resist composition or a negative-type resist composition. The composition of the invention may be a resist composition for alkali development and may be a resist composition for organic solvent development.

The resist composition is typically a chemical amplification-type resist composition.

First, various components of the actinic ray-sensitive or radiation-sensitive resin composition will be described in detail.

<Compound represented by general formula (S1)>

The composition of the invention includes a compound represented by general formula (S1).

Preferably, the compound represented by general formula (S1) is a compound that functions as a photoacid generator.

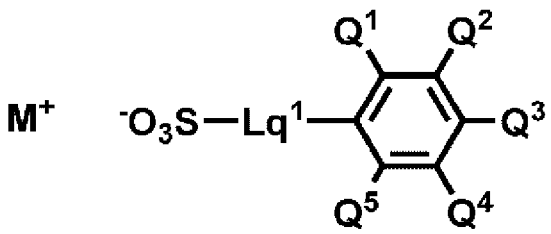

(S1)

In general formula (S1), $Q^1$, $Q^2$, $Q^3$, $Q^4$, and $Q^5$ each independently represent a hydrogen atom or a substituent, provide that at least one of $Q^1$, $Q^2$, $Q^3$, $Q^4$, or $Q^5$ represents a substituent including an aryloxy group represented by general formula (QR1) below. $Lq^1$ represents a single bond or a divalent linking group. $M^+$ represents an organic cation.

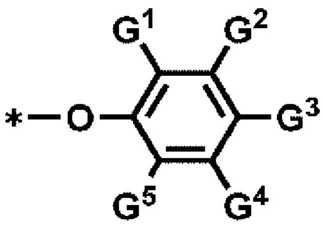

(QR1)

In general formula (QR1), $G^1$, $G^2$, $G^3$, $G^4$, and $G^5$ each independently represent a hydrogen atom or a substituent, provided that at least one of $G^1$, $G^2$, $G^3$, $G^4$, or $G^5$ represents a substituent including an ester group. * represents a bonding position.

In general formula (S1), when $Q^1$, $Q^2$, $Q^3$, $Q^4$, or $Q^5$ represents a substituent, no particular limitation is imposed on the substituent. Examples of the substituent include: halogen atoms such as a fluorine atom, a chlorine atom, a bromine atom, and an iodine atom; monovalent halogenated hydrocarbon groups (i.e., monovalent halogenated hydrocarbon groups having 1 to 20 carbon atoms) such as a trifluoromethyl group; alkoxy groups (e.g., alkoxy groups having 1 to 10 carbon atoms) such as a methoxy group, an ethoxy group, and a tert-butoxy group; aryloxy groups (e.g., aryloxy groups having 6 to 20 carbon atoms) such as a phenoxy group; alkoxycarbonyl groups (e.g., alkoxycarbonyl groups having 2 to 10 carbon atoms) such as a methoxycarbonyl group and a butoxycarbonyl group; aryloxycarbonyl groups (e.g., aryloxycarbonyl groups having 7 to 20 carbon atoms) such as a phenoxycarbonyl group; acyloxy groups (e.g., acyloxy groups having 2 to 20 carbon atoms) such as an acetoxy group, a propionyloxy group, and a benzoyloxy group; acyl groups (e.g., acyl groups having 2 to 20 carbon atoms) such as an acetyl group, a benzoyl group, an isobutyryl group, an acryloyl group, a methacryloyl group, and a methoxalyl group; alkyl groups (e.g., alkyl groups having 1 to 10 carbon atoms) such as a methyl group, an ethyl group, and a tert-butyl group; cycloalkyl groups (e.g., cycloalkyl groups having 3 to 15 carbon atoms; and an oxo group (=O) may be bonded to at least one carbon atom in the ring members) such as a cyclopentyl group, a cyclohexyl group, a norbornyl group, a tetracyclodecanyl group, a tetracyclododecanyl group, and an adamantyl group; aliphatic heterocyclic groups (e.g., aliphatic heterocyclic groups having 2 to 20 carbon atoms and including at least one heteroatom selected from the group consisting of a sulfur atom, an oxygen atom, and a nitrogen atom; an oxo group (=O) may be bonded to at least one atom in the ring members; and two atoms in the ring members may be linked together through at least one linking group selected from the group consisting of -O-, -CO-, -COO-, $-SO_2-$, and -NHCO-) such as a tetrahydropyranyl group; aryl groups (e.g., aryl groups having 6 to 20 carbon atoms); heteroaryl groups (e.g., heteroaryl groups having 2 to 20 carbon atoms and including at least one heteroatom selected from the group consisting of a sulfur atom, an oxygen atom, and a nitrogen atom); a hydroxy group; a carboxy group; a cyano group; a nitro group; an amino group; cyclic groups formed by fusing at least one selected from the group consisting of the above cycloalkyl groups and the above aliphatic heterocyclic groups and at least one selected from the group consisting of the above aryl groups and the above heteroaryl groups; and combinations of the above groups.

Preferably, at least one of $Q^1$, $Q^2$, $Q^3$, $Q^4$, or $Q^5$ in general formula (S1) represents an electron-withdrawing group. No particular limitation is imposed on the electron-withdrawing group. The electron-withdrawing group is, for example, preferably a halogen atom, a monovalent halogenated hydrocarbon group, a cyano group, a nitro group, a carboxy group, an alkoxycarbonyl group, an acyl group, etc., more preferably a fluorine atom, a chlorine atom, a monovalent fluorinated hydrocarbon group, a nitro group, or a cyano group, and still more preferably a fluorine atom or a monovalent fluorinated hydrocarbon group. The monovalent fluorinated hydrocarbon group is preferably a fluoroalkyl group and more preferably a perfluoroalkyl group. The number of carbon atoms in the monovalent fluorinated hydrocarbon group is preferably from 1 to 20 and more preferably from 1 to 10.

More preferably, at least two selected from the group consisting of $Q^1$, $Q^2$, $Q^3$, $Q^4$, and $Q^5$ in general formula

(S1) each represent an electron-withdrawing group. Still more preferably, at least three of them each represent an electron-withdrawing group. Particularly preferably, four of them each represent an electron-withdrawing group. Most preferably, $Q^1$, $Q^2$, $Q^4$, and $Q^5$ each represent an electron-withdrawing group.

When $Lq^1$ in general formula (S1) represents a divalent linking group, no particular limitation is imposed on the divalent linking group. Examples of the divalent linking group include -CO-, -O-, -S-, -SO-, $-SO_2-$, hydrocarbon groups (such as alkylene groups, cycloalkylene groups, alkenylene groups, and arylene groups), and linking groups formed by linking a plurality of groups selected from the above groups.

Preferably, $Lq^1$ in general formula (S1) represents a single bond.

Preferably, at least one of $Q^1$, $Q^2$, $Q^3$, $Q^4$, or $Q^5$ in general formula (S1) represents a substituent including the aryloxy group represented by general formula (QR1), and at least one of $Q^1$, $Q^2$, $Q^3$, $Q^4$, or $Q^5$ represents the aryloxy group represented by general formula (QR1). More preferably, $Q^3$ represents the aryloxy group represented by general formula (QR1).

When $G^1$, $G^2$, $G^3$, $G^4$, or $G^5$ in general formula (QR1) represents a substituent, no particular limitation is imposed on the substituent. Examples of the substituent include: halogen atoms such as a fluorine atom, a chlorine atom, a bromine atom, and an iodine atom; monovalent halogenated hydrocarbon groups (e.g., monovalent halogenated hydrocarbon groups having 1 to 20 carbon atoms) such as a trifluoromethyl group; alkoxy groups (e.g., alkoxy groups having 1 to 10 carbon atoms) such as a methoxy group, an ethoxy group, and a tert-butoxy group; aryloxy groups (e.g., aryloxy groups having 6 to 20 carbon atoms) such as a phenoxy group; acyl groups (e.g., acyl groups having 2 to 20 carbon atoms) such as an acetyl group, a benzoyl group, an isobutyryl group, an acryloyl group, a methacryloyl group, and a methoxalyl group; alkyl groups (e.g., alkyl groups having 1 to 10 carbon atoms) such as a methyl group, an ethyl group, and a tert-butyl group; cycloalkyl groups (e.g., cycloalkyl groups having 3 to 15 carbon atoms; and an oxo group (=O) may be bonded to at least one carbon atom in the ring members) such as a cyclopentyl group, a cyclohexyl group, a norbornyl group, a tetracyclodecanyl group, a tetracyclododecanyl group, and an adamantyl group; aliphatic heterocyclic groups (e.g., aliphatic heterocyclic groups having 2 to 20 carbon atoms and including at least one heteroatom selected from the group consisting of a sulfur atom, an oxygen atom, and a nitrogen atom; an oxo group (=O) may be bonded to at least one atom in the ring members; and two atoms in the ring members may be linked together through at least one linking group selected from the group consisting of -O-, -CO-, -COO-, $-SO_2-$, and -NHCO-) such as a tetrahydropyranyl group; aryl groups (e.g., aryl groups having 6 to 20 carbon atoms); heteroaryl groups (e.g., heteroaryl groups having 2 to 20 carbon atoms and including at least one heteroatom selected from the group consisting of a sulfur atom, an oxygen atom, and a nitrogen atom); a hydroxy group; a carboxy group; a cyano group; a nitro group; an amino group; cyclic groups formed by fusing at least one selected from the group consisting of the above cycloalkyl groups and the above aliphatic heterocyclic groups and at least one selected from the group consisting of the above aryl groups and the above heteroaryl groups; and combinations of the above groups.

At least one of $G^1$, $G^2$, $G^3$, $G^4$, or $G^5$ in general formula (QR1) represents the substituent including an ester group. Preferably, at least two selected from the group consisting of $G^1$, $G^2$, $G^3$, $G^4$, and $G^5$ each represent the substituent including an ester group.

Preferably, at least one of $G^2$, $G^3$, or $G^4$ in general formula (QR1) represents the substituent including an ester group. Moe preferably, at least two selected from the group consisting of $G^2$, $G^3$, and $G^4$ each represent the substituent including an ester group.

Since the compound represented by general formula (S1) has the substituent including an ester group, the polarity of the compound is high, or the compound is bulky. In this case, diffusion of an acid generated when the compound represented by general formula (S1) is irradiated with actinic rays or radiation is reduced. This is preferable because a pattern to be formed can easily have a rectangular cross-sectional shape.

The substituent including an ester group is preferably a group represented by the following general formula (GR1) or (GR2) and more preferably a group represented by the following general formula (GR1).

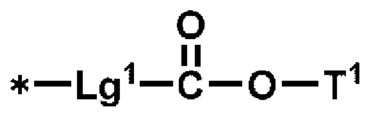

(GR1)

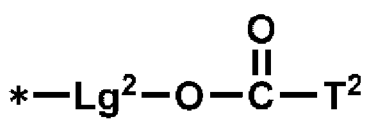

(GR2)

$Lg^1$ and $Lg^2$ in general formulas (GR1) and (GR2) each independently represent a single bond or a divalent

linking group. $T^1$ and $T^2$ each independently represent an organic group. * represents a bonding position to the benzene ring in general formula (QR1).

When $Lg^1$ in general formula (GR1) or $Lg^2$ in general formula (GR2) represents a divalent linking group, no particular limitation is imposed on the divalent linking group. Examples of the divalent linking group include -CO-, -O-, -S-, -SO-, $-SO_2-$, hydrocarbon groups (such as alkylene groups, cycloalkylene groups, alkenylene groups, and arylene groups), and linking groups formed by linking a plurality of groups selected from the above groups.

Preferably, $Lg^1$ in general formula (GR1) and $Lg^2$ in general formula (GR2) each represent a single bond.

No particular limitation is imposed on the organic group represented by $T^1$ in general formula (GR1) and the organic group represented by $T^2$ in general formula (GR2). Each organic group is preferably an organic group having 1 to 20 carbon atoms and more preferably an organic group having 1 to 15 carbon atoms.

Preferably, $T^1$ in general formula (GR1) and $T^2$ in general formula (GR2) each independently represent a chain aliphatic group optionally having a heteroatom or a cyclic aliphatic group optionally having a heteroatom.

When $T^1$ in general formula (GR1) or $T^2$ in general formula (GR2) represents a chain aliphatic group, the chain aliphatic group is preferably a linear or branched alkyl group, a linear or branched alkenyl group, or a linear or branched alkynyl group, more preferably a linear or branched alkyl group, and still more preferably an alkyl group having 1 to 5 carbon atoms such as a methyl group, an ethyl group, a n-propyl group, an isopropyl group, a n-butyl group, an isobutyl group, or a t-butyl group.

The chain aliphatic group may include a heteroatom. The heteroatom is preferably at least one selected from the group consisting of a sulfur atom, an oxygen atom, and a nitrogen atom and is more preferably an oxygen atom.

When $T^1$ in general formula (GR1) or $T^2$ in general formula (GR2) represents a chain aliphatic group, the chain aliphatic group may have a substituent. Examples of the substituent include: halogen atoms such as a fluorine atom, a chlorine atom, a bromine atom, and an iodine atom; monovalent halogenated hydrocarbon groups (e.g., monovalent halogenated hydrocarbon groups having 1 to 20 carbon atoms) such as a trifluoromethyl group; aryloxy groups (e.g., aryloxy groups having 6 to 20 carbon atoms) such as a phenoxy group; alkoxycarbonyl groups (e.g., alkoxycarbonyl groups having 2 to 10 carbon atoms) such as a methoxycarbonyl group and a butoxycarbonyl group; aryloxycarbonyl groups (e.g., aryloxycarbonyl groups having 7 to 20 carbon atoms) such as a phenoxycarbonyl group; acyloxy groups (e.g., acyloxy groups having 2 to 20 carbon atoms) such as an acetoxy group, a propionyloxy group, and a benzoyloxy group; acyl groups (e.g., acyl groups having 2 to 20 carbon atoms) such as an acetyl group, a benzoyl group, an isobutyryl group, an acryloyl group, a methacryloyl group, and a methoxalyl group; cycloalkyl groups (e.g., cycloalkyl groups having 3 to 15 carbon atoms; and an oxo group (=O) may be bonded to at least one carbon atom in the ring members) such as a cyclopentyl group, a cyclohexyl group, a norbornyl group, a tetracyclodecanyl group, a tetracyclododecanyl group, and an adamantyl group; aliphatic heterocyclic groups (e.g., aliphatic heterocyclic groups having 2 to 20 carbon atoms and including at least one heteroatom selected from the group consisting of a sulfur atom, an oxygen atom, and a nitrogen atom; an oxo group (=O) may be bonded to at least one atom in the ring members; and two atoms in the ring members may be linked together through at least one linking group selected from the group consisting of -O-, -CO-, -COO-, $-SO_2-$, and -NHCO-) such as a tetrahydropyranyl group; aryl groups (e.g., aryl groups having 6 to 20 carbon atoms); heteroaryl groups (e.g., heteroaryl groups having 2 to 20 carbon atoms and including at least one heteroatom selected from the group consisting of a sulfur atom, an oxygen atom, and a nitrogen atom); a hydroxy group; a carboxy group; a cyano group; a nitro group; an amino group; cyclic groups formed by fusing at least one selected from the group consisting of the above cycloalkyl groups and the above aliphatic heterocyclic groups and at least one selected from the group consisting of the above aryl groups and the above heteroaryl groups; and combinations of the above groups.

When $T^1$ in general formula (GR1) or $T^2$ in general formula (GR2) represents a cyclic aliphatic group, the cyclic aliphatic group is preferably a cycloalkyl group or an aliphatic heterocyclic group.

The cycloalkyl group is preferably a cycloalkyl group having 3 to 20 carbon atoms and more preferably a cycloalkyl group having 3 to 15 carbon atoms. In the cycloalkyl group, an oxo group (=O) may be bonded to at least one carbon atom in the ring members. The cycloalkyl group may be a monocyclic cycloalkyl group such as a cyclopentyl group or a cyclohexyl group or may be a polycyclic cycloalkyl group such as a norbornyl group, a tetracyclodecanyl group, a tetracyclododecanyl group, or an adamantyl group.

The aliphatic heterocyclic group is preferably an aliphatic heterocyclic group having 3 to 20 carbon atoms and more preferably an aliphatic heterocyclic group having 3 to 15 carbon atoms. Preferably, the aliphatic heterocyclic group is an aliphatic heterocyclic group including at least one heteroatom selected from the group consisting of a sulfur atom, an oxygen atom, and a nitrogen atom. In the aliphatic heterocyclic group, an oxo group (=O) may be bonded to at least one atom (preferably a carbon atom) in the ring members. In the aliphatic heterocyclic group, two atoms (preferably carbon atoms) in the ring members may be linked together through at least one linking group selected from the group consisting of -O-, -CO-, -COO-, $-SO_2-$, and -NHCO-. The aliphatic heterocyclic group may be a monocyclic aliphatic heterocyclic group or may be a polycyclic aliphatic heterocyclic group.

When $T^1$ in general formula (GR1) or $T^2$ in general formula (GR2) represents a cyclic aliphatic group, the cyclic aliphatic group may have a substituent. Examples of the substituent include: halogen atoms such as a fluorine atom, a

chlorine atom, a bromine atom, and an iodine atom; monovalent halogenated hydrocarbon groups (e.g., monovalent halogenated hydrocarbon groups having 1 to 20 carbon atoms) such as a trifluoromethyl group; alkoxy groups (e.g., alkoxy groups having 1 to 10 carbon atoms) such as a methoxy group, an ethoxy group, and a tert-butoxy group; aryloxy groups (e.g., aryloxy groups having 6 to 20 carbon atoms) such as a phenoxy group; alkoxycarbonyl groups (e.g., alkoxycarbonyl groups having 2 to 10 carbon atoms) such as a methoxycarbonyl group and a butoxycarbonyl group; aryloxycarbonyl groups (e.g., aryloxycarbonyl groups having 7 to 20 carbon atoms) such as a phenoxycarbonyl group; acyloxy groups (e.g., acyloxy groups having 2 to 20 carbon atoms) such as an acetoxy group, a propionyloxy group, and a benzoyloxy group; acyl groups (e.g., acyl groups having 2 to 20 carbon atoms) such as an acetyl group, a benzoyl group, an isobutyryl group, an acryloyl group, a methacryloyl group, and a methoxalyl group; alkyl groups (e.g., alkyl groups having 1 to 10 carbon atoms) such as a methyl group, an ethyl group, and a tert-butyl group; aryl groups (e.g., aryl groups having 6 to 20 carbon atoms); heteroaryl groups (e.g., heteroaryl groups having 2 to 20 carbon atoms and including at least one heteroatom selected from the group consisting of a sulfur atom, an oxygen atom, and a nitrogen atom); a hydroxy group; a carboxy group; a cyano group; a nitro group; an amino group; and combinations of the above groups.

The substituent including an ester group and represented by at least one of $G^1$, $G^2$, $G^3$, $G^4$, or $G^5$ in general formula (QR1) may be an acid-decomposable group or may be a group different from an acid-decomposable group (i.e., a group resistant to acid decomposition) but is preferably a group different from an acid-decomposable group.

The acid-decomposable group is a group that is decomposed by the action of an acid to generate a polar group and preferably has a structure in which the polar group is protected by a leaving group that leaves by the action of an acid. Specific examples of the acid-decomposable group, the polar group, and the leaving group and their preferred ranges are the same as those of an acid decomposable resin that will be described later.

In general formula (S1), $M^+$ represents an organic cation.

No particular limitation is imposed on the organic cation represented by $M^+$. The valence of the organic cation may be 1 or two or more.

In particular, the organic cation is preferably a cation represented by formula (ZaI) (hereinafter referred to as a "cation (ZaI)") or a cation represented by formula (ZaII) (hereinafter referred to as a "cation (ZaII)").

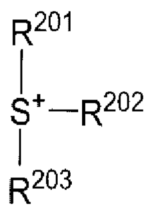

(ZaI)

$R^{204}$-$I^+$-$R^{205}$ (ZaII)

In formula (ZaI), $R^{201}$, $R^{202}$, and $R^{203}$ each independently represent an organic group.

The number of carbon atoms in each of the organic groups used as $R^{201}$, $R^{202}$, and $R^{203}$ is preferably 1 to 30 and more preferably 1 to 20. Two selected from the group consisting of $R^{201}$ to $R^{203}$ may be bonded together to form a ring structure, and the ring may include an oxygen atom, a sulfur atom, an ester group, an amido group, or a carbonyl group. Examples of the group formed from two selected from the group consisting of $R^{201}$ to $R^{203}$ that are bonded together include alkylene groups (such as a butylene group and a pentylene group) and $-CH_2-CH_2-O-CH_2-CH_2-$.

Preferred examples of the form of the organic cation in formula (ZaI) include a cation (ZaI-1), a cation (ZaI-2), an organic cation (cation (ZaI-3b)) represented by formula (ZaI-3b), and an organic cation (cation (ZaI-4b)) represented by formula (ZaI-4b) that will be described later.

First, the cation (ZaI-1) will be described.

The cation (ZaI-1) is an arylsulfonium cation in which at least one of $R^{201}$, $R^{202}$, or $R^{203}$ in formula (ZaI) is an aryl group.

In the arylsulfonium cation, each of $R_{201}$ to $R_{203}$ may be an aryl group. Alternatively, some of $R_{201}$ to $R_{203}$ may be an aryl group, and the rest may be an alkyl group or a cycloalkyl group.

Alternatively, one of $R_{201}$, $R_{202}$, or $R_{203}$ may be an aryl group, and the remaining two of $R^{201}$ to $R^{203}$ may be bonded together to form a ring structure. The ring may include an oxygen atom, a sulfur atom, an ester group, an amido group, or a carbonyl group. Examples of the group formed by bonding two selected from the group consisting of $R^{201}$ to $R^{203}$ together include alkylene groups in which at least one methylene group is replaced by an oxygen atom, a sulfur atom, an ester group, an amido group, and/or a carbonyl group (such as a butylene group, a pentylene group, and a $-CH_2-CH_2-O-CH_2-CH_2-$).

Examples of the arylsulfonium cation include triarylsulfonium cations, diarylalkylsulfonium cations, aryldialkylsulfonium cations, diarylcycloalkylsulfonium cations, and aryldicycloalkylsulfonium cations.

Each aryl group included in the arylsulfonium cation is preferably a phenyl group or a naphthyl group and is more preferably a phenyl group. The aryl group may have a heterocyclic structure having an oxygen atom, a nitrogen atom, a sulfur atom, etc. Examples of the heterocyclic structure include a pyrrole residue, a furan residue, a thiophene residue, an indole residue, a benzofuran residue, and a benzothiophene residue. When the arylsulfonium cation has two or more aryl groups, the two or more aryl groups may be the same or different.

The alkyl group or the cycloalkyl group optionally included in the arylsulfonium cation is preferably a linear alkyl group having 1 to 15 carbon atoms, a branched alkyl group having 3 to 15 carbon atoms, or a cycloalkyl group having 3 to 15 carbon atoms and more preferably a methyl group, an ethyl group, a propyl group, a n-butyl group, a sec-butyl group, a t-butyl group, a cyclopropyl group, a cyclobutyl group, or a cyclohexyl group.

The aryl, alkyl, and cycloalkyl groups in $R^{201}$ to $R^{203}$ may each have a substituent, and the substituent is preferably an alkyl group (having, for example, 1 to 15 carbon atoms), a cycloalkyl group (having, for example, 3 to 15 carbon atoms), an aryl group (having, for example, 6 to 14 carbon atoms), an alkoxy group (having, for example, 1 to 15 carbon atoms), a cycloalkylalkoxy group (having, for example, 1 to 15 carbon atoms), a halogen atom (for example, fluorine or iodine), a hydroxy group, a carboxy group, an ester group, a sulfinyl group, a sulfonyl group, an alkylthio group, or a phenylthio group.

Each substituent may have a substituent if possible. It is also preferable that the alkyl group has a halogen atom as a substituent and is therefore a halogenated alkyl group such as a trifluoromethyl group.

It is also preferable that any of these substituents are combined together to form an acid-decomposable group.

The acid-decomposable group is a group that is decomposed by the action of an acid to generate a polar group and preferably has a structure in which the polar group is protected by a leaving group that leaves by the action of an acid. Specific examples of the acid-decomposable group, the polar group, and the leaving group and their preferred ranges are the same as those of the acid decomposable resin that will be described later.

Next, the cation (ZaI-2) will be described.

The cation (ZaI-2) is a cation in which $R^{201}$ to $R^{203}$ in formula (ZaI) each independently represent an organic group having no aromatic ring. The aromatic ring is intended to encompass an aromatic ring including a heteroatom.

The number of carbon atoms in each of the organic groups having no aromatic ring and represented by $R^{201}$ to $R^{203}$ is preferably 1 to 30 and more preferably 1 to 20.

$R^{201}$ to $R^{203}$ are each independently preferably an alkyl group, a cycloalkyl group, an allyl group, or a vinyl group, more preferably a linear or branched 2-oxoalkyl group, a 2-oxocycloalkyl group, or an alkoxycarbonylmethyl group, and still more preferably a linear or branched 2-oxoalkyl group.

Examples of the alkyl and cycloalkyl groups in $R^{201}$ to $R^{203}$ include: linear alkyl groups having 1 to 10 carbon atoms and branched alkyl groups having 3 to 10 carbon atoms (such as a methyl group, an ethyl group, a propyl group, a butyl group, and a pentyl group); and cycloalkyl groups having 3 to 10 carbon atoms (such as a cyclopentyl group, a cyclohexyl group, and a norbornyl group).

$R^{201}$ to $R^{203}$ may each be further substituted with a halogen atom, an alkoxy group (having, for example, 1 to 5 carbon atoms), a hydroxy group, a cyano group, or a nitro group.

It is also preferable that the substituents in $R^{201}$ to $R^{203}$ are each independently combined with another substituent to form an acid-decomposable group.

Next, the cation (ZaI-3b) will be described.

The cation (ZaI-3b) is a cation represented by the following formula (ZaI-3b).

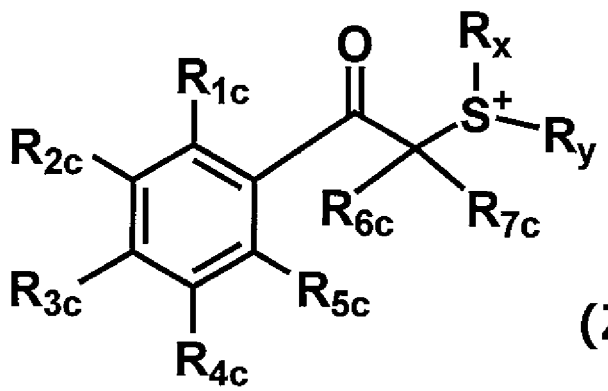

(ZaI-3b)

In formula (ZaI-3b), $R_{1c}$ to $R_{5c}$ each independently represent a hydrogen atom, an alkyl group, a cycloalkyl group, an aryl group, an alkoxy group, an aryloxy group, an alkoxycarbonyl group, an alkylcarbonyloxy group, a cycloalkylcarbonyloxy group, a halogen atom, a hydroxy group, a nitro group, an alkylthio group, or an arylthio group.

$R_{6c}$ and $R_{7c}$ each independently represent a hydrogen atom, an alkyl group (such as a t-butyl group), a cycloalkyl group, a halogen atom, a cyano group, or an aryl group.

$R_x$ and $R_y$ each independently represent an alkyl group, a cycloalkyl group, a 2-oxoalkyl group, a 2-oxocycloalkyl group, an alkoxycarbonylalkyl group, an allyl group, or a vinyl group.

$R_{1c}$ to $R_{7c}$, $R_x$, and $R_y$ may each have a substituent, and it is also preferable that these substituents are each independently combined with another substituent to form an acid-decomposable group.

A combination of two or more selected from the group consisting of $R_{1c}$ to $R_{5c}$, a pair of $R_{5c}$ and $R_{6c}$, a pair of $R_{6c}$ and $R_{7c}$, a pair of $R_{5c}$ and $R_x$, and a pair of $R_x$ and $R_y$ may each be bonded together to form a ring. These rings may each independently include an oxygen atom, a sulfur atom, a ketone group, an ester bond, or an amide bond.

Each ring may be an aromatic or non-aromatic hydrocarbon ring, an aromatic or non-aromatic heterocyclic ring, or a polycyclic condensed ring formed by combining two or more of the above rings. The ring may be a 3- to 10-membered ring and is preferably a 4- to 8-membered ring and more preferably a 5- or 6-membered ring.

Examples of the groups formed by bonding two or more selected from the group consisting of $R_{1c}$ to $R_{5c}$, bonding $R_{6c}$ and $R_{7c}$, and bonding $R_x$ and $R_y$ include alkylene groups such as a butylene group and a pentylene group. A methylene group in the alkylene group may be replaced with a heteroatom such as an oxygen atom.

The group formed by bonding $R_{5c}$ and Rec and the group formed by bonding $R_{5c}$ and $R_x$ are each preferably a single bond or an alkylene group. Examples of the alkylene group include a methylene group and an ethylene group.

$R_{1c}$ to $R_{5c}$, $R_{6c}$, $R_{7c}$, $R_x$, $R_y$, the ring formed by bonding together a combination of two or more selected from the group consisting of $R_{1c}$ to $R_{5c}$, the ring formed by bonding together a pair of $R_{5c}$ and $R_{6c}$, the ring formed by bonding together a pair of $R_{6c}$ and $R_{7c}$, the ring formed by bonding together a pair of $R_{5c}$ and $R_x$, and the ring formed by bonding together a pair of $R_x$ and $R_y$ may each have a substituent.

Next, the cation (ZaI-4b) will be described.

The cation (ZaI-4b) is a cation represented by the following formula (ZaI-4b).

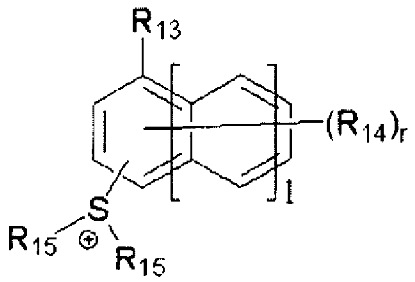

(ZaI-4b)

In formula (ZaI-4b), 1 represents an integer of from 0 to 2.

r represents an integer of from 0 to 8.

$R_{13}$ represents a hydrogen atom, a halogen atom (such as a fluorine atom or an iodine atom), a hydroxy group, an alkyl group, a halogenated alkyl group, an alkoxy group, a carboxy group, an alkoxycarbonyl group, or a group including a cycloalkyl group (a cycloalkyl group itself or a group including a cycloalkyl group as a part thereof). These groups may each have a substituent.

$R_{14}$ represents a hydroxy group, a halogen atom (such as a fluorine atom or an iodine atom), an alkyl group, a halogenated alkyl group, an alkoxy group, an alkoxycarbonyl group, an alkylcarbonyl group, an alkylsulfonyl group, a cycloalkylsulfonyl group, or a group including a cycloalkyl group (a cycloalkyl group itself or a group including a cycloalkyl group as a part thereof). These groups may each have a substituent. When a plurality of $R_{14}$'s are present, they each independently represent any of the above groups such as a hydroxy group.

$R_{15}$'s each independently represent an alkyl group, a cycloalkyl group, or a naphthyl group. The two $R_{15}$'s may be bonded together to form a ring. When the two $R_{15}$'s are bonded together to form a ring, the skeleton of the ring may include a heteroatom such as an oxygen atom or a nitrogen atom.

In one preferred mode, the two $R_{15}$'s are each an alkylene group and are bonded together to form a ring structure. The above alkyl, cycloalkyl, and naphthyl groups and the ring formed by bonding the two $R_{15}$'s may each have a substituent.

In formula (ZaI-4b), the alkyl group represented by each of $R_{13}$, $R_{14}$, and $R_{15}$s may be a linear or branched alkyl group. Preferably, the number of carbon atoms in the alkyl group is 1 to 10. Each alkyl group is preferably a methyl group, an ethyl group, a n-butyl group, a t-butyl group, etc.

It is also preferable that the substituents in $R_{13}$ to $R_{15}$'s, $R_x$, and $R_y$ are each independently combined with another substituent to form an acid-decomposable group.

Next, formula (ZaII) will be described.

In formula (ZaII), $R^{204}$ and $R^{205}$ each independently represent an aryl group, an alkyl group, or a cycloalkyl group.

The aryl group represented by each of $R^{204}$ and $R^{205}$ is preferably a phenyl group or a naphthyl group and more preferably a phenyl group. The aryl group represented by each of $R^{204}$ and $R^{205}$ may be an aryl group having a heterocycle having an oxygen atom, a nitrogen atom, or a sulfur atom. Examples of the skeleton of the aryl group having a heterocycle include pyrrole, furan, thiophene, indole, benzofuran, and benzothiophene.

The alkyl or cycloalkyl group represented by each of $R^{204}$ and $R^{205}$ is preferably a linear alkyl group having 1 to 10 carbon atoms or a branched alkyl group having 3 to 10 carbon atoms (such as a methyl group, an ethyl group, a propyl group, a butyl group, or a pentyl group) or is a cycloalkyl group having 3 to 10 carbon atoms (such as a cyclopentyl group, a cyclohexyl group, or a norbornyl group).

The aryl, alkyl, and cycloalkyl groups represented by $R^{204}$ and $R^{205}$ may each independently have a substituent. Examples of the optional substituents in the aryl, alkyl, and cycloalkyl groups represented by $R^{204}$ and $R^{205}$ include alkyl groups (having, for example, 1 to 15 carbon atoms), cycloalkyl groups (having, for example, 3 to 15 carbon atoms), aryl groups (having, for example, 6 to 15 carbon atoms), alkoxy groups (having, for example, 1 to 15 carbon atoms), halogen atoms, a hydroxy group, and a phenylthio group. It is also preferable that the substituents in $R^{204}$ and $R^{205}$ are each independently combined with another substituent to form an acid-decomposable group.

Specific examples of the organic cation are shown below. However, the invention is not limited thereto.

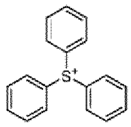
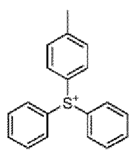
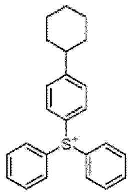
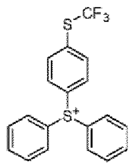

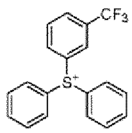
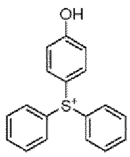
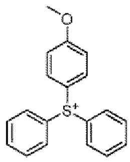
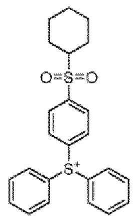

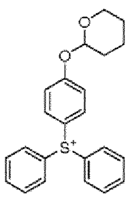
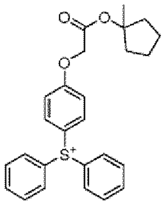
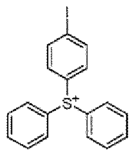
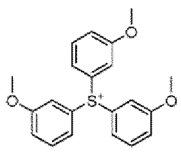

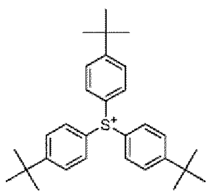
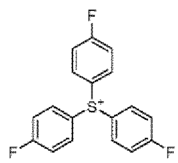
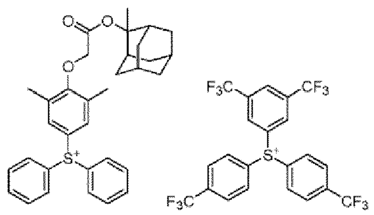

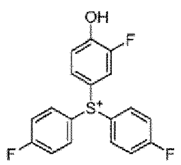
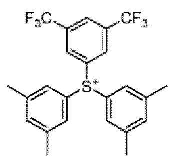
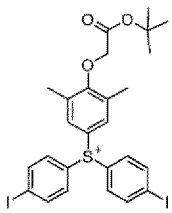
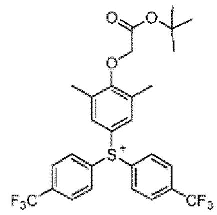

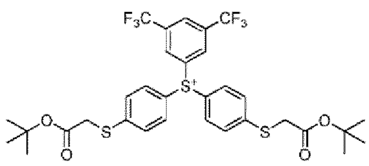
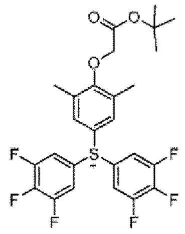
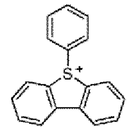

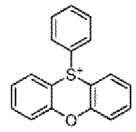
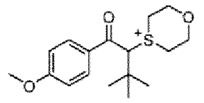
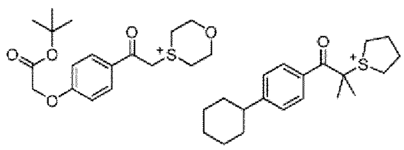

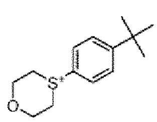
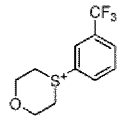
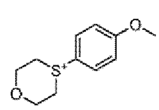
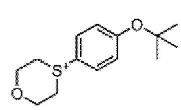

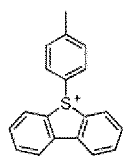
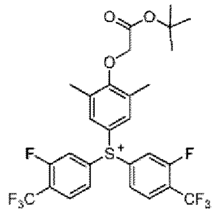

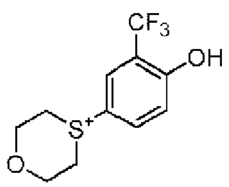
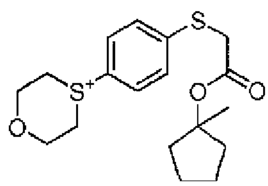
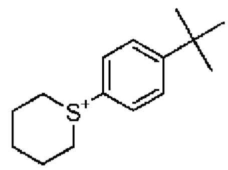

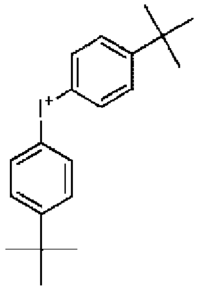
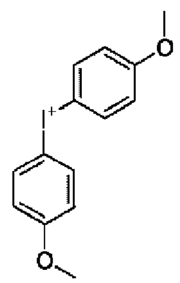
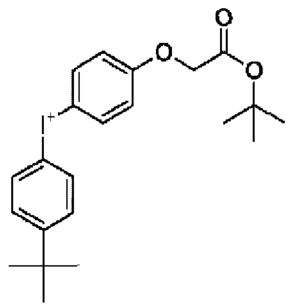

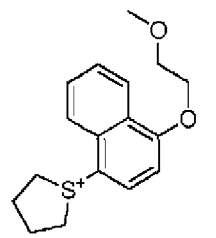
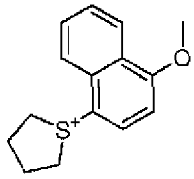

The molecular weight of the compound represented by general formula (S1) is preferably 3000 or less, more preferably 2000 or less, and still more preferably 1500 or less. No particular limitation is imposed on the lower limit of

the molecular weight, but the molecular weight is preferably 300 or more.

The number of compounds represented by general formula (S1) and included in the composition of the invention may be one or two or more.

No particular limitation is imposed on the content of the compound represented by general formula (S1) in the composition of the invention. The content of the compound with respect to the total mass of solids in the composition of the invention is preferably 0.5% by mass or more, more preferably 1.0% by mass or more, and still more preferably 2.0% by mass or more. The content of the compound represented by general formula (S1) with respect to the total mass of the solids in the composition of the invention is preferably 65.0% by mass or less, more preferably 55.0% by mass or less, and still more preferably 45.0% by mass or less.

<Acid decomposable resin>

The composition of the invention includes an acid decomposable resin (hereinafter referred to as a "resin (A)").

The resin (A) generally includes a group that is decomposed by the action of an acid and thereby increased in polarity (this groups is hereafter referred to also as an "acid-decomposable group") and preferably includes a repeating unit having the acid-decomposable group.

In the pattern forming method of the invention, when a developer used is an alkali developer, a preferred positive-type pattern is typically formed. When the developer used is an organic-based developer, a preferred negative-type pattern is typically formed.

The repeating unit having an acid-decomposable group is preferably a (repeating unit having an acid-decomposable group) described later and is also preferably a (repeating unit having an acid-decomposable group including an unsaturated bond).

(Repeating unit having acid-decomposable group)

The acid-decomposable group is a group that is decomposed by the action of an acid to generate a polar group. Preferably, the acid-decomposable group has a structure in which the polar group is protected by a leaving group that leaves by the action of an acid. Specifically, the resin (A) has a repeating unit having a group that is decomposed by the action of an acid to generate a polar group. The resin having this repeating unit is increased in polarity by the action of an acid. The degree of solubility in an alkali developer thereby increases, and the degree of solubility in an organic solvent decreases.

The polar group is preferably an alkali-soluble group, and examples thereof include: acidic groups such as a carboxy group, phenolic hydroxy groups, fluorinated alcohol groups, sulfonic acid groups, phosphoric acid groups, sulfonamido groups, sulfonylimido groups, (alkylsulfonyl)(alkylcarbonyl)methylene groups, (alkylsulfonyl)(alkylcarbonyl)imido groups, bis(alkylcarbonyl)methylene groups, bis(alkylcarbonyl)imido groups, bis(alkylsulfonyl)methylene groups, bis(alkylsulfonyl)imido groups, tris(alkylcarbonyl)methylene groups, and tris(alkylsulfonyl)methylene groups; and alcoholic hydroxyl groups.

In particular, the polar group is preferably a carboxy group, a phenolic hydroxy group, a fluorinated alcohol group (preferably a hexafluoroisopropanol group), or a sulfonic acid group.

Examples of the leaving group that leaves by the action of an acid include groups represented by formulas (Y1) to (Y4).

Formula (Y1): $-C(Rx_1)(Rx_2)(Rx_3)$

Formula (Y2): $-C(=O)OC(Rx_1)(Rx_2)(Rx_3)$

Formula (Y3): $-C(R_{36})(R_{37})(OR_{38})$

Formula (Y4): $-C(Rn)(H)(Ar)$

In formulas (Y1) and (Y2), $Rx_1$ to $Rx_3$ each independently represent an alkyl group (linear or branched alkyl group), a cycloalkyl group (monocyclic or polycyclic cycloalkyl group), an alkenyl group (linear or branched alkenyl group), or an aryl group (monocyclic or polycyclic aryl group). When all of $Rx_1$ to $Rx_3$ are alkyl groups (linear or branched alkyl groups), it is preferable that at least two selected from the group consisting of $Rx_1$ to $Rx_3$ are each a methyl group.

In particular, it is preferable that $Rx_1$ to $Rx_3$ each independently represent a linear or branched alkyl group, and it is more preferable that $Rx_1$ to $Rx_3$ each independently represent a linear alkyl group.

Two selected from the group consisting of $Rx_1$ to $Rx_3$ may be bonded together to form a monocyclic or polycyclic ring.

The alkyl group represented by each of $Rx_1$ to $Rx_3$ is preferably an alkyl group having 1 to 5 carbon atoms such as a methyl group, an ethyl group, a n-propyl group, an isopropyl group, a n-butyl group, an isobutyl group, or a t-butyl group.

The cycloalkyl group represented by each of $Rx_1$ to $Rx_3$ is preferably a monocyclic cycloalkyl group such as a cyclopentyl group or a cyclohexyl group or a polycyclic cycloalkyl group such as a norbornyl group, a tetracyclodecanyl group, a tetracyclododecanyl group, or an adamantyl group.

The aryl group represented by each of $Rx_1$ to $Rx_3$ is preferably an aryl group having 6 to 10 carbon atoms, and examples thereof include a phenyl group, a naphthyl group, and an anthryl group.

The alkenyl group represented by each of $Rx_1$ to $Rx_3$ is preferably a vinyl group.

The ring formed by bonding two selected from the group consisting of $Rx_1$ to $Rx_3$ is preferably a cycloalkyl group. The cycloalkyl group formed by bonding two selected from the group consisting of $Rx_1$ to $Rx_3$ is preferably a monocyclic cycloalkyl group such as a cyclopentyl group or a cyclohexyl group or a polycyclic cycloalkyl group such as a norbornyl group, a tetracyclodecanyl group, a tetracyclododecanyl group, or an adamantyl group and is more preferably a monocyclic cycloalkyl group having 5 to 6 carbon atoms.

One methylene group included in the ring in the cycloalkyl group formed by bonding two selected from the group consisting of $Rx_1$ to $Rx_3$ may be replaced with a heteroatom such as an oxygen atom, a group including a heteroatom such as a carbonyl group, or a vinylidene group. In any of these cycloalkyl groups, at least one ethylene group included in the cycloalkane ring may be replaced with a vinylene group.

In the group represented by formula (Y1) or formula (Y2), it is preferable that, for example, $Rx_1$ is a methyl group or an ethyl group and that $Rx_2$ and $Rx_3$ are bonded together to form the cycloalkyl group described above.

When the composition of the invention is, for example, a resist composition for EUV exposure, it is also preferable that the alkyl, cycloalkyl, alkenyl, and aryl groups represented by $Rx_1$ to $Rx_3$ and the ring formed by bonding two selected from the group consisting of $Rx_1$ to $Rx_3$ each further have a fluorine atom or an iodine atom as a substituent.

In formula (Y3), $R_{36}$ to $R_{38}$ each independently represent a hydrogen atom or a monovalent organic group. $R_{37}$ and $R_{38}$ may be bonded together to form a ring. Examples of the monovalent organic group include alkyl groups, cycloalkyl groups, aryl groups, aralkyl groups, and alkenyl groups. It is also preferable that $R_{36}$ is a hydrogen atom.

The alkyl, cycloalkyl, aryl, and aralkyl groups described above may each include a heteroatom such as an oxygen atom and/or a group including a heteroatom such as a carbonyl group. For example, in the alkyl, cycloalkyl, aryl, and aralkyl groups described above, at least one methylene group may be replaced with a heteroatom such as an oxygen atom and/or a group including a heteroatom such as a carbonyl group.

$R_{38}$ may be bonded to another substituent included in the main chain of the repeating unit to form a ring. The group formed by bonding $R_{38}$ and another substituent included in the main chain of the repeating unit is preferably an alkylene group such as a methylene group.

When the composition of the invention is, for example, a resist composition for EUV exposure, it is also preferable that the monovalent organic groups represented by $R_{36}$ to $R_{38}$ and the group formed by bonding $R_{37}$ and $R_{38}$ together each further have a fluorine atom or an iodine atom as a substituent.

Formula (Y3) is preferably a group represented by the following formula (Y3-1).

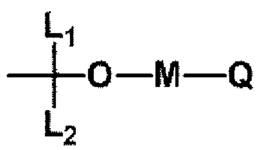

(Y3-1)

$L_1$ and $L_2$ each independently represent a hydrogen atom, an alkyl group, a cycloalkyl group, an aryl group, or a group formed by combining any of them (for example, a group formed by combining an alkyl group and an aryl group).

M represents a single bond or a divalent linking group.

Q represents an alkyl group optionally including a heteroatom, a cycloalkyl group optionally including a heteroatom, an aryl group optionally including a heteroatom, an amino group, an ammonium group, a mercapto group, a cyano group, an aldehyde group, or a group formed by combining any of them (for example, a group formed by combining an alkyl group and a cycloalkyl group).

In the alkyl and cycloalkyl groups, for example, one methylene group may be replaced with a heteroatom such as an oxygen atom or a group including a heteroatom such as a carbonyl group.

It is preferable that one of $L_1$ or $L_2$ is a hydrogen atom and that the other is an alkyl group, a cycloalkyl group, an aryl group, or a group formed by combining an alkylene group and an aryl group.

At least two selected from the group consisting of Q, M, and $L_1$ may be bonded together to form a ring (preferably a 5-membered or 6-membered ring).

From the viewpoint of obtaining a finer pattern, $L_2$ is preferably a secondary or tertiary alkyl group and more

preferably a tertiary alkyl group. Examples of the secondary alkyl group include an isopropyl group, a cyclohexyl group, and a norbornyl group, and examples of the tertiary alkyl group include a tert-butyl group and an adamantane group. In these modes, since Tg (glass transition temperature) and activation energy are high, high film hardness is obtained, and the occurrence of fogging can be reduced.

When the composition of the invention is, for example, a resist composition for EUV exposure, it is also preferable that the alkyl, cycloalkyl, and aryl groups represented by $L_1$ and $L_2$ and a group formed by combining any of these groups each further have a fluorine atom or an iodine atom as a substituent. It is also preferable that the alkyl, cycloalkyl, aryl, and aralkyl groups each include a heteroatom such as an oxygen atom other than a fluorine atom and an iodine atom (i.e., in the alkyl, cycloalkyl, aryl, and aralkyl groups, for example, one methylene group is replaced with a heteroatom such as an oxygen atom or a group including a heteroatom such as a carbonyl group).

When the composition of the invention is, for example, a resist composition for EUV exposure, it is also preferable that, in the alkyl group optionally including a heteroatom, the cycloalkyl group optionally including a heteroatom, the aryl group optionally including a heteroatom, the amino group, the ammonium group, the mercapto group, the cyano group, and the aldehyde group that are represented by Q and a combination of any of these groups, the heteroatom is one selected from the group consisting of a fluorine atom, an iodine atom, and an oxygen atom.

In formula (Y4), Ar represents an aromatic ring group. Rn represents an alkyl group, a cycloalkyl group, or an aryl group. Rn and Ar may be bonded together to form a non-aromatic ring. Ar is preferably an aryl group.

When the composition of the invention is, for example, a resist composition for EUV exposure, it is also preferable that the aromatic ring group represented by Ar and the alkyl, cycloalkyl, or aryl group represented by Rn each have a fluorine atom or an iodine atom as a substituent.

When, in the leaving group protecting the polar group, a non-aromatic ring is bonded directly to the polar group (or its residue), it is also preferable that a ring member atom adjacent to the ring member atom bonded directly to the polar group (or its residue) in the non-aromatic ring does not have a halogen atom such as a fluorine atom as a substituent, because the repeating unit can have good acid-decomposability.

The leaving group that leaves by the action of an acid may also be a 2-cyclopentenyl group having a substituent (e.g., an alkyl group) such as a 3-methyl-2-cyclopentenyl group or a cyclohexyl group having a substituent (e.g., an alkyl group) such as a 1,1,4,4-tetramethylcyclohexyl group.

The repeating unit having an acid-decomposable group is also preferably a repeating unit represented by formula (A).

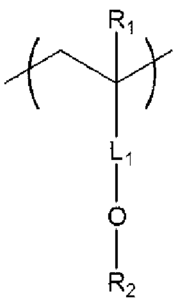

(A)

$L_1$ represents a divalent linking group optionally having a fluorine atom or an iodine atom, and $R_1$ represents a hydrogen atom, a fluorine atom, an iodine atom, an alkyl group optionally having a fluorine atom or an iodine atom, or an aryl group optionally having a fluorine atom or an iodine atom. $R_2$ represents a leaving group that optionally has a fluorine atom or an iodine atom and leaves by the action of an acid. At least one of $L_1$, $R_1$, or $R_2$ has a fluorine atom or an iodine atom.

$L_1$ represents a divalent linking group optionally having a fluorine atom or an iodine atom. Examples of the divalent linking group optionally having a fluorine atom or an iodine atom include -CO-, -O-, -S-, -SO-, $-SO_2-$, hydrocarbon groups optionally having a fluorine atom or an iodine atom (such as alkylene groups, cycloalkylene groups, alkenylene groups, and arylene groups), and linking groups formed by linking a plurality of groups selected from the above groups. In particular, $L_1$ is preferably -CO-, an arylene group, or -arylene group-fluorine or iodine atom-containing alkylene group- and more preferably -CO- or -arylene group-fluorine or iodine atom-containing alkylene group-.

The arylene group is preferably a phenylene group.

The alkylene group may by a linear or branched alkylene group. No particular limitation is imposed on the number of carbon atoms in the alkylene group, but the number of carbon atoms is preferably 1 to 10 and more preferably 1 to 3.

No particular limitation is imposed on the total number of fluorine or iodine atoms included in the fluorine or iodine atom-containing alkylene group, but the total number of fluorine or iodine atoms is preferably two or more, more

preferably 2 to 10, and still more preferably 3 to 6.

$R_1$ represents a hydrogen atom, a fluorine atom, an iodine atom, an alkyl group optionally having a fluorine atom or an iodine atom, or an aryl group optionally having a fluorine atom or an iodine atom.

The alkyl group may be a linear or branched alkyl group. No particular limitation is imposed on the number of carbon atoms in the alkyl group, but the number of carbon atoms is preferably 1 to 10 and more preferably 1 to 3.

No particular limitation is imposed on the total number of fluorine or iodine atoms included in the fluorine or iodine atom-containing alkyl group, but the total number of fluorine or iodine atoms is preferably 1 or more, more preferably 1 to 5, and still more preferably 1 to 3.

The alkyl group may include a heteroatom such as an oxygen atom other than halogen atoms.

$R_2$ represents a leaving group that leaves by the action of an acid and that optionally has a fluorine atom or an iodine atom. Examples of the leaving group optionally having a fluorine atom or an iodine atom include leaving groups represented by formulas (Y1) to (Y4) described above and having a fluorine atom or an iodine atom.

It is also preferable that the repeating unit having an acid-decomposable group is a repeating unit represented by formula (AI).

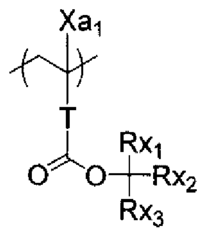

(A I)

In formula (AI), $Xa_1$ represents a hydrogen atom or an alkyl group optionally having a substituent. T represents a single bond or a divalent linking group. $Rx_1$ to $Rx_3$ each independently represent an alkyl group (linear or branched alkyl group), a cycloalkyl group (monocyclic or polycyclic cycloalkyl group), an alkenyl group (linear or branched alkenyl group), or an aryl group (monocyclic or polycyclic aryl group). However, when all of $Rx_1$ to $Rx_3$ are alkyl groups (linear or branched alkyl groups), it is preferable that at least two selected from the group consisting of $Rx_1$ to $Rx_3$ are each a methyl group.

Two selected from the group consisting of $Rx_1$ to $Rx_3$ may be bonded together to form a monocyclic or polycyclic group (such as a monocyclic or polycyclic cycloalkyl group).

Examples of the alkyl group optionally having a substituent and represented by $Xa_1$ include a methyl group and a group represented by $-CH_2-R_{11}$. $R_{11}$ represents a halogen atom (such as a fluorine atom), a hydroxy group, or a monovalent organic group, and examples thereof include alkyl groups having 5 or less carbon atoms and optionally substituted with a halogen atom, acyl groups having 5 or less carbon atoms and optionally substituted with a halogen atom, and alkoxy groups having 5 or less carbon atoms and optionally substituted with a halogen atom. $R_{11}$ is preferably an alkyl group having 3 or less carbon atoms and more preferably a methyl group. $Xa_1$ is preferably a hydrogen atom, a methyl group, a trifluoromethyl group, or a hydroxymethyl group.

Examples of the divalent linking group represented by T include alkylene groups, aromatic ring groups, a -COO-Rt-group, and an -O-Rt-group. In these formulas, Rt represents an alkylene group or a cycloalkylene group.

T is preferably a single bond or a -COO-Rt-group. When T represents a -COO-Rt-group, Rt is preferably an alkylene group having 1 to 5 carbon atoms and more preferably a $-CH_2-$ group, a $-(CH_2)_2-$ group, or a $-(CH_2)_3-$ group.

The alkyl group represented by each of $Rx_1$ to $Rx_3$ is preferably an alkyl group having 1 to 4 carbon atoms such as a methyl group, an ethyl group, a n-propyl group, an isopropyl group, a n-butyl group, an isobutyl group, or a t-butyl group.

The cycloalkyl group represented by each of $Rx_1$ to $Rx_3$ is preferably a monocyclic cycloalkyl group such as a cyclopentyl group or a cyclohexyl group or a polycyclic cycloalkyl group such as a norbornyl group, a tetracyclodecanyl group, a tetracyclododecanyl group, or an adamantyl group.

The aryl group represented by each of $Rx_1$ to $Rx_3$ is preferably an aryl group having 6 to 10 carbon atoms, and examples thereof include a phenyl group, a naphthyl group, and an anthryl group.

The alkenyl group represented by each of $Rx_1$ to $Rx_3$ is preferably a vinyl group.

The cycloalkyl group formed by bonding two selected from the group consisting of $Rx_1$ to $Rx_3$ is preferably a monocyclic cycloalkyl group such as a cyclopentyl group or a cyclohexyl group. The cycloalkyl group is also preferably a polycyclic cycloalkyl group such as a norbornyl group, a tetracyclodecanyl group, a tetracyclododecanyl group, or an adamantyl group. In particular, a monocyclic cycloalkyl group having 5 to 6 carbon atoms is preferred.

In the cycloalkyl group formed by bonding two selected from the group consisting of $Rx_1$ to $Rx_3$, for example, one methylene group included in the ring may be replaced with a heteroatom such as an oxygen atom, a group including a heteroatom such as a carbonyl group, or a vinylidene group. In each cycloalkyl group, at least one ethylene group included in the cycloalkane ring may be replaced with a vinylene group.

In the repeating unit represented by formula (AI), it is preferable that, for example, $Rx_1$ is a methyl group or an ethyl group and that $Rx_2$ and $Rx_3$ are bonded together to form the cycloalkyl group described above.

When any of the above-described groups has a substituent, examples of the substituent include alkyl groups (having 1 to 4 carbon atoms), halogen atoms, a hydroxy group, alkoxy groups (having 1 to 4 carbon atoms), a carboxy group, and alkoxycarbonyl groups (having 2 to 6 carbon atoms). The number of carbon atoms in the substituent is preferably 8 or less.

The repeating unit represented by formula (AI) is preferably an acid-decomposable tertiary alkyl (meth)acrylate-based repeating unit (a repeating unit in which $Xa_1$ represents a hydrogen atom or a methyl group and T represents a single bond).

Specific examples of the repeating unit having an acid-decomposable group are shown below, but the present invention is not limited thereto. In the formulas below, $Xa_1$ represents H, $CH_3$, $CF_3$, or $CH_2OH$, and Rxa and Rxb each independently represent a linear or branched alkyl group having 1 to 5 carbon atoms.

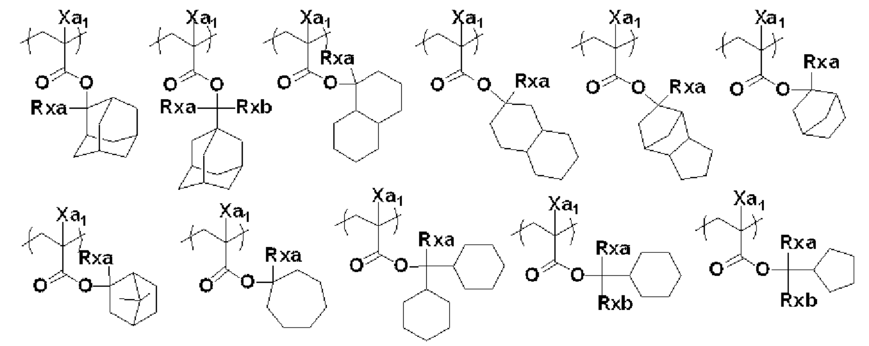

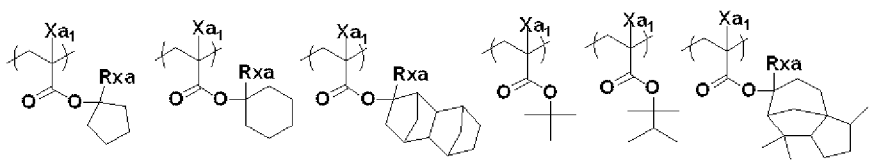

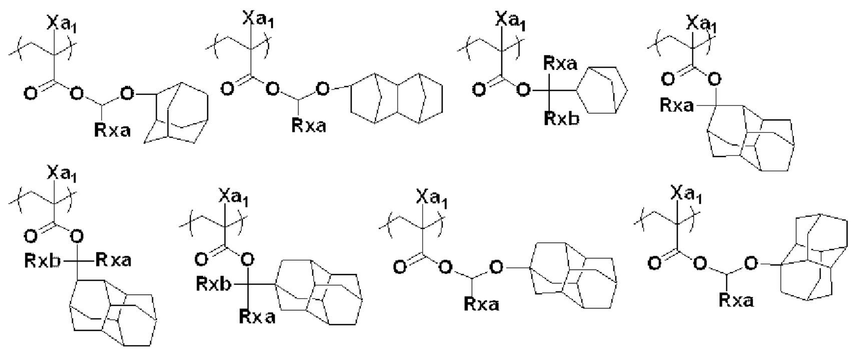

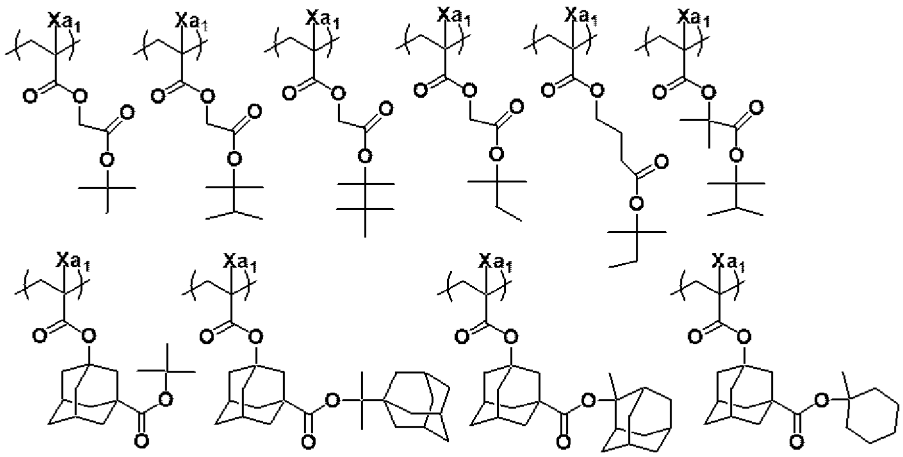

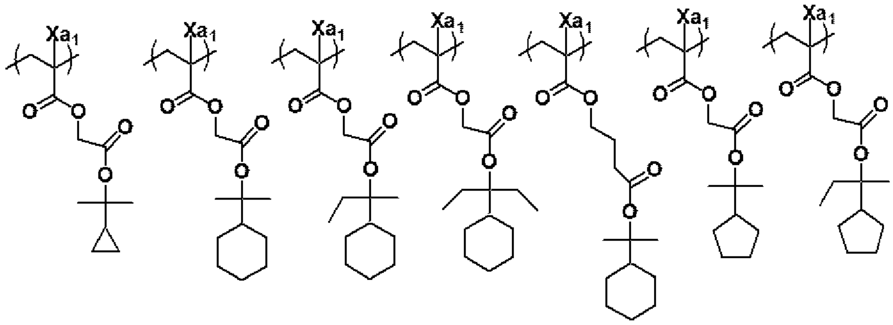

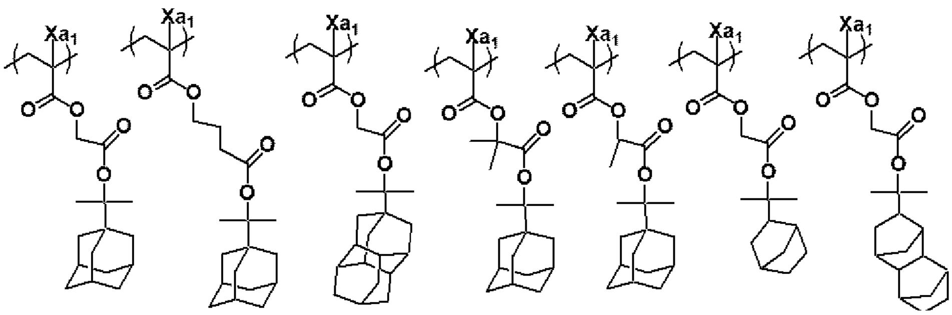

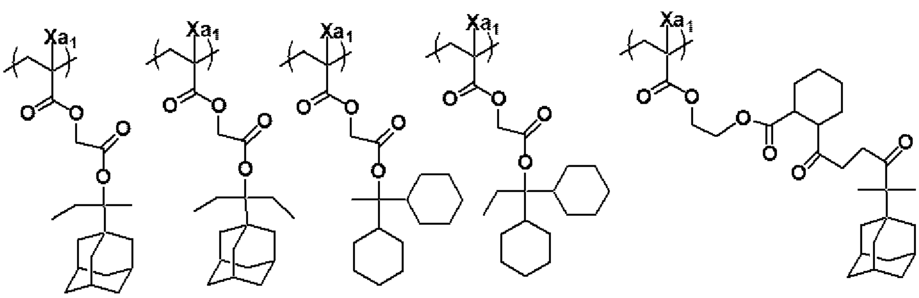

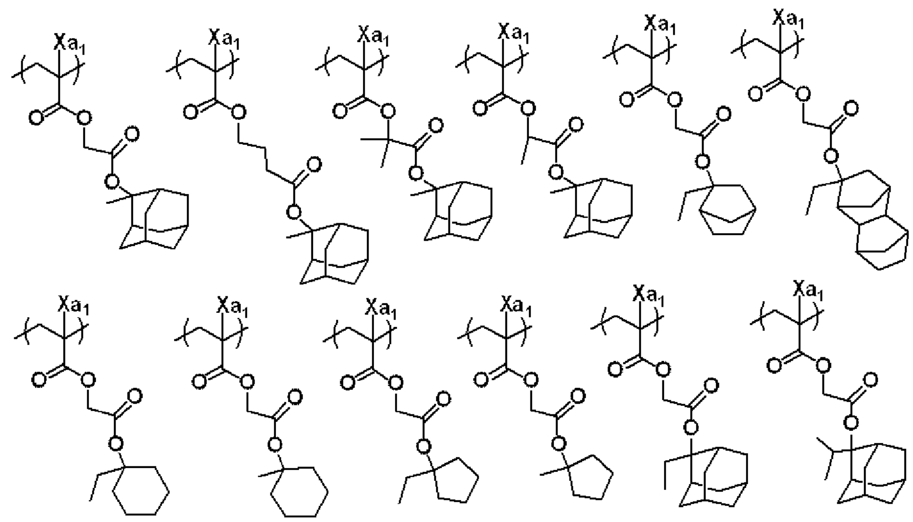

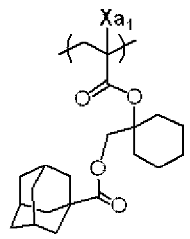

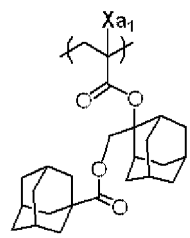

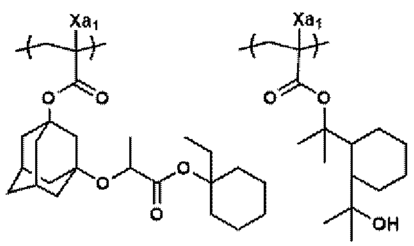

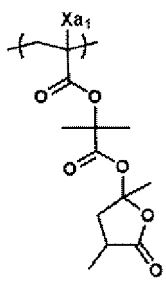

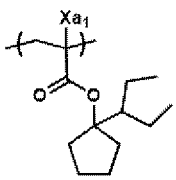

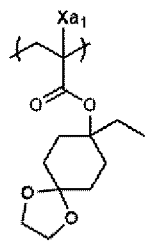

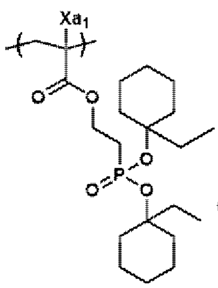

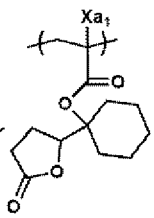

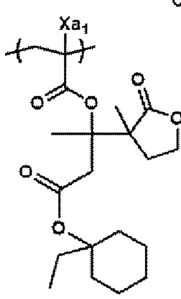

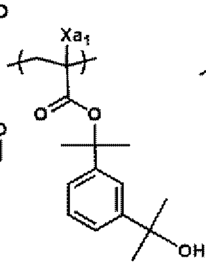

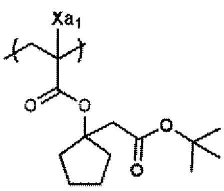

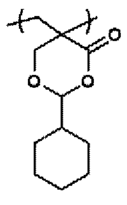
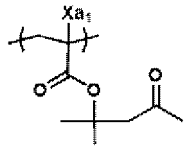
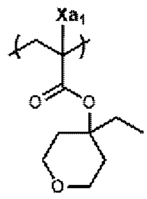
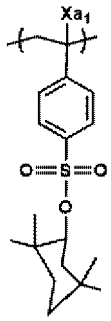
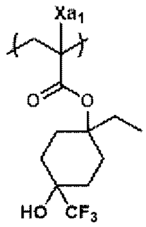
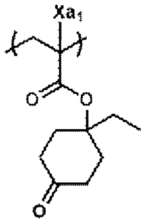
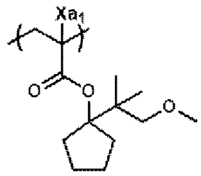
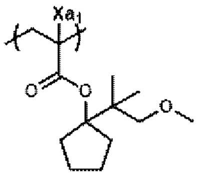

The resin (A) may include, as the repeating unit having the acid-decomposable group, a repeating unit having an acid-decomposable group including an unsaturated bond.

The repeating unit having an acid-decomposable group including an unsaturated bond is preferably a repeating unit represented by formula (B).

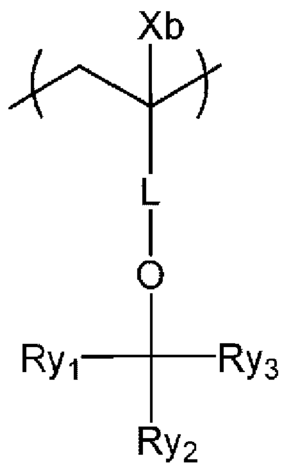

(B)

In formula (B), Xb represents a hydrogen atom, a halogen atom, or an alkyl group optionally having a substituent. L represents a single bond or a divalent linking group optionally having a substituent. $Ry_1$ to $Ry_3$ each independently represent a linear or branched alkyl group, a monocyclic or polycyclic cycloalkyl group, an alkenyl group, an alkynyl group, or a monocyclic or polycyclic aryl group. However, at least one of Ryi, $Ry_2$, or $Ry_3$ represents an alkenyl group, an alkynyl group, a monocyclic or polycyclic cycloalkenyl group, or a monocyclic or polycyclic aryl group.

Two selected from the group consisting of $Ry_1$ to $Ry_3$ may be bonded together to form a monocyclic or polycyclic ring (such as a monocyclic or polycyclic cycloalkyl group or a monocyclic or polycyclic cycloalkenyl group).

The alkyl group optionally having a substituent and represented by Xb is, for example, a methyl group or a group represented by $-CH_2-R_{11}$. $R_{11}$ represents a halogen atom (such as a fluorine atom), a hydroxy group, or a monovalent organic group, and examples thereof include alkyl groups having 5 or less carbon atoms and optionally substituted with a halogen atom, acyl groups having 5 or less carbon atoms and optionally substituted with a halogen atom, and alkoxy groups having 5 or less carbon atoms and optionally substituted with a halogen atom. $R_{11}$ is preferably an alkyl group having 3 or less carbon atoms and more preferably a methyl group. Xb is preferably a hydrogen atom, a fluorine atom, a methyl group, a trifluoromethyl group, or a hydroxymethyl group.

Examples of the divalent linking group represented by L include an -Rt- group, a - CO- group, a -COO-Rt- group, a -COO-Rt-CO- group, an -Rt-CO- group, and an -O-Rt- group. In these formulas, Rt represents an alkylene group, a cycloalkylene group, or an aromatic ring group and is preferably an aromatic ring group.

L is preferably an -Rt- group, a -CO- group, a -COO-Rt-CO- group, or an -Rt-CO-group. Rt may have a substituent such as a halogen atom, a hydroxy group, or an alkoxy group. Rt is preferably an aromatic group.

The alkyl group represented by each of $Ry_1$ to $Ry_3$ is preferably an alkyl group having 1 to 4 carbon atoms such as a methyl group, an ethyl group, a n-propyl group, an isopropyl group, a n-butyl group, an isobutyl group, or a t-butyl group.

The cycloalkyl group represented by each of $Ry_1$ to $Ry_3$ is preferably a monocyclic cycloalkyl group such as a cyclopentyl group or a cyclohexyl group or a polycyclic cycloalkyl group such as a norbornyl group, a tetracyclodecanyl group, a tetracyclododecanyl group, or an adamantyl group.

The aryl group represented by each of $Ry_1$ to $Ry_3$ is preferably an aryl group having 6 to 10 carbon atoms, and examples thereof include a phenyl group, a naphthyl group, and an anthryl group.

The alkenyl group represented by each of $Ry_1$ to $Ry_3$ is preferably a vinyl group.

The alkynyl group represented by each of $Ry_1$ to $Ry_3$ is preferably an ethynyl group.

The cycloalkenyl group represented by each of $Ry_1$ to $Ry_3$ is preferably a structure including a monocyclic cycloalkyl group such as a cyclopentyl group or a cyclohexyl group with a double bond present in part of the monocyclic cycloalkyl group.

The cycloalkyl group formed by bonding two selected from the group consisting of $Ry_1$ to $Ry_3$ is preferably a monocyclic cycloalkyl group such as a cyclopentyl group or a cyclohexyl group or a polycyclic cycloalkyl group such as a norbornyl group, a tetracyclodecanyl group, a tetracyclododecanyl group, or an adamantyl group. In particular, the cycloalkyl group is more preferably a monocyclic cycloalkyl group having 5 to 6 carbon atoms.

In the cycloalkyl or cycloalkenyl group formed by bonding two selected from the group consisting of $Ry_1$ to Rys, for example, one methylene group included in the ring may be replaced with a heteroatom such as an oxygen atom, a group including a heteroatom such as a carbonyl group, an $-SO_2-$ group, or an $-SO_3-$ group, a vinylidene group, or a combination thereof. In the cycloalkyl or cycloalkenyl group, at least one ethylene group included in the cycloalkane or cycloalkenyl ring may be replaced with a vinylene group.

In the repeating unit represented by formula (B), it is preferable that, for example, $Ry_1$ is a methyl group, an ethyl group, a vinyl group, an allyl group, or an aryl group and that $Ry_2$ and $Rx_3$ are bonded together to form the cycloalkyl or cycloalkenyl group described above.

When any of the groups described above has a substituent, examples of the substituent include alkyl groups (having 1 to 4 carbon atoms), halogen atoms, a hydroxy group, alkoxy groups (having 1 to 4 carbon atoms), a carboxy group, and alkoxycarbonyl groups (having 2 to 6 carbon atoms). The number of carbon atoms in the substituent is preferably 8 or less.

The repeating unit represented by formula (B) is preferably an acid-decomposable (meth)acrylic acid tertiary ester-based repeating unit (a repeating unit in which Xb represents a hydrogen atom or a methyl group and L represents a -CO- group), an acid-decomposable hydroxystyrene tertiary alkyl ether-based repeating unit (a repeating unit in which Xb represents a hydrogen atom or a methyl group and L represents a phenyl group), or an acid-decomposable styrenecarboxylic acid tertiary ester-based repeating unit (a repeating unit in which Xb represents a hydrogen atom or a methyl group and L represents an -Rt-CO- group (Rt is an aromatic group)).

The content of the repeating unit having the acid-decomposable group including an unsaturated bond with respect to the total amount of the repeating units in the resin (A) is preferably 15% by mole or more, more preferably 20% by mole or more, and still more preferably 30% by mole or more. The upper limit of the content of the repeating unit with respect to the total amount of the repeating units in the resin (A) is preferably 80% by mole or less, more preferably 70% by mole or less, and particularly preferably 60% by mole or less.

Specific example of the repeating unit having the acid-decomposable group including an unsaturated bond are shown below, but the invention is not limited thereto. In the formulas below, Xb and L1 each represent any of the above-described substituents and linking groups, and Ar represents an aromatic group. R represents a substituent such as a hydrogen atom, an alkyl group, a cycloalkyl group, an aryl group, an aralkyl group, an alkenyl group, a hydroxy group, an alkoxy group, an acyloxy group, a cyano group, a nitro group, an amino group, a halogen atom, an ester group (-OCOR‴ or -COOR‴: R‴ represents an alkyl group having 1 to 20 carbon atoms or a fluorinated alkyl group having 1 to 20 carbon atoms), or a carboxy group, and R' represents a linear or branched alkyl group, a monocyclic or polycyclic cycloalkyl group, an alkenyl group, an alkynyl group, or a monocyclic or polycyclic aryl group. Q represents a heteroatom such as an oxygen atom, a group including a heteroatom such as a carbonyl group, an $-SO_2-$ group, or an $-SO_3-$ group, a vinylidene group, or a combination thereof. 1, n, and m each represent an integer of 0 or more.

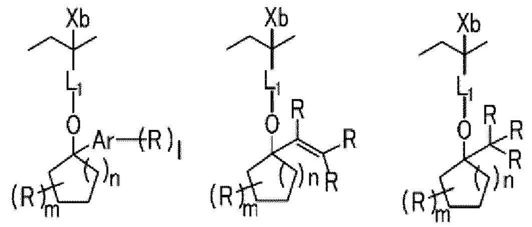

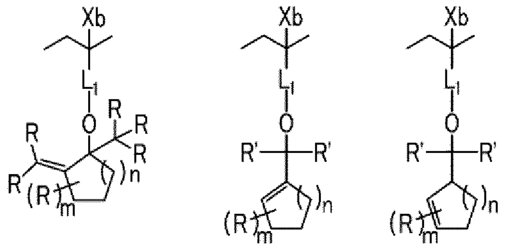

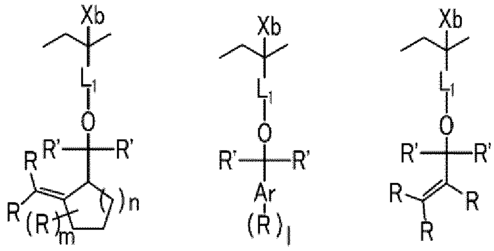

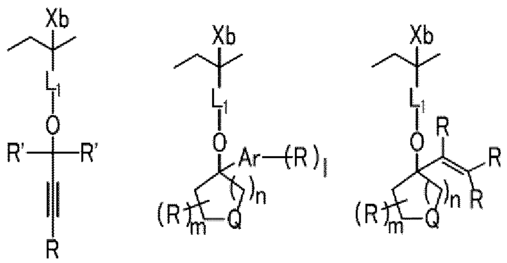

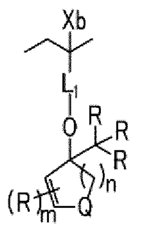

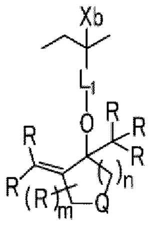

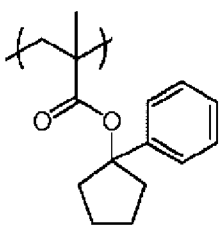

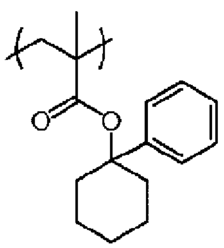

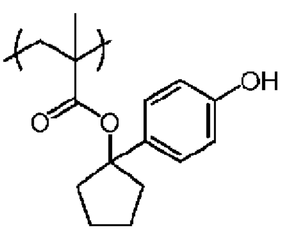

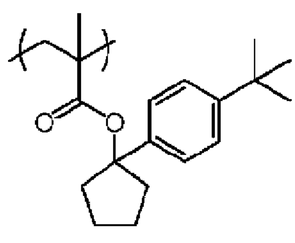

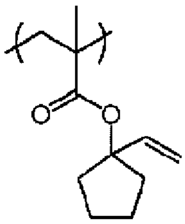
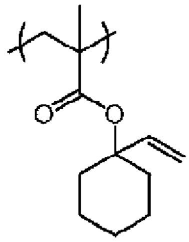
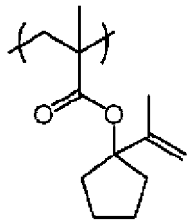
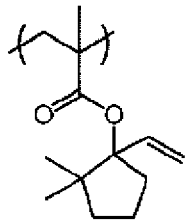
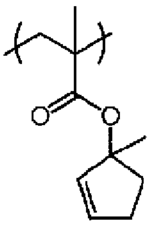
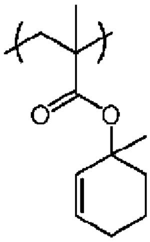
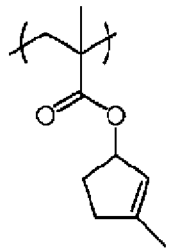
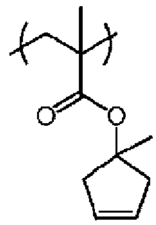
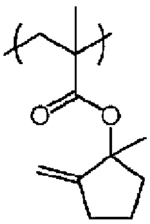
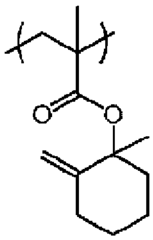
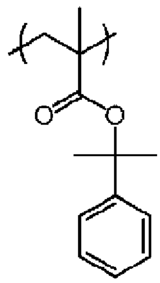
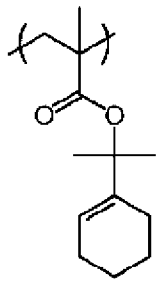
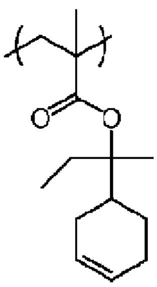
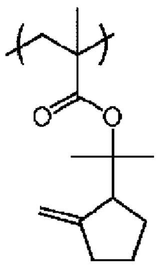
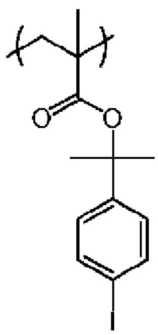
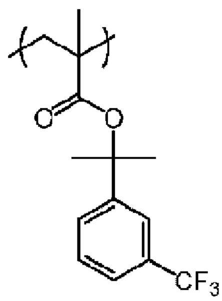
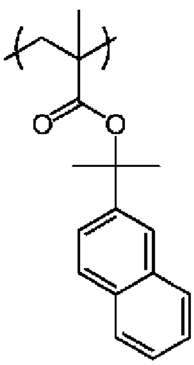
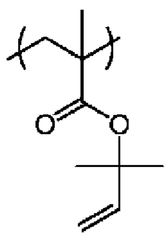
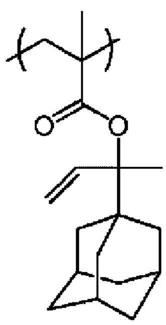
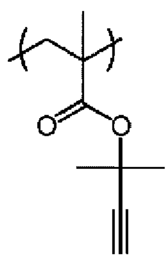

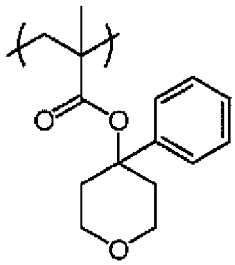
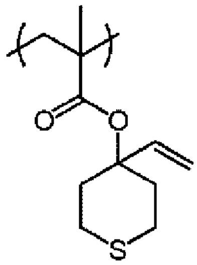
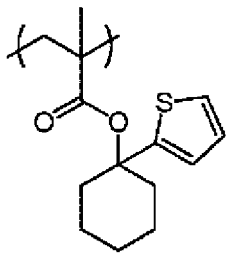
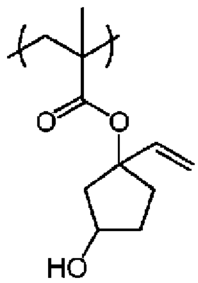

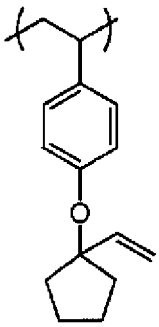
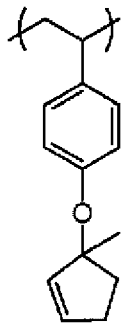
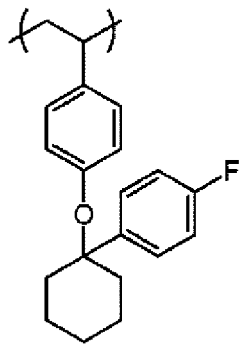
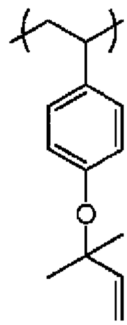

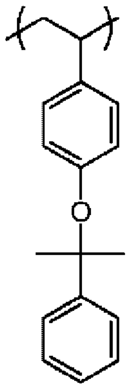
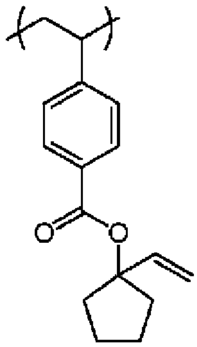
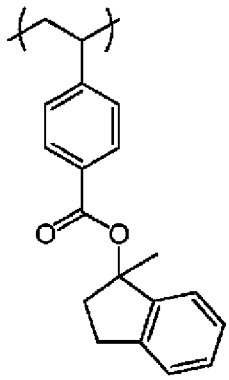
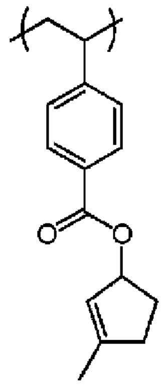

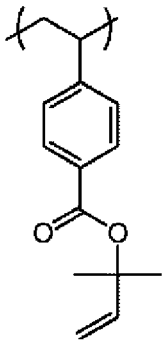
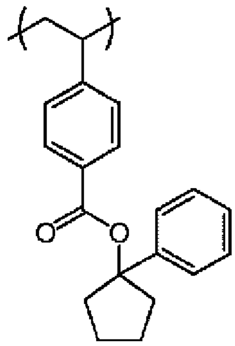
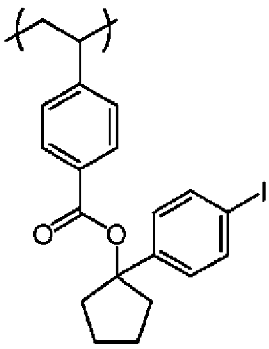
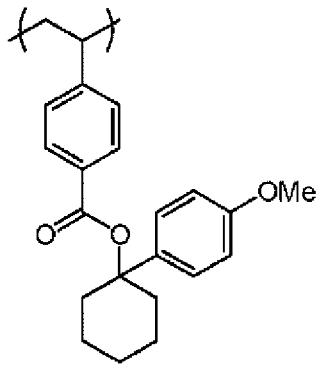

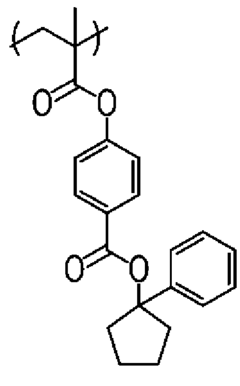
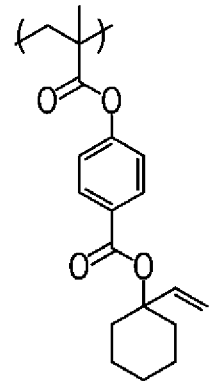
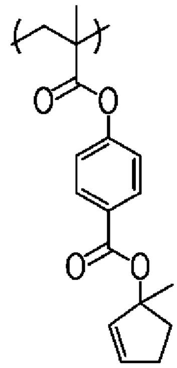

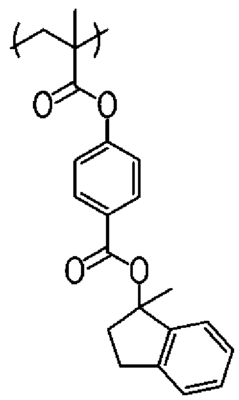
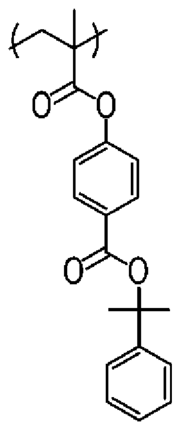
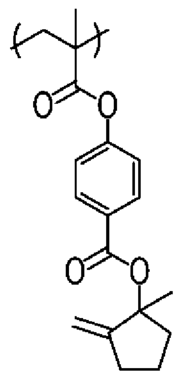

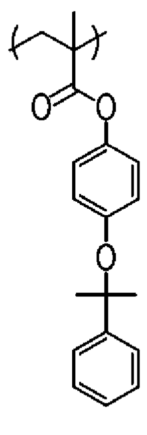
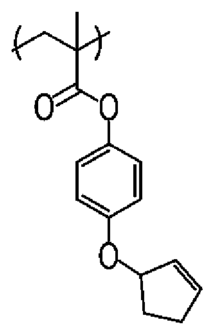

The content of the repeating unit having the acid-decomposable group with respect to the total amount of the repeating units in the resin (A) is preferably 15% by mole or more, more preferably 20% by mole or more, and still more preferably 30% by mole or more. The upper limit of the content of the repeating unit with respect to the total amount of the repeating units in the resin (A) is preferably 90% by mole or less, more preferably 80% by mole or less, still more preferably 70% by mole or less, and particularly preferably 60% by mole or less.

The resin (A) may include at least one repeating unit selected from the following group A and/or at least one repeating unit selected from the following group B.

Group A: The group consisting of the following repeating units (20) to (29).

(20) A repeating unit having an acid group, which will be described later.

(21) A repeating unit having no acid-decomposable group and no acid group but having a fluorine atom, a bromine atom, or an iodine atom, which will be described later.

(22) A repeating unit having a lactone group, a sultone group, or a carbonate group, which will be described later.

(23) A repeating unit having a photoacid generating group, which will be described later.

(24) A repeating unit represented by formula (V-1) or formula (V-2) below, which will be described later. (25) A repeating unit represented by formula (A), which will be described later.

(26) A repeating unit represented by formula (B), which will be described later.

(27) A repeating unit represented by formula (C), which will be described later.

(28) A repeating unit represented by formula (D), which will be described later. (29) A repeating unit represented by formula (E), which will be described later.

Group B: The group consisting of the following repeating units (30) to (32).

(30) A repeating unit having at least one group selected from the group consisting of lactone groups, sultone groups, carbonate groups, a hydroxy group, a cyano group, and alkali-soluble groups, which will be described later.

(31) A repeating unit having an alicyclic hydrocarbon structure and exhibiting no acid decomposability, which will be described later.

(32) A repeating unit represented by formula (III) and having no hydroxy group and no cyano group, which will be described later.

The resin (A) has preferably an acid group and includes preferably a repeating unit having an acid group as described later. The definition of the acid group will be described later along with preferred modes of the repeating unit having an acid group. When the resin (A) has the acid group, the interaction between the resin (A) and the acid generated from the photoacid generator is enhanced. This results in a further reduction in diffusion of the acid, and a pattern to be formed may have a sharper rectangular cross-sectional shape.

When the composition of the invention is used as an actinic ray-sensitive or radiation-sensitive resin composition for EUV light, it is preferable that the resin (A) has at least one repeating unit selected from the group A.

When the composition of the invention is used as an actinic ray-sensitive or radiation-sensitive resin composition for EUV light, it is preferable that the resin (A) includes at least one of a fluorine atom or an iodine atom. When the resin (A) includes both a fluorine atom and an iodine atom, the resin (A) may have one type of repeating unit including both a fluorine atom and an iodine atom or may include two types of repeating units including a repeating unit including a fluorine atom and a repeating unit including an iodine atom.

When the composition of the invention is used as an actinic ray-sensitive or radiation-sensitive resin composition for EUV light, it is also preferable that the resin (A) has a repeating unit having an aromatic group.

When the composition of the invention is used as an actinic ray-sensitive or radiation-sensitive resin composition for ArF light, it is preferable that the resin (A) has at least one type of repeating unit selected from the group B.

When the composition of the invention is used as an actinic ray-sensitive or radiation-sensitive resin composition for ArF light, it is preferable that the resin (A) includes no fluorine atom and no silicon atom.

When the composition of the invention is used as an actinic ray-sensitive or radiation-sensitive resin composition for ArF light, it is preferable that the resin (A) has no aromatic group.

(Repeating unit having acid group)

The resin (A) may have a repeating unit having an acid group.

The acid group is preferably an acid group having a pKa of 13 or less. The acid dissociation constant of the acid group is preferably 13 or less, more preferably 3 to 13, and still more preferably 5 to 10.

When the resin (A) has the acid group having a pKa of 13 or less, no particular limitation is imposed on the content of the acid group in the resin (A), but the content is often 0.2 to 6.0 mmol/g. In particular, the content is preferably 0.8 to 6.0 mmol/g, more preferably 1.2 to 5.0 mmol/g, and still more preferably 1.6 to 4.0 mmol/g. When the content of the acid group is within the above range, development proceeds smoothly, and a pattern to be formed has a good profile, so that high resolution is achieved.

The acid group is preferably, for example, a carboxy group, a phenolic hydroxy group, a fluorinated alcohol group (preferably a hexafluoroisopropanol group), a sulfonic group, a sulfonamido group, or an isopropanol group.

In the hexafluoroisopropanol group, one or more (preferably one to two) fluorine atoms may each be replaced with a group other than a fluorine atom (such as an alkoxycarbonyl group). The acid group is also preferably $-C(CF_3)(OH)-CF_2-$ formed as described above. At least one fluorine atom may be replaced with a group other than a fluorine atom to form a ring including $-C(CF_3)(OH)-CF_2-$.

Preferably, the repeating unit having the acid group is a repeating unit different from the above-described repeating unit having a structure in which a polar group is protected by a leaving group that leaves by the action of an acid and from a repeating unit having a lactone group, a sultone group, or a carbonate group that is described later.

The repeating unit having the acid group may have a fluorine atom or an iodine atom.

Examples of the repeating unit having the acid group include the following repeating units.

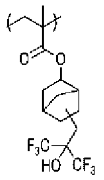
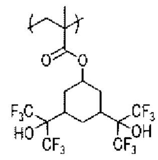
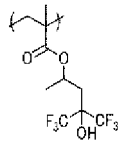

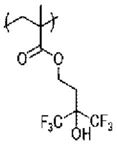
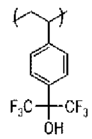
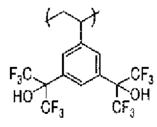

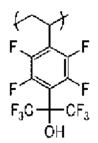
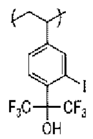
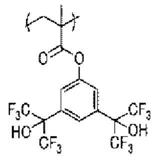
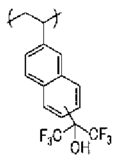

The repeating unit having the acid group is preferably a repeating unit represented by the following formula (1).

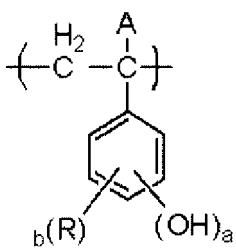
(1)

In formula (1), A represents a hydrogen atom, an alkyl group, a cycloalkyl group, a halogen atom, or a cyano group. R represents a halogen atom, an alkyl group, a cycloalkyl group, an aryl group, an alkenyl group, an aralkyl group, an alkoxy group, an alkylcarbonyloxy group, an alkylsulfonyloxy group, an alkyloxycarbonyl group, or an aryloxycarbonyl group. When a plurality of R's are present, they may be the same or different. When a plurality of R's are present, they may together form a ring. R is preferably a hydrogen atom. a represents an integer of from 1 to 3. b represents and integer of 0 to (5 - a).

Examples of the repeating unit having the acid group are shown below. In the following formulas, a represents 1 or 2.

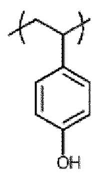
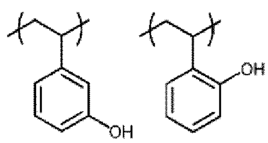

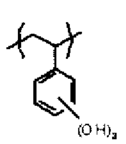
(B-1)

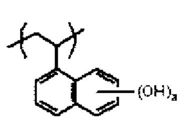
(B-2)

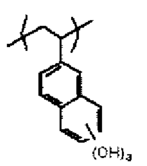
(B-3)

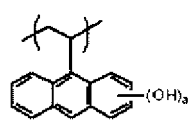
(B-4)

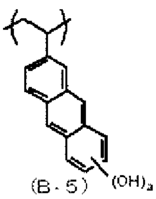
(B-5)

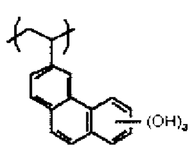
(B-6)
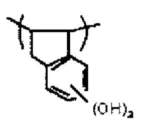
(B-7)
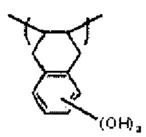
(B-8)
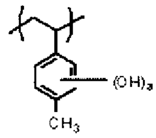
(B-9)
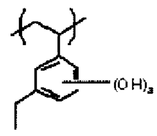
(B-10)

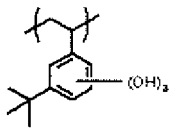
(B-11)
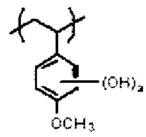
(B-12)
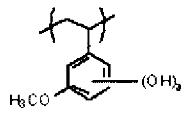
(B-13)
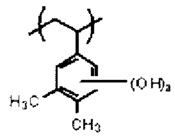
(B-14)
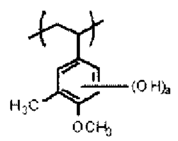
(B-15)

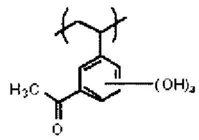
(B-16)
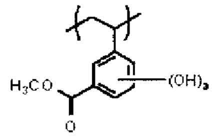
(B-17)
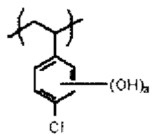
(B-18)
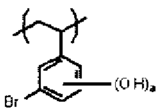
(B-19)
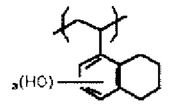
(B-20)

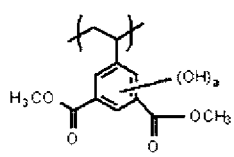
(B-21)
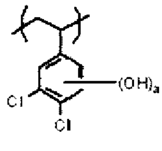
(B-22)
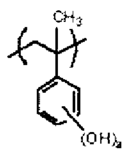
(B-23)
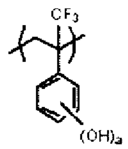
(B-24)
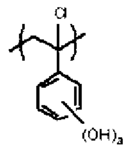
(B-25)

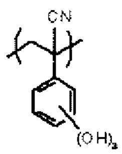
(B-26)
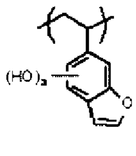
(B-27)
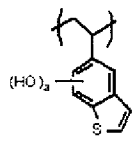
(B-28)
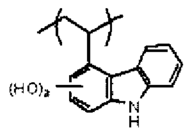
(B-29)
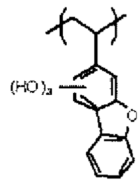
(B-30)

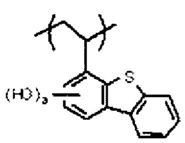
(B-31)
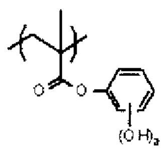
(B-32)
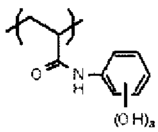
(B-33)
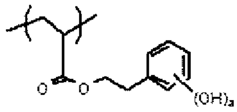
(B-34)

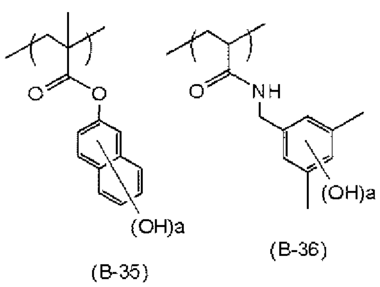
(B-35)
(B-36)
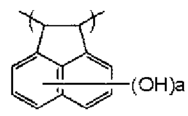
(B-37)
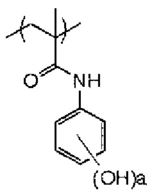
(B-38)

Among the above repeating units, repeating units specifically described below are preferred. In the following formulas, R represents a hydrogen atom or a methyl group, and a represents 2 or 3.

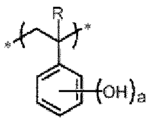 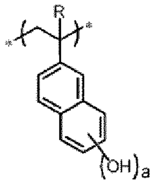 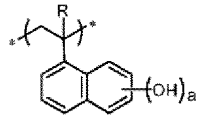 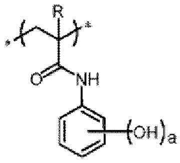

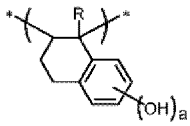 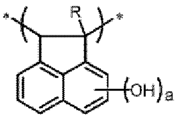 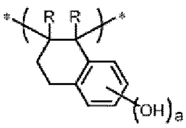 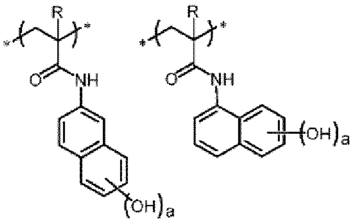

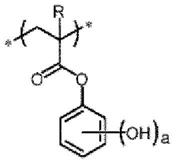 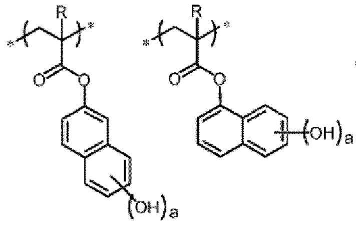 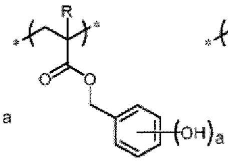 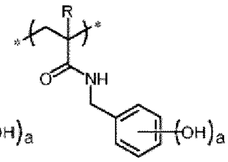

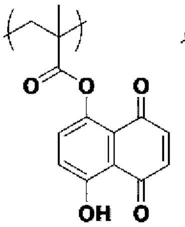 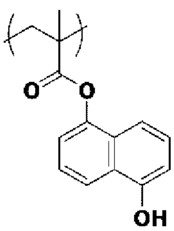 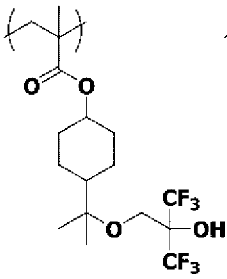 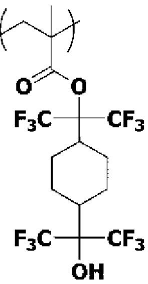 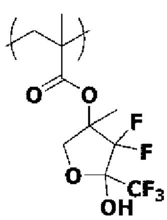 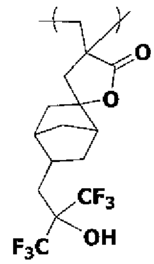

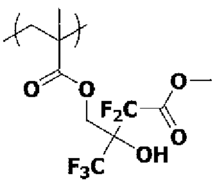 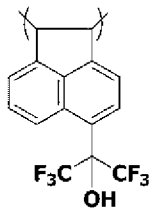 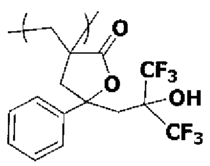 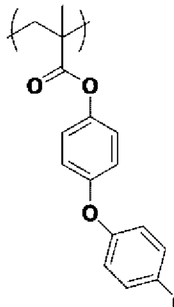 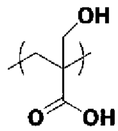 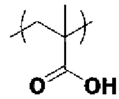

The content of the repeating unit having the acid group with respect to the total amount of the repeating units in the resin (A) is preferably 10% by mole or more and more preferably 15% by mole or more. The upper limit of the content with respect to the total amount of the repeating units in the resin (A) is preferably 70% by mole or less, more preferably 65% by mole or less, and still more preferably 60% by mole or less.

(Repeating unit having no acid-decomposable group and no acid group but having fluorine atom, bromine atom, or iodine atom)

The resin (A) may have, in addition to the above-described <repeating unit having the acid-decomposable group> and the above-described <repeating unit having the acid group>, a repeating unit having no acid-decomposable

group and no acid group but having a fluorine atom, a bromine atom, or an iodine atom (this repeating unit is hereinafter referred to also as a unit X). Preferably, the <repeating unit having no acid-decomposable group and no acid group but having a fluorine atom, a bromine atom, or an iodine atom> differs from other types of repeating units belonging to the group A such as the <repeating unit having a lactone group, a sultone group, or a carbonate group> described later and the <repeating unit having a photoacid generating group> described later.

The unit X is preferably a repeating unit represented by formula (C).

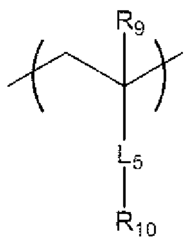

(C)

$L_5$ represents a single bond or an ester group. $R_9$ represents a hydrogen atom or an alkyl group optionally having a fluorine atom or an iodine atom. $R_{10}$ represents a hydrogen atom, an alkyl group optionally having a fluorine atom or an iodine atom, a cycloalkyl group optionally having a fluorine atom or an iodine atom, an aryl group optionally having a fluorine atom or an iodine atom, or a combination thereof.

Examples of the repeating unit having a fluorine atom or an iodine atom are shown below.

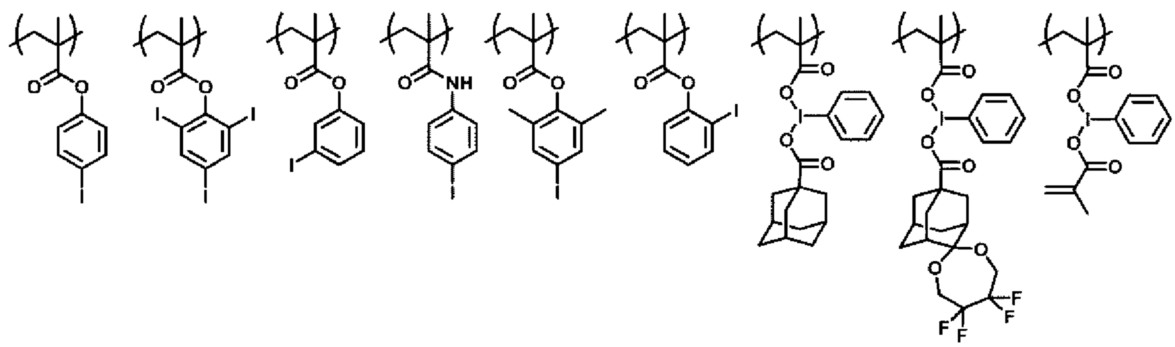

The content of the unit X with respect to the total amount of the repeating units in the resin (A) is preferably 0% by mole or more, more preferably 5% by mole or more, and still more preferably 10% by mole or more. The upper limit of the content of the unit X with respect to the total amount of the repeating units in the resin (A) is preferably 50% by mole or less, more preferably 45% by mole or less, and still more preferably 40% by mole or less.

Among the repeating units in the resin (A), the total amount of repeating units including at least one of a fluorine atom, a bromine atom, or an iodine atom with respect to the total amount of the repeating units in the resin (A) is preferably 10% by mole or more, more preferably 20% by mole or more, still more preferably 30% by mole or more, and particularly preferably 40% by mole or more. No particular limitation is imposed on the upper limit of the total amount, but the amount with respect to the total amount of the repeating units in the resin (A) is, for example, 100% by mole or less.

Examples of the repeating units including at least one of a fluorine atom, a bromine atom, or an iodine atom include: a repeating unit having a fluorine atom, a bromine atom, or an iodine atom and having the acid-decomposable group; a repeating unit having a fluorine atom, a bromine atom, or an iodine atom and having the acid group; and a repeating unit having a fluorine atom, a bromine atom, or an iodine atom.

(Repeating unit having lactone group, sultone group, or carbonate group)

The resin (A) may have a repeating unit having at least one selected from the group consisting of lactone groups, sultone groups, and carbonate groups (this repeating unit is hereafter referred to also as a "unit Y").

It is also preferable that the unit Y does not have a hydroxy group and an acid group such as a hexafluoropropanol group.

The lactone or sultone group may be any lactone or sultone group so long as it has a lactone or sultone structure. The lactone or sultone structure is preferably a 5- to 7-membered lactone or sultone structure. In particular, a 5- to 7-membered lactone structure with another ring structure fused thereto to form a bicyclo or spiro structure or a 5- to 7-membered sultone structure with another ring structure fused thereto to form a bicyclo or spiro structure is more preferred.

Preferably, the resin (A) has a repeating unit having a lactone or sultone group formed by removing at least one hydrogen atom from a ring member atom of a lactone structure represented by any of the following formulas (LC1-1) to (LC1-21) or a sultone structure represented by any of the following formulas (SL1-1) to (SL1-3).

The lactone or sultone group may be bonded directly to the main chain. For example, a ring member atom of the lactone or sultone group may be included in the main chain of the resin (A).

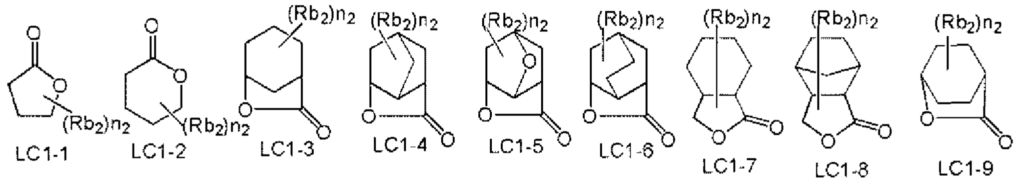

LC1-1 LC1-2 LC1-3 LC1-4 LC1-5 LC1-6 LC1-7 LC1-8 LC1-9

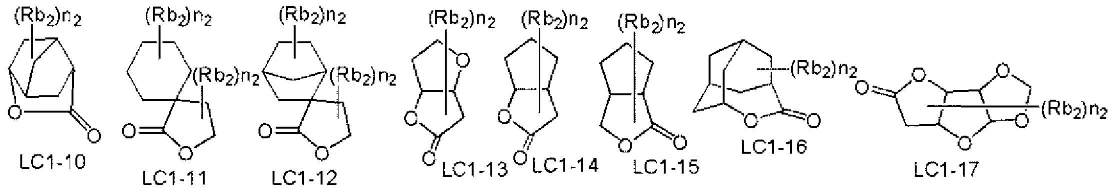

LC1-10 LC1-11 LC1-12 LC1-13 LC1-14 LC1-15 LC1-16 LC1-17

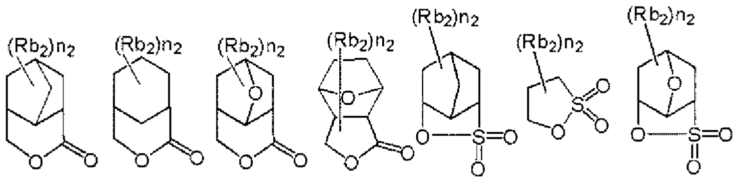

LC1-18 LC1-19 LC1-20 LC1-21 SL1-1 SL1-2 SL1-3

Each of the lactone and sultone structures may have a substituent ($Rb_2$). Preferred examples of the substituent (Rb2) include alkyl groups having 1 to 8 carbon atoms, cycloalkyl groups having 4 to 7 carbon atoms, alkoxy groups having 1 to 8 carbon atoms, alkoxycarbonyl groups having 1 to 8 carbon atoms, a carboxy group, halogen atoms, a cyano group, and acid-decomposable groups. $n_2$ represents an integer of from 0 to 4. A plurality of $Rb_2$'s present when $n_2$ is 2 or more may be different from each other, and the plurality of $Rb_2$'s present may be bonded together to form a ring.

Examples of the repeating unit having a group including the lactone structure represented by any of formulas (LC1-1) to (LC1-21) or the sultone structure represented by any of formulas (SL1-1) to (SL1-3) include a repeating unit represented by the following formula (AII).

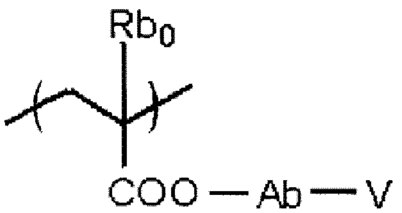

(AII)

In formula (AII), $Rb_0$ represents a hydrogen atom, a halogen atom, or an alkyl group having 1 to 4 carbon atoms. The alkyl group represented by $Rb_0$ may have a substituent, and preferred examples of the substituent include a hydroxy group and halogen atoms.

Examples of the halogen atom represented by $Rb_0$ include a fluorine atom, a chlorine atom, a bromine atom, and an iodine atom. $Rb_0$ is preferably a hydrogen atom or a methyl group.

Ab represents a single bond, an alkylene group, a divalent linking group having a monocyclic or polycyclic alicyclic hydrocarbon structure, an ether group, an ester group, a carbonyl group, a carboxy group, or a divalent group formed by combining any of the above groups. In particular, Ab is preferably a single bond or a linking group represented by $-Ab_1-CO_2-$. $Ab_1$ is a linear or branched alkylene group or a monocyclic or polycyclic cycloalkylene group and is preferably a methylene group, an ethylene group, a cyclohexylene group, an adamantylene group, or a norbornylene group.

V represents a group formed by removing one hydrogen atom from a ring member atom in the lactone structure

represented by any of formulas (LC1-1) to (LC1-21) or a group formed by removing one hydrogen atom from a ring member atom in the sultone structure represented by any of formulas (SL1-1) to (SL1-3).

When the repeating unit having the lactone or sultone group has optical isomers, any of the optical isomers may be used. One optical isomer may be used alone, or a mixture of a plurality of optical isomers may be used. When one optical isomer is mainly used, the optical purity (ee) thereof is preferably 90% or more and more preferably 95% or more.

The carbonate group is preferably a cyclic carbonate group.

The repeating unit having a cyclic carbonate group is preferably a repeating unit represented by the following formula (A-1).

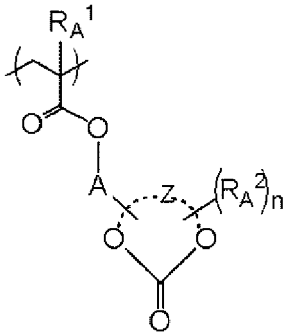

(A-1)

In formula (A-1), $R_A{}^1$ represents a hydrogen atom, a halogen atom, or a monovalent organic group (preferably a methyl group). n represents an integer of 0 or more. $R_A{}^2$ represents a substituent. A plurality of $R_A{}^2$'s present when n is 2 or more may be the same or different. A represents a single bond or a divalent linking group. The divalent linking group is preferably an alkylene group, a divalent linking group having a monocyclic or polycyclic alicyclic hydrocarbon structure, an ether group, an ester group, a carbonyl group, a carboxy group, or a divalent group formed by combining any of them. Z represents an atomic group forming a monocyclic or polycyclic ring together with a group represented by -O-CO-O- in formula (A-1).

Examples of the unit Y are shown below. In these formulas, Rx represents a hydrogen atom, $-CH_3$, $-CH_2OH$, or $-CF_3$.

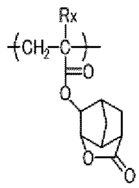 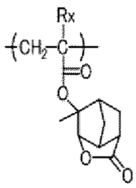 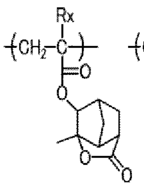 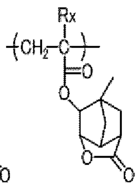

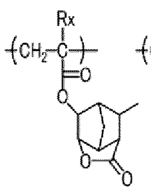 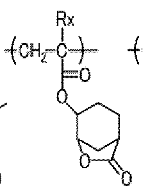 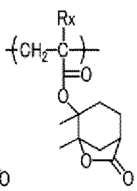 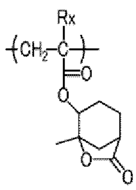

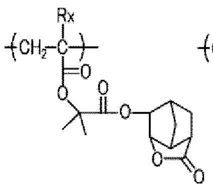 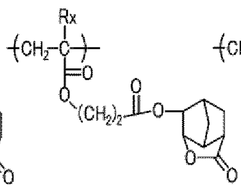 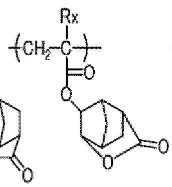 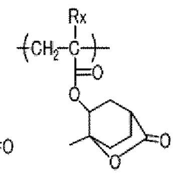

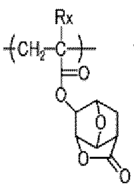 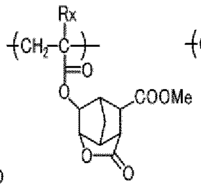 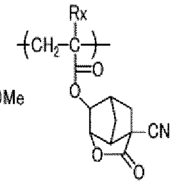 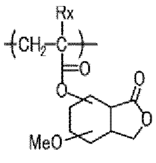

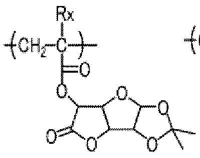 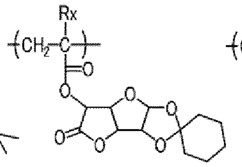 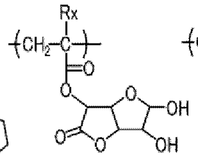 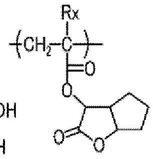

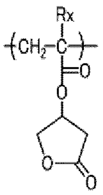 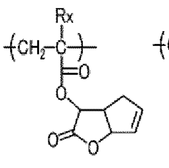 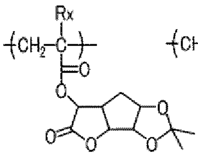 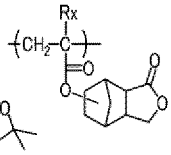 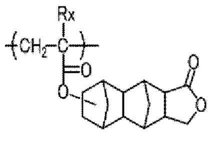

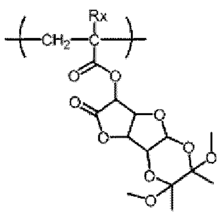 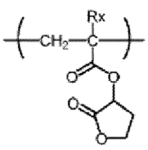 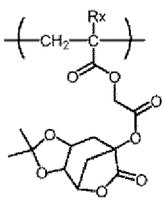 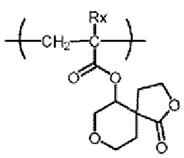

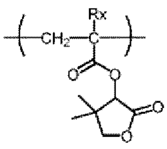 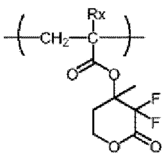 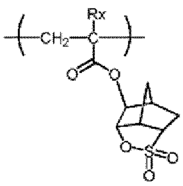 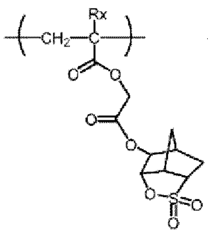 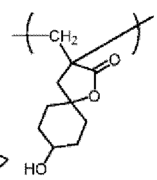

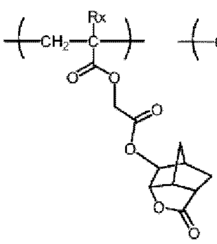 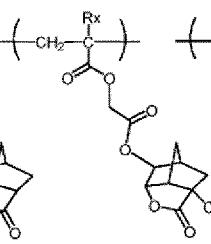 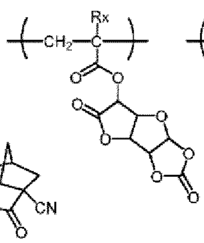 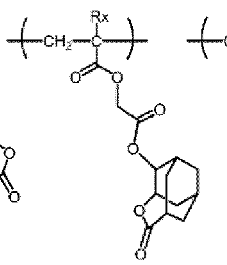 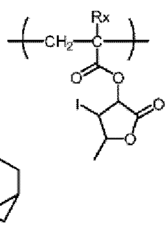

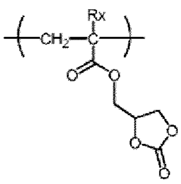 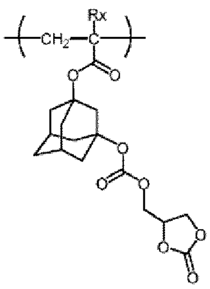

The content of the unit Y with respect to the total amount of the repeating units in the resin (A) is preferably 1% by mole or more and more preferably 10% by mole or more. The upper limit of the content of the unit Y with respect to the total amount of the repeating units in the resin (A) is preferably 85% by mole or less, more preferably 80% by mole or less, still more preferably 70% by mole or less, and particularly preferably 60% by mole or less.

(Repeating unit having photoacid generating group)

The resin (A) may include a repeating unit that is different from those described above and has a group that generates an acid when irradiated with actinic rays or radiation (this group is hereinafter referred to also as a "photoacid generating group").
Examples of the repeating unit having the photoacid generating group include a repeating unit represented by formula (4).

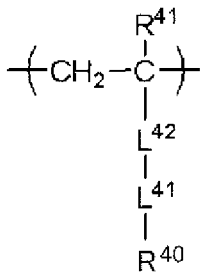

(4)

$R^{41}$ represents a hydrogen atom or a methyl group. $L^{41}$ represent a single bond or a divalent linking group. $L^{42}$ represents a divalent linking group. $R^{40}$ represents a structural moiety that is decomposed when irradiated with actinic rays or radiation and thereby generates an acid on a side chain.
Examples of the repeating unit having the photoacid generating group are shown below.

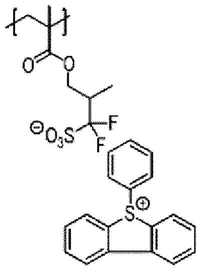
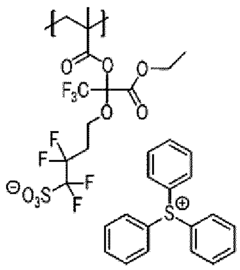
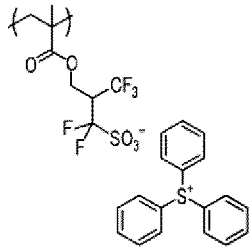

Other examples of the repeating unit represented by formula (4) include repeating units described in paragraphs [0094] to [0105] of JP2014-041327A and repeating units described in paragraph [0094] of WO2018/193954A.
The content of the repeating unit having the photoacid generating group with respect to the total amount of the repeating units in the resin (A) is preferably 1% by mole or more and more preferably 5% by mole or more. The upper limit of the content with respect to the total amount of the repeating units in the resin (A) is preferably 40% by mole or less, more preferably 35% by mole or less, and still more preferably 30% by mole or less.

(Repeating unit represented by formula (V-1) or formula (V-2))

The resin (A) may have a repeating unit represented by formula (V-1) or (V-2) below.
Preferably, the repeating unit represented by the following formula (V-1) or (V-2) differs from the repeating units described above.

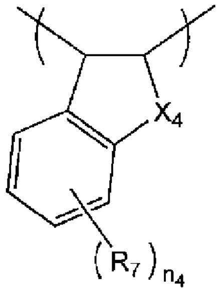

(V-1)

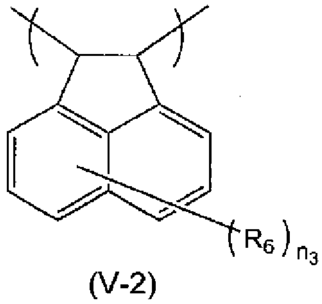

(V-2)

In these formulas, $R_6$ and $R_7$ each independently represent a hydrogen atom, a hydroxy group, an alkyl group, an alkoxy group, an acyloxy group, a cyano group, a nitro group, an amino group, a halogen atom, an ester group (-OCOR or -COOR: R represents an alkyl group having 1 to 6 carbon atoms or a fluorinated alkyl group having 1 to 6 carbon atoms), or a carboxy group. The alkyl group is preferably a linear, branched, or cyclic alkyl group having 1 to 10 carbon atoms.

$n_3$ represents an integer of from 0 to 6.

$n_4$ represents an integer of from 0 to 4.

$X^4$ is a methylene group, an oxygen atom, or a sulfur atom.

Examples of the repeating unit represented by formula (V-1) or (V-2) are shown below.

Examples of the repeating unit represented by formula (V-1) or (V-2) include repeating units described in paragraph [0100] of WO2018/193954A.

(Repeating unit for reducing mobility of main chain)

The higher the glass transition temperature (Tg) of the resin (A), the better because excessive diffusion of the acid generated or pattern collapse during development can be prevented. The Tg is preferably higher than 90°C, more preferably higher than 100°C, still more preferably higher than 110°C, and particularly preferably higher than 125°C. The Tg is preferably 400°C or lower and more preferably 350°C or lower because the rate of dissolution in a developer is high.

In the present specification, the glass transition temperature (Tg) of a polymer such as the resin (A) (hereinafter referred to as the "Tg of a repeating unit") is computed by the following method. First, the Tg of each of the homopolymers formed from the respective repeating units included in the polymer is computed by the Bicerano method. Next, the mass ratios (%) of the repeating units with respect to the total mass of the repeating units in the polymer are computed. Next, the Tg of each repeating unit at the corresponding mass ratio is computed using the Fox formula (described, for example, in Materials Letters 62 (2008) 3152), and the computed Tg's are summed to obtain the Tg (°C) of the polymer.

The Bicerano method is described in Prediction of polymer properties, Marcel Dekker Inc, New York (1993). The computation of Tg by the Bicerano method can be performed using software for estimating physical properties of a polymer, MDL Polymer (MDL Information Systems, Inc.).

To increase the Tg of the resin (A) (to increase the Tg to preferably higher than 90°C), it is preferable to reduce the mobility of the main chain of the resin (A). Examples of a method for reducing the mobility of the main chain of the resin (A) include methods (a) to (e) described below. (a) Introduction of a bulky substituent into the main chain. (b) Introduction of a plurality of substituents into the main chain. (c) Introduction of a substituent that induces the interaction between molecules of the resin (A) into the vicinities of their main chains. (d) Formation of the main chain having a ring structure. (e) Linkage of a ring structure to the main chain

Preferably, the resin (A) has a repeating unit whose homopolymer has a Tg of 130°C or higher.

No particular limitation is imposed on the type of repeating unit whose homopolymer has a Tg of 130°C or higher, and any repeating unit can be used so long as the Tg of the homopolymer computed by the Bicerano method is 130°C or higher. With repeating units represented by formulas (A) to (E) described below, homopolymers formed from the repeating units can have a Tg of 130°C or higher, but this depends on the types of functional groups in the repeating units.

One specific example of means for achieving the method (a) is a method in which the repeating unit represented by formula (A) is introduced into the resin (A).

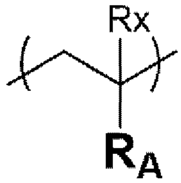

(A)

In Formula (A), $R_A$ represents a group including a polycyclic structure. $R_x$ represents a hydrogen atom, a methyl group, or an ethyl group. The group including the polycyclic structure is a group including a plurality of ring structures, and the plurality of ring structures may or may not be fused.

Specific examples of the repeating unit represented by formula (A) include those described in paragraphs [0107] to [0119] of WO2018/193954A.

One specific example of means for achieving the method (b) is a method in which the repeating unit represented by formula (B) is introduced into the resin (A).

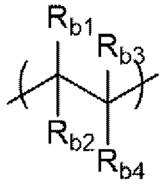

(B)

In formula (B), $R_{b1}$ to $R_{b4}$ each independently represent a hydrogen atom or an organic group, and at least two selected from the group consisting of $R_{b1}$ to $R_{b4}$ each represent an organic group.

When at least one of the organic groups is a group whose ring structure is linked directly to the main chain of the repeating unit, no particular limitation is imposed on the types of other organic groups.

When each of the organic groups is not a group whose ring structure is linked directly to the main chain of the repeating unit, at least two of the organic groups are each a substituent in which the number of constituent atoms excluding hydrogen atoms is 3 or more.

Specific examples of the repeating unit represented by formula (B) include those described in paragraphs [0113] to [0115] of WO2018/193954A.

One specific example of means for achieving the method (c) is a method in which the repeating unit represented by formula (C) is introduced into the resin (A).

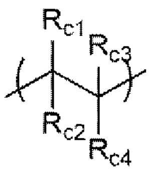

(C)

In formula (C), $R_{c1}$ to $R_{c4}$ each independently represent a hydrogen atom or an organic group, and at least one of $R_{c1}$ , $R_{c2}$, $R_{c3}$, or $R_{c4}$ is a group including a hydrogen-bonding hydrogen atom at a position within 3 atoms from a carbon atom in the main chain. In particular, it is preferable that the hydrogen-bonding hydrogen atom is present at a position within two atoms (at a position closer to the main chain) in order to induce the interaction between the main chains of molecules of the resin (A).

Specific examples of the repeating unit represented by formula (C) include those described in paragraphs [0119] to [0121] of WO2018/193954A.

One specific example of means for achieving the method (d) is a method in which the repeating unit represented by formula (D) is introduced into the resin (A).

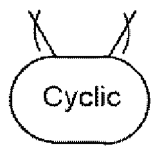

(D)

In formula D, "cylic" represents a group having a ring structure forming the main chain. No particular limitation

is imposed on the number of atoms forming the ring.

Specific examples of the repeating unit represented by formula (D) include those described in paragraphs [0126] to [0127] of WO2018/193954A.

One specific example of means for achieving the method (e) is a method in which the repeating unit represented by formula (E) is introduced into the resin (A).

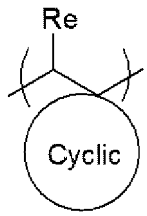

(E)

In formula (E), Re's each independently represent a hydrogen atom or an organic group. Examples of the organic group include alkyl groups, cycloalkyl groups, aryl groups, aralkyl groups, and alkenyl groups, each of which may have a substituent.

"Cyclic" is a cyclic group including a carbon atom included in the main chain. No particular limitation is imposed on the number of atoms included in the cyclic group.

Specific examples of the repeating unit represented by formula (E) include those described in paragraphs [0131] to [0133] of WO2018/193954A.

(Repeating unit having at least one group selected from group consisting of lactone groups, sultone groups, carbonate groups, hydroxy group, cyano group, and alkali-soluble groups)

The resin (A) may have a repeating unit having at least one group selected from the group consisting of lactone groups, sultone groups, carbonate groups, a hydroxy group, a cyano group, and alkali-soluble groups.

The repeating unit having a lactone group, a sultone group, or a carbonate group and included in the resin (A) may be any of the repeating units described above for the <repeating unit having a lactone group, a sultone group, or a carbonate group>. A preferred content of the repeating unit is also as described above for the <repeating unit having a lactone group, a sultone group, or a carbonate group>.

The resin (A) may have a repeating unit having a hydroxy group or a cyano group. In this case, the adhesiveness to a substrate and the affinity for a developer are improved.

The repeating unit having a hydroxy group or a cyano group is preferably a repeating unit having an alicyclic hydrocarbon structure substituted with a hydroxy group or a cyano group.

Preferably, the repeating unit having a hydroxy group or a cyano group has no acid-decomposable group. Examples of the repeating unit having a hydroxy group or a cyano group include those described in paragraphs [0081] to [0084] of JP2014-098921A.

The resin (A) may have a repeating unit having an alkali-soluble group.

Examples of the alkali-soluble group include a carboxy group, a sulfonamido group, a sulfonylimido group, a bissulfonylimido group, and aliphatic alcohols substituted with an electron-withdrawing group at the $\alpha$-position (e.g., a hexafluoroisopropanol group), and the alkali-soluble group is preferably a carboxy group. When the resin (A) includes the repeating unit having an alkali-soluble group, resolution in contact hole applications is increased. Examples of the repeating unit having an alkali-soluble group include those described in paragraphs [0085] and [0086] of JP2014-098921A.

(Repeating unit having alicyclic hydrocarbon structure and exhibiting no acid decomposability)

The resin (A) may have a repeating unit having an alicyclic hydrocarbon structure and exhibiting no acid decomposability. In this case, elution of a low-molecular weight component from the resist film to an immersion liquid during liquid immersion exposure can be reduced. Examples of such a repeating unit include repeating units derived from 1-adamantyl (meth)acrylate, diamantyl (meth)acrylate, tricyclodecanyl (meth)acrylate, and cyclohexyl (meth)acrylate.

(Repeating unit represented by formula (III) and having no hydroxy group and no cyano group)

The resin (A) may have a repeating unit represented by formula (III) and having no hydroxy group and no cyano group.

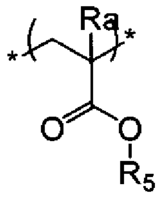

(III)

In formula (III), $R_5$ represents a hydrocarbon group having at least one ring structure and having no hydroxy group and no cyano group.

Ra represents a hydrogen atom, an alkyl group, or a $-CH_2-O-Ra_2$ group. In this formula, $Ra_2$ represents a hydrogen atom, an alkyl group, or an acyl group.

Examples of the repeating unit represented by formula (III) and having no hydroxy group and no cyano group include those described in paragraphs [0087] to [0094] of JP2014-098921A.

(Additional repeating units)

The resin (A) may further have a repeating unit other than the repeating units described above.

For example, the resin (A) may have a repeating unit selected from the group consisting of a repeating unit having an oxathiane ring group, a repeating unit having an oxazolone ring group, a repeating unit having a dioxane ring group, and a repeating unit having a hydantoin ring group.

Examples of such a repeating unit are shown below.

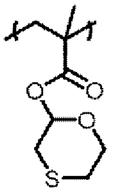

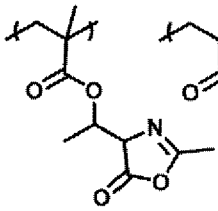

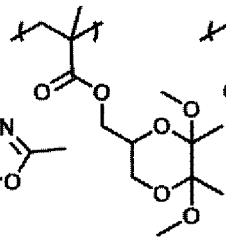

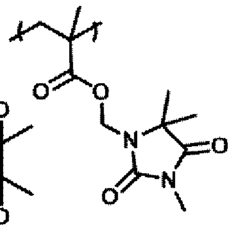

The resin (A) may have, in addition to the repeating structural units described above, various repeating structural units for the purpose of controlling dry etching resistance, suitability for a standard developer, adhesiveness to a substrate, a resist profile, resolution, heat resistance, sensitivity, etc.

Preferably, in the resin (A) (particularly when the composition is used as an actinic ray-sensitive or radiation-sensitive resin composition for ArF light), all the repeating units are composed of repeating units derived from compounds having an ethylenically unsaturated bond. In particular, it is also preferable that all the repeating units are composed of (meth)acrylate-based repeating units. In the resin (A) used in this case, all the repeating units may be methacrylate-based repeating units, or all the repeating units may be acrylate-based repeating units. Alternatively, the repeating units may each be a methacrylate-based repeating unit or an acrylate-based repeating unit. It is preferable that the content of the acrylate-based repeating units with respect to the total amount of the repeating units is 50% by mole or less.

The resin (A) can be synthesized by a routine method (for example, radical polymerization).

The weight average molecular weight of the resin (A) that is determined as a polystyrene-equivalent value by the GPC method is preferably 30,000 or less, more preferably 1,000 to 30,000, still more preferably 3,000 to 30,000, and particularly preferably 5,000 to 15,000.

The dispersity (molecular weight distribution) of the resin (A) is preferably 1 to 5, more preferably 1 to 3, still more preferably 1.2 to 3.0, and particularly preferably 1.2 to 2.0. The smaller the dispersity, the better the resolution and the resist profile, and the smoother the side surfaces of the resist pattern, so that better roughness quality is obtained.

In the composition of the invention, the content of the resin (A) with respect to the total amount of the solids in the composition of the invention is preferably 10.0 to 99.5% by mass, more preferably 35.0 to 99.0% by mass, still more preferably 40.0 to 98.0% by mass, and particularly preferably 60.0 to 90.0% by mass.

Ony type of resin (A) may be used alone, or a combination of a plurality of types may be used.

<Photoacid generator (B)>

The composition of the invention may further include a photoacid generator (B) in addition to the compound represented by general formula (S1) described above.

The photoacid generator (B) may be in the form of a low-molecular weight compound or may be in the form in which the photoacid generator (B) is incorporated into part of a polymer (e.g., the resin (A)). A combination of the form of a low-molecular-weight compound and the form in which the photoacid generator (B) is incorporated into part of a polymer may also be used.

When the photoacid generator (B) is in the form of a low-molecular weight compound, the molecular weight of the photoacid generator is preferably 3000 or less, more preferably 2000 or less, and still more preferably 1000 or less. No particular limitation is imposed on the lower limit of the molecular weight, but the molecular weight is 100 or more.

When the photoacid generator (B) is in the form in which the photoacid generator (B) is incorporated into part of a polymer, the photoacid generator (B) may be incorporated into part of the resin (A) or into a resin different from the resin (A).

In the present invention, it is preferable that the photoacid generator (B) is in the form of a low-molecular weight compound.

The photoacid generator (B) is, for example, a compound (onium salt) represented by "$M^+X^-$" and is preferably a compound that generates an organic acid upon exposure to light.

Examples of the organic acid include sulfonic acids (such as aliphatic sulfonic acids, aromatic sulfonic acids, and camphorsulfonic acid), carboxylic acids (such as aliphatic carboxylic acids, aromatic carboxylic acids, and aralkyl carboxylic acids), carbonylsulfonylimidic acid, bis(alkylsulfonyl)imidic acids, and tris(alkylsulfonyl)methide acids.

In the compound represented by "$M^+X^-$," $M^+$ represents an organic cation. Specific examples of the organic cation and its preferred ranges are the same as those of the organic cation represented by the above-described $M^+$ in general formula (S1).

In the compound represented by "$M^+X^-$," $X^-$ represents an organic anion.

No particular limitation is imposed on the organic anion, and examples thereof include monovalent organic anions and divalent and higher valent organic anions.

The organic anion is preferably an anion whose ability to cause a nucleophilic reaction is very low and is more preferably a non-nucleophilic anion.

Examples of the non-nucleophilic anion include sulfonate anions (such as aliphatic sulfonate anions, aromatic sulfonate anions, and a camphorsulfonate anion), carboxylate anions (such as aliphatic carboxylate anions, aromatic carboxylate anions, and aralkyl carboxylate anions), sulfonylimide anions, bis(alkylsulfonyl)imide anions, and tris(alkylsulfonyl)methide anions.

In the aliphatic sulfonate anions and the aliphatic carboxylate anions, the aliphatic moiety may be a linear or branched alkyl group or a cycloalkyl group and is preferably a linear or branched alkyl group having 1 to 30 carbon atoms or a cycloalkyl group having 3 to 30 carbon atoms.

The alkyl group may be, for example, a fluoroalkyl group (which may have a substituent other than a fluorine atom or may be a perfluoroalkyl group).

In the aromatic sulfonate anions and the aromatic carboxylate anions, the aryl group is preferably an aryl group having 6 to 14 carbon atoms such as a phenyl group, a tolyl group, or a naphthyl group.

The above-described alkyl, cycloalkyl, and aryl groups may each have a substituent. No particular limitation is imposed on the substituent, and examples thereof include a nitro group, halogen atoms such as a fluorine atom and a chlorine atom, a carboxy group, a hydroxy group, an amino group, a cyano group, alkoxy groups (having preferably 1 to 15 carbon atoms), alkyl groups (having preferably 1 to 10 carbon atoms), cycloalkyl groups (having preferably 3 to 15 carbon atoms), aryl groups (having preferably 6 to 14 carbon atoms), alkoxycarbonyl groups (having preferably 2 to 7 carbon atoms), acyl groups (having preferably 2 to 12 carbon atoms), alkoxycarbonyloxy groups (having preferably 2 to 7 carbon atoms), alkylthio groups (having preferably 1 to 15 carbon atoms), alkylsulfonyl groups (having preferably 1 to 15 carbon atoms), alkyliminosulfonyl groups (having preferably 1 to 15 carbon atoms), and aryloxysulfonyl groups (having preferably 6 to 20 carbon atoms).

In the aralkyl carboxylate anions, the aralkyl group is preferably an aralkyl group having 7 to 14 carbon atoms.

Examples of the aralkyl group having 7 to 14 carbon atoms include a benzyl group, a phenethyl group, a naphthylmethyl group, a naphthylethyl group, and a naphthylbutyl group.

Examples of the sulfonylimide anion include a saccharin anion.

In the bis(alkylsulfonyl)imide anions and the tris(alkylsulfonyl)methide anions, the alkyl group is preferably an alkyl group having 1 to 5 carbon atoms. These alkyl groups may have a substituent, and examples of the substituent include halogen atoms, alkyl groups substituted with halogen atoms, alkoxy groups, alkylthio groups, alkyloxysulfonyl groups, aryloxysulfonyl groups, and cycloalkylaryloxysulfonyl groups. The substituent is preferably a fluorine atom or an alkyl group substituted with a fluorine atom.

In the bis(alkylsulfonyl)imide anions, the alkyl groups may be bonded together to form a ring structure. In this case, the strength of the acid increases.

Other examples of the non-nucleophilic anion include phosphorus fluoride (such as $PF_6$ ), boron fluoride (such as $BF_4^-$), and antimony fluoride (such as $SbF_6^-$).

The non-nucleophilic anion is preferably an aliphatic sulfonate anion substituted with a fluorine atom at least at the $\alpha$-position of the sulfonic acid, an aromatic sulfonate anion substituted with a fluorine atom or a fluorine atom-containing group, a bis(alkylsulfonyl)imide anion in which an alkyl group is substituted with a fluorine atom, or a tris(alkylsulfonyl)methide anion in which an alkyl group is substituted with a fluorine atom. In particular, the non-nucleophilic anion is more preferably a perfluoroaliphatic sulfonate anion (having preferably 4 to 8 carbon atoms) or a benzenesulfonate anion having a fluorine atom and still more preferably a nonafluorobutanesulfonate anion, a perfluorooctanesulfonate anion, a pentafluorobenzenesulfonate anion, or a 3,5-bis(trifluoromethyl)benzenesulfonate anion.

The non-nucleophilic anion is also preferably an anion represented by the following formula (AN1).

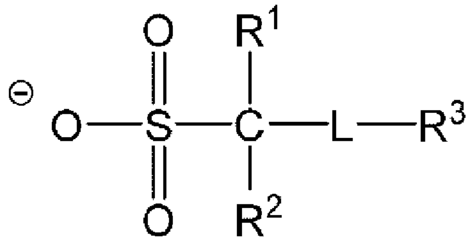

(AN1)

In formula (AN1), $R^1$ and $R^2$ each independently represent a hydrogen atom or a substituent.

No particular limitation is imposed on the substituent, but the substituent is preferably a group other than electron-withdrawing groups. Example of the group other than electron-withdrawing groups include hydrocarbon groups, a hydroxy group, oxyhydrocarbon groups, oxycarbonyl hydrocarbon groups, an amino group, hydrocarbon-substituted amino groups, and hydrocarbon-substituted amido groups.

Preferably, these groups other than electron-withdrawing groups are each independently -R', -OH, -OR', -OCOR', $-NH_2$, $-NR'_2$, -NHR', or -NHCOR'. R' is a monovalent hydrocarbon group.

Examples of the monovalent hydrocarbon group represented by R' include: linear or branched monovalent hydrocarbon groups including alkyl groups such as a methyl group, an ethyl group, a propyl group, and a butyl group, alkenyl groups such as an ethenyl group, a propenyl group, and a butenyl group, and alkynyl groups such as an ethynyl group, a propynyl group, and a butynyl group; monovalent alicyclic hydrocarbon groups including cycloalkyl groups such as a cyclopropyl group, a cyclobutyl group, a cyclopentyl group, a cyclohexyl group, a norbornyl group, and a adamantyl group and cycloalkenyl groups such as a cyclopropenyl group, a cyclobutenyl group, a cyclopentenyl group, and a norbornenyl group; and monovalent aromatic hydrocarbon groups including aryl groups such as a phenyl group, a tolyl group, a xylyl group, a mesityl group, a naphthyl group, a methylnaphthyl group, an anthryl group, and a methylanthryl group and aralkyl groups such as a benzyl group, a phenethyl group, a phenylpropyl group, a naphthylmethyl group, and an anthrylmethyl group.

In particular, it is preferable that $R^1$ and $R^2$ each independently represent a hydrocarbon group (preferably a cycloalkyl group) or a hydrogen atom.

L represents a divalent linking group.

When a plurality of L's are present, they may be the same or different.

Examples of the divalent linking group include -O-CO-O-, -COO-, -CONH-, -CO-, -O-, -S-, -SO-, $-SO_2-$, alkylene groups (having preferably 1 to 6 carbon atoms), cycloalkylene groups (having preferably 3 to 15 carbon atoms), alkenylene groups (having preferably 2 to 6 carbon atoms), and divalent linking groups formed by combining any of these groups. In particular, the divalent linking group is preferably -O-CO-O-, -COO-, -CONH-, -CO-, -O-, $-SO_2-$, -O-CO-O-alkylene group-, -COO-alkylene group-, or -CONH-alkylene group- and more preferably -O-CO-O-, -O-CO-O-alkylene group-, -COO-, -CONH-, $-SO_2-$, or -COO-alkylene group-.

Preferably, L is, for example, a group represented by the following formula (AN1-1).

$$*^{a}-(CR^{2a}_2)_X-Q-(CR^{2b}_2)_Y-*^{b} \qquad \text{(AN1-1)}$$

In formula (AN1-1), $*^a$ represents a bonding position to $R^3$ in formula (AN1).

$*^b$ represents a bonding position to $-C(R^1)(R^2)-$ in formula (AN1).

X and Y each independently represent an integer of from 0 to 10 and preferably an integer of 0 to 3.

$R^{2a}$ and $R^{2b}$ each independently represent a hydrogen atom or a substituent.

When a plurality of $R^{2a}$'s are present, they may be the same or different. When a plurality of $R^{2b}$'s are present, they may be the same or different.

When Y is 1 or more, $R^{2b}$ in $CR^{2b}_2$ that is bonded directly to $-C(R^1)(R^2)-$ in formula (AN1) differs from a fluorine atom.

Q represents $*^A-O-CO-O-*^B$, $*^A-CO-*^B$, $*^A-CO-O-*^B$, $*^A-O-CO-*^B$, $*^A-O-*^B$, $*^A-S-*^B$, or $*^A-SO_2-*^B$.

When X + Y in formula (AN1-1) is 1 or more and $R^{2a}$'s and $R^{2b}$'s in formula (AN1-1) are each a hydrogen atom, and Q represents $*^{A}$-O-CO-O-$*^{B}$, $*^{A}$-CO-$*^{B}$, $*^{A}$-O-CO-$*^{B}$, $*^{A}$-O-$*^{B}$, $*^{A}$-S-$*^{B}$, or $*^{A}$-$SO_2$-$*^{B}$.

$*^{A}$ represents a bonding position on the $R^3$ side in formula (AN1), and $*^{B}$ represents a bonding position on the -$SO_3^-$ side in formula (AN1).

In formula (AN1), $R^3$ represents an organic group.

No particular limitation is imposed on the organic group, so long as it has at least one carbon atom. The organic group may be a linear group (e.g., a linear alkyl group), a branched group (e.g., a branched alkyl group such as a t-butyl group), or a cyclic group. The organic group may or may not have a substituent. The organic group may or may not have a heteroatom (such as an oxygen atom, a sulfur atom, and/or a nitrogen atom).

In particular, $R^3$ is preferably an organic group having a ring structure. The ring structure may be a monocyclic structure or a polycyclic structure and may have a substituent. Preferably, the ring in the organic group including the ring structure is bonded directly to L in formula (AN1).

The organic group having the ring structure may or may not have, for example, a heteroatom (for example, an oxygen atom, a sulfur atom, and/or, a nitrogen atom). At least one carbon atom included in the ring structure may be replaced with a heteroatom.

The organic group having the ring structure is preferably a hydrocarbon group having a ring structure, a lactone ring group, or a sultone ring group. In particular, the organic group having the ring structure is preferably a hydrocarbon group having a ring structure.

The hydrocarbon group having a ring structure is preferably a monocyclic or polycyclic cycloalkyl group. These groups may have a substituent.

The cycloalkyl group may be a monocyclic group (such as a cyclohexyl group) or a polycyclic group (such as an adamantyl group), and the number of carbon atoms is preferably 5 to 12.

Preferably, the lactone group and the sultone group are each, for example, a group formed by removing one hydrogen atom from a ring member atom included in the lactone or sultone structure in any of the structures represented by formulas (LC1-1) to (LC1-21) and formulas (SL1-1) to (SL1-3) described above.

The non-nucleophilic anion may be a benzenesulfonate anion and is preferably a benzenesulfonate anion substituted with a branched alkyl group or a cycloalkyl group.

The non-nucleophilic anion is also preferably an anion represented by the following formula (AN2).

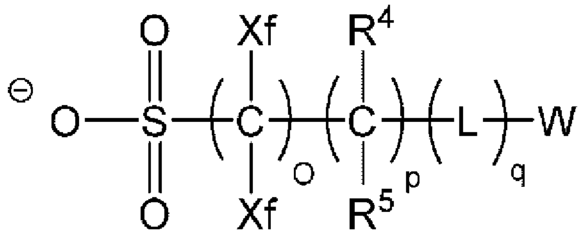

(AN2)

In formula (AN2), o represents an integer of from 1 to 3. p represents an integer of from 0 to 10. q represents an integer of from 0 to 10.

Xfs each represent a hydrogen atom, a fluorine atom, an alkyl group substituted with at least one fluorine atom, or an organic group having no fluorine atom. The number of carbon atoms in the alkyl group is preferably 1 to 10 and more preferably 1 to 4. The alkyl group substituted with at least one fluorine atom is preferably a perfluoroalkyl group.

Xf's are each preferably a fluorine atom or a perfluoroalkyl group having 1 to 4 carbon atoms and more preferably a fluorine atom or $CF_3$. It is still more preferable that each of Xfs is a fluorine atom.

$R^4$ and $R^5$ each independently represent a hydrogen atom, a fluorine atom, an alkyl group, or an alkyl group substituted with at least one fluorine atom. When a plurality of $R^4$'s are present, they may be the same or different. When a plurality of $R^5$'s are present, they may be the same or different.

The number of carbon atoms in each of the alkyl groups represented by $R^4$ and $R^5$ is preferably 1 to 4. Each alkyl group may have a substituent. $R_4$ and $R_5$ are each preferably a hydrogen atom.

L represents a divalent linking group. The definition of L is the same as the definition of L in formula (AN1).

W represents an organic group including a ring structure. In particular, W is preferably a cyclic organic group.

Examples of the cyclic organic group include alicyclic groups, aryl groups, and heterocyclic groups.

The alicyclic group may be a monocyclic alicyclic group or a polycyclic alicyclic group. Examples of the monocyclic alicyclic group include monocyclic cycloalkyl groups such as a cyclopentyl group, a cyclohexyl group, and a cyclooctyl group. Examples of the polycyclic alicyclic group include polycyclic cycloalkyl groups such as a norbornyl group, a tricyclodecanyl group, a tetracyclodecanyl group, a tetracyclododecanyl group, and an adamantyl group. Of these, alicyclic groups having 7 or more carbon atoms and a bulky structure such as a norbornyl group, a tricyclodecanyl

group, a tetracyclodecanyl group, a tetracyclododecanyl group, and an adamantyl group are preferred.

The aryl group may be a monocyclic or polycyclic aryl group. Examples of such an aryl group include a phenyl group, a naphthyl group, a phenanthryl group, and an anthryl group.

The heterocyclic group may be a monocyclic or polycyclic heterocyclic group. In particular, when the heterocyclic group is a polycyclic heterocyclic group, diffusion of acid can be further reduced. The heterocyclic group may or may not have aromaticity. Examples of the heterocycle having aromaticity include a furan ring, a thiophene ring, a benzofuran ring, a benzothiophene ring, a dibenzofuran ring, a dibenzothiophene ring, and a pyridine ring. Examples of the heterocycle having no aromaticity include a tetrahydropyran ring, lactone rings, sultone rings, and a decahydroisoquinoline ring. The heterocycle in the heterocyclic group is preferably a furan ring, a thiophene ring, a pyridine ring, or a decahydroisoquinoline ring.

The above cyclic organic group may have a substituent. Examples of the substituent include alkyl groups (which may be linear or branched and have preferably 1 to 12 carbon atoms), cycloalkyl groups (which may be monocyclic, polycyclic, or spirocyclic and have preferably 3 to 20 carbon atoms), aryl groups (having preferably 6 to 14 carbon atoms), a hydroxy group, alkoxy groups, ester groups, amido groups, urethane groups, ureide groups, thioether groups, sulfonamido groups, and sulfonate groups. Carbon included in the cyclic organic group (carbon contributing to the formation of the ring) may be carbonyl carbon.

The anion represented by formula (AN2) is preferably $SO_3^-$-$CF_2$-$CH_2$-OCO-$(L)_{q'}$-W, $SO_3^-$-$CF_2$-CHF-$CH_2$-OCO-$(L)_{q'}$-W, $SO_3^-$-$CF_2$-COO-$(L)_{q'}$-W, SO3-$CF_2$-$CF_2$-$CH_2$-$CH_2$-$(L)_q$-W, or $SO_3^-$-$CF_2$-CH($CF_3$)-OCO-$(L)_{q'}$-W. L, q, and W are the same as those in formula (AN2). q' represents an integer of from 0 to 10.

The non-nucleophilic anion is also preferably an aromatic sulfonate anion represented by the following formula (AN3).

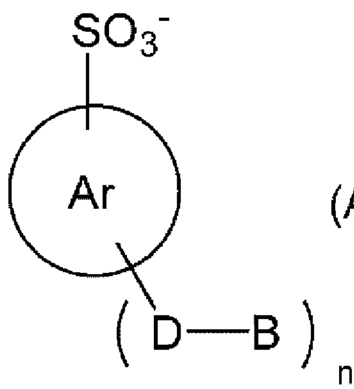

(AN3)

In formula (AN3), Ar represents an aryl group (such as a phenyl group) and may further have a substituent other than a sulfonate anion and the -(D-B) group. Examples of the substituent include a fluorine atom and a hydroxy group.

n represents an integer of 0 or more. n is preferably 1 to 4, more preferably 2 to 3, and still more preferably 3.

D represents a single bond or a divalent linking group. Examples of the divalent linking group include ether groups, thioether groups, a carbonyl group, sulfoxide groups, a sulfone group, sulfonate groups, ester groups, and groups formed by combining two or more of these groups.

B represents a hydrocarbon group.

B is preferably an aliphatic hydrocarbon group and more preferably an isopropyl group, a cyclohexyl group, or an aryl group optionally having a substituent (such as a tricyclohexylphenyl group).

The non-nucleophilic anion is also preferably a disulfonamide anion.

The disulfonamide anion is, for example, an anion represented by $N^-(SO_2-R^q)_2$.

Here, $R^q$ represents an alkyl group optionally having a substituent and is preferably a fluoroalkyl group and more preferably a perfluoroalkyl group. Two $R^q$'s may be bonded together to form a ring. The group formed by bonding two $R^q$'s is preferably an alkylene group optionally having a substituent, preferably a fluoroalkylene group, and still more preferably a perfluoroalkylene group. The number of carbon atoms in the alkylene group is preferably 2 to 4.

Other examples of the non-nucleophilic anion include anions represented by the following formulas (d1-1) to (d1-4).

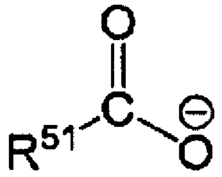

(d1-1)

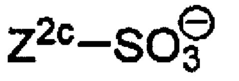

(d1-2)

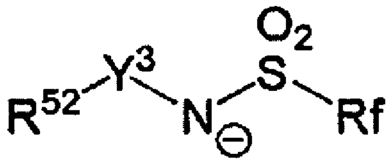

(d1-3)

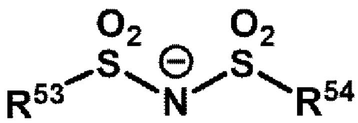

(d1-4)

In formula (d1-1), $R^{51}$ represents a hydrocarbon group (e.g., an aryl group such as a phenyl group) optionally having a substituent (e.g., a hydroxy group).

In formula (d1-2), $Z^{2c}$ represents a hydrocarbon group having 1 to 30 carbon atoms and optionally having a substituent (provided that the carbon atom adjacent to S is not substituted with a fluorine atom).

The hydrocarbon group represented by $Z^{2c}$ may be linear or branched and may have a ring structure. A carbon atom in the hydrocarbon group (preferably, a carbon atom serving as a ring member atom when the hydrocarbon group has a ring structure) may be carbonyl carbon (-CO-). Examples of the hydrocarbon group include a group having a norbornyl group optionally having a substituent. A carbon atom included in the norbornyl group may be carbonyl carbon.

It is preferable that "$Z^{2c}$-$SO_3^-$" in formula (d1-2) differs from the anions represented by formulas (AN1) to (AN3) above. For example, $Z^{2c}$ differs from an aryl group. For example, in $Z^{2c}$, the atoms at the $\alpha$- and $\beta$-positions with respect to -$SO_3^-$ are each preferably an atom different from a carbon atom having a fluorine atom as a substituent. For example, in $Z^{2c}$, the atom at the $\alpha$-position and/or the atom at the $\beta$-position with respect to -$SO_3^-$is preferably a ring member atom of the cyclic group.

In formula (d1-3), $R^{52}$ represents an organic group (preferably a hydrocarbon group having a fluorine atom), and $Y^3$ represents a linear, branched, or cyclic alkylene group, an arylene group, or a carbonyl group. Rf represents a hydrocarbon group.

In formula (d1-4), $R^{53}$ and $R^{54}$ each independently represent an organic group (preferably a hydrocarbon group having a fluorine atom). $R^{53}$ and $R^{54}$ may be bonded together to form a ring.

One type of organic anion may be used alone, or two or more types of organic anions may be used.

It is also preferable that the photoacid generator (B) is at least one selected from the group consisting of compounds (I) to (II).

(Compound (I))

The compound (I) is a compound having at least one structural moiety X described below and at least one structural moiety Y described below and is a compound that generates an acid including a first acidic moiety described below and derived from the structural moiety X and a second acidic moiety described below and derived from the structural moiety Y when irradiated with actinic rays or radiation.

Structural moiety X: A structural moiety including an anionic moiety $A_1^-$ and a cationic moiety $M_1^+$ and forms the first acidic moiety represented by $HA_1$ when irradiated with actinic rays or radiation.

Structural moiety Y: A structural moiety including an anionic moiety $A_2^-$ and a cationic moiety $M_2^+$ and forms the second acidic moiety represented by $HA_2$ when irradiated with actinic rays or radiation.

The compound (I) satisfies the following condition I.

Condition I: A compound PI formed by replacing each of the cationic moiety $M_1^+$ in the structural moiety X in the compound (I) and the cationic moiety $M_2^+$ in the structural moiety Y with $H^+$ has an acid dissociation constant a1 derived from the acidic moiety represented by $HA_1$ formed by replacing the cationic moiety $M_1^+$ in the structural moiety X with $H^+$ and an acid dissociation constant a2 derived from the acidic moiety represented by $HA_2$ formed by replacing the cationic moiety $M_2^+$ in the structural moiety Y with $H^+$, and the acid dissociation constant a2 is larger than the acid dissociation constant a1.

The condition I will be specifically described.

When the compound (I) is a compound that generates an acid having one first acidic moiety derived from the structural moiety X and one second acidic moiety derived from the structural moiety Y, the compound PI corresponds

to a "compound having $HA_1$ and $HA_2$."

The acid dissociation constant a1 and the acid dissociation constant a2 of this compound PI will be specifically described. When the acid dissociation constants of the compound PI are determined, the pKa when the compound PI becomes a "compound having $A_1^-$and $HA_2$" is the acid dissociation constant a1, and the pKa when the "compound having $A_1^-$and $HA_2$" becomes a "compound having $A_1^-$ and $A_2^-$" is the acid dissociation constant a2.

When the compound (I) is, for example, a compound that generates an acid having two first acidic moieties derived from the structural moieties X and one second acidic moiety derived from the structural moiety Y, the compound PI corresponds to a "compound having two $HA_1$'s and one $HA_2$."

When the acid dissociation constants of the compound PI are determined, the acid dissociation constant when the compound PI becomes a "compound having one $A_1^-$, one $HA_1$, and one $HA_2$," and the acid dissociation constant when the "compound having one $A_1^-$, one $HA_1$, and one $HA_2$" becomes a "compound having two $A_1^-$'s and one $HA_2$" each correspond to the acid dissociation constant a1. Moreover, the acid dissociation constant when the "compound having two $A_1^-$'s and one $HA_2$" becomes a "compound having two $A_1^-$'s and $A_2^-$" corresponds to the acid dissociation constant a2. Specifically, such a compound PI has a plurality of acid dissociation constants derived from acidic moieties represented by $HA_1$ formed by replacing cationic moieties $M_1^+$ in the structural moieties X with $H^+$. In this case, the acid dissociation constant a2 is larger than the largest one of the plurality of acid dissociation constants a1. Let aa be the acid dissociation constant when the compound PI becomes the "compound having one $A_1^-$, one $HA_1$, and one $HA_2$," and ab be the acid dissociation constant when the "compound having one $A_1^-$, one $HA_1$, and one $HA_2$" becomes the "compound having two $A_1^-$'s and one $HA_2$." Then aa and ab satisfy the relation aa < ab.

The acid dissociation constants a1 and a2 are determined by the acid dissociation constant measurement method described above.

The compound PI corresponds to the acid generated when the compound (I) is irradiated with actinic rays or radiation.

When the compound (I) has two or more structural moieties X, these structural moieties X may be the same or different. Moreover, two or more $A_1^-$'s may be the same or different, and two or more $M_1^+$'s may be the same or different.

In compound (I), $A_1^-$ and $A_2^-$ may be the same or different, and $M_1^+$ and $M_2^+$ may be the same or different. However, it is preferable that $A_1^-$ and $A_2^-$ are different from each other.

In the compound PI, the difference (absolute difference) between the acid dissociation constant a1 (when a plurality of acid dissociation constants a1 are present, the maximum value of the acid dissociation constants) and the acid dissociation constant a2 is preferably 0.1 or more, more preferably 0.5 or more, and still more preferably 1.0 or more. No particular limitation is imposed on the upper limit of the difference (absolute difference) between the acid dissociation constant a1 (when a plurality of acid dissociation constants a1 are present, the maximum value of the acid dissociation constants) and the acid dissociation constant a2, but the upper limit is, for example, 16 or less.

In the compound PI, the acid dissociation constant a2 is preferably 20 or less and more preferably 15 or less. The lower limit of the acid dissociation constant a2 is preferably - 4.0 or more.

In the compound PI, the acid dissociation constant a1 is preferably 2.0 or less and more preferably 0 or less. The lower limit of the acid dissociation constant a1 is preferably - 20.0 or more.

The anionic moiety $A_1^-$ and the anionic moiety $A_2^-$ are each a structural moiety including a negatively charged atom or atomic group and are each, for example, a structural moiety selected from the group consisting of formulas (AA-1) to (AA-3) and formulas (BB-1) to (BB-6) shown below.

The anionic moiety $A_1^-$ is preferably a moiety capable of forming an acidic moiety having a small acid dissociation constant, more preferably a moiety represented by any one of (AA-1) to (AA-3), and still more preferably a moiety represented by any one of formulas (AA-1) and (AA-3).

The anionic moiety $A_2^-$ is preferably a moiety capable of forming an acidic moiety having a larger acid dissociation constant than the anionic moiety $A_1^-$, more preferably a moiety represented by any one of formulas (BB-1) to (BB-6), and still more preferably a moiety represented by any one of formulas (BB-1) and (BB-4).

In formulas (AA-1) to (AA-3) and formulas (BB-1) to (BB-6) below, * represents a bonding position.

In formula (AA-2), $R^A$ represents a monovalent organic group. No particular limitation is imposed on the monovalent organic group represented by $R^A$, and examples thereof include a cyano group, a trifluoromethyl group, and a methanesulfonyl group.

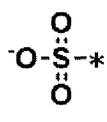
AA-1

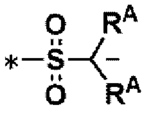
AA-2

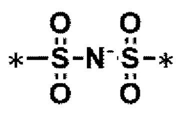
AA-3

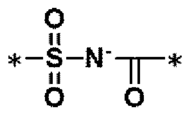 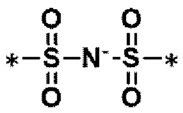 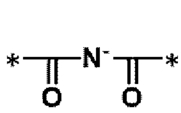 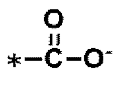 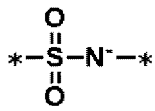 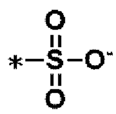

BB-1 BB-2 BB-3 BB-4 BB-5 BB-6

The cationic moiety $M_1^+$ and the cationic moiety $M_2^+$ are each a structural moiety including a positively charged atom or atomic group and are each, for example, a singly charged organic cation. Examples of the organic cation include the above-described organic cation represented by $M^+$.

No particular limitation is imposed on the specific structure of the compound (I), and examples thereof include compounds represented by formulas (Ia-1) to (Ia-5) described below.

- Compound represented by formula (Ia-1) -

First, the compound represented by formula (Ia-1) will be described.

$$M_{11}^{+}A_{11}^{-}\text{-}L_1\text{-}A_{12}^{-}M_{12}^{+} \qquad \text{(Ia-1)}$$

The compound represented by formula (Ia-1) generates an acid represented by $HA_{11}$-$L_1$-$A_{12}H$ when irradiated with actinic rays or radiation.

In formula (Ia-1), $M_{11}^+$ and $M_{12}^+$ each independently represent an organic cation. $A_{11}^-$ and $A_{12}^-$ each independently represent a monovalent anionic functional group.

$L_1$ represents a divalent linking group.

$M_{11}^+$ and $M_{12}^+$ may be the same or different.

$A_{11}^-$ and $A_{12}^-$ may be the same or different, but it is preferable that they differ from each other.

In a compound PIa ($HA_{11}$-$L_1$-$A_{12}H$) formed by replacing each of the cations represented by $M_{11}^+$ and $M_{12}^+$ in formula (Ia-1) above with $H^+$, the acid dissociation constant a2 derived from the acidic moiety represented by $A_{12}H$ is larger than the acid dissociation constant a1 derived from the acidic moiety represented by $HA_{11}$. Preferred values of the acid dissociation constants a1 and a2 are as described above. The compound PIa is the same as the acid generated from the compound represented by formula (Ia-1) upon irradiation with actinic rays or radiation.

At least one of $M_{11}^+$, $M_{12}^+$, $A_{11}^-$, $A_{12}^-$, or $L_1$ may have an acid-decomposable group as a substituent.

In formula (Ia-1), the organic cations represented by $M_1^+$ and $M_2^+$ are as described above.

The monovalent anionic functional group represented by $A_{11}^-$ means a monovalent group including the anionic moiety $A_1^-$ described above. The monovalent anionic functional group represented by $A_{12}^-$ means a monovalent group including the anionic moiety $A_2^-$described above.

The monovalent anionic functional groups represented by $A_{11}^-$ and $A_{12}^-$ are each preferably a monovalent anionic functional group including the anionic moiety represented by any of formulas (AA-1) to (AA-3) and formulas (BB-1) to (BB-6) described above and more preferably a monovalent anionic functional group selected from the group consisting of formulas (AX-1) to (AX-3) and formulas (BX-1) to (BX-7). In particular, the monovalent anionic functional group represented by $A_{11}^-$ is preferably a monovalent anionic functional group represented by any of formulas (AX-1) to (AX-3). In particular, the monovalent anionic functional group represented by $A_{12}^-$ is preferably a monovalent anionic functional group represented by any of formulas (BX-1) to (BX-7) and more preferably a monovalent anionic functional group represented by any of formulas (BX-1) to (BX-6).

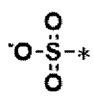 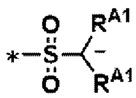 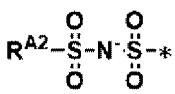

AX-1 AX-2 AX-3

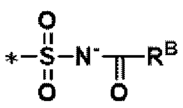 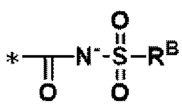 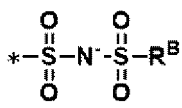 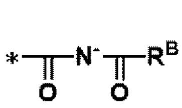 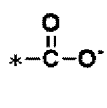 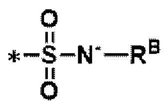 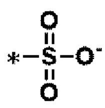

BX-1 BX-2 BX-3 BX-4 BX-5 BX-6 BX-7

In formulas (AX-1) to (AX-3), $R^{A1}$ and $R^{A2}$ each independently represent a monovalent organic group. * represents a bonding position.

No particular limitation is imposed on the monovalent organic group represented by $R^{A1}$, and examples thereof include a cyano group, a trifluoromethyl group, and a methanesulfonyl group.

The monovalent organic group represented by $R^{A2}$ is preferably a linear, branched, or cyclic alkyl group or an aryl group.

The number of carbon atoms in the alkyl group is preferably 1 to 15, more preferably 1 to 10, and still more preferably 1 to 6.

The alkyl group may have a substituent. The substituent is preferably a fluorine atom or a cyano group and more preferably a fluorine atom. When the alkyl group has a fluorine atom as a substituent, the alkyl group may be a perfluoroalkyl group.

The aryl group is preferably a phenyl group or a naphthyl group and more preferably a phenyl group.

The aryl group may have a substituent. The substituent is preferably a fluorine atom, an iodine atom, a perfluoroalkyl group (for example, having preferably 1 to 10 carbon atoms and more preferably 1 to 6 carbon atoms), or a cyano group and is more preferably a fluorine atom, an iodine atom, or a perfluoroalkyl group.

In formulas (BX-1) to (BX-4) and (BX-6), $R^B$ represents a monovalent organic group. * represents a bonding position.

The monovalent organic group represented by $R^B$ is preferably a linear, branched, or cyclic alkyl group or an aryl group.

The number of carbon atoms in the alkyl group is preferably 1 to 15, more preferably 1 to 10, and still more preferably 1 to 6.

The alkyl group may have a substituent. No particular limitation is imposed on the substituent, but the substituent is preferably a fluorine atom or a cyano group and more preferably a fluorine atom. When the alkyl group has a fluorine atom as a substituent, the alkyl group may be a perfluoroalkyl group.

When a carbon atom in the alkyl group at a bonding position (for example, the carbon atom in the alkyl group that is bonded directly to -CO- in any of formulas (BX-1) and (BX-4), the carbon atom shown in the alkyl group that is bonded directly to $-SO_2-$ in any of formulas (BX-2) and (BX-3), or the carbon atom shown in the alkyl group that is bonded directly to $N^-$in formula (BX-6)) has a substituent, it is also preferable that the substituent differs from a fluorine atom and a cyano group.

In the alkyl group, a carbon atom may be replaced with carbonyl carbon.

The aryl group is preferably a phenyl group or a naphthyl group and more preferably a phenyl group.

The aryl group may have a substituent. The substituent is preferably a fluorine atom, an iodine atom, a perfluoroalkyl group (for example, having preferably 1 to 10 carbon atoms and more preferably 1 to 6 carbon atoms), a cyano group, an alkyl group (for example, having preferably 1 to 10 carbon atoms and more preferably 1 to 6 carbon atoms), an alkoxy group (for example, having preferably 1 to 10 carbon atoms and more preferably 1 to 6 carbon atoms), or an alkoxycarbonyl group (for example, having preferably 2 to 10 carbon atoms and more preferably 2 to 6 carbon atoms) and more preferably a fluorine atom, an iodine atom, a perfluoroalkyl group, an alkyl group, an alkoxy group, or an alkoxycarbonyl group.

In formula (Ia-1), no particular limitation is imposed on the divalent linking group represented by $L_1$, and examples of $L_1$ include -CO-, -NR-, -CO-, -O-, -S-, -SO-, $-SO_2-$, alkylene groups (which may be linear or branched and have preferably 1 to 6 carbon atoms), cycloalkylene groups (having preferably 3 to 15 carbon atoms), alkenylene groups (having preferably 2 to 6 carbon atoms), divalent aliphatic heterocyclic groups (preferably 5- to 10-membered rings, more preferably 5- to 7-membered rings, and still more preferably 5- to 6-membered rings, each of which has at least one N atom, O atom, S atom, or Se atom in the ring structure), divalent aromatic heterocyclic groups (preferably 5- to 10-membered rings, more preferably 5- to 7-membered rings, and still more preferably 5- to 6-membered rings, each of which has at least one N atom, O atom, S atom, or Se atom in the ring structure), divalent aromatic hydrocarbon ring groups (preferably 6- to 10-membered rings and more preferably 6-membered rings), and divalent linking groups formed by combining a plurality of groups selected from the above groups. R is a hydrogen atom or a monovalent organic group. No particular limitation is imposed on the monovalent organic group, but the monovalent organic group is preferably, for example, an alkyl group (having preferably 1 to 6 carbon atoms).

The alkylene, cycloalkylene, alkenylene, divalent aliphatic heterocyclic, divalent aromatic heterocyclic, and divalent aromatic hydrocarbon ring groups described above may each have a substituent. Examples of the substituent include halogen atoms (preferably a fluorine atom).

In particular, the divalent linking group represented by $L_1$ is preferably a divalent linking group represented by formula (L1).

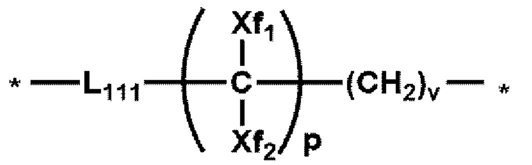
(L1)

In formula (L1), $L_{111}$ represents a single bond or a divalent linking group.

No particular limitation is imposed on the divalent linking group represented by $L_{111}$, and examples of the divalent linking group include -CO-, -NH-, -O-, -SO-, $-SO_2-$, alkylene groups (which may be linear or branched and have preferably 1 to 6 carbon atoms) optionally having a substituent, cycloalkylene groups (having preferably 3 to 15 carbon atoms) optionally having a substituent, aryl groups (having preferably 6 to 10 carbon atoms) optionally having a substituent, and divalent linking groups formed by combining a plurality of groups selected from the above groups. No particular limitation is imposed on the substituent, and examples thereof include halogen atoms.

p represents an integer of from 0 to 3 and preferably represents an integer of from 1 to 3. v represents an integer of 0 or 1.

$Xf_1$'s each independently represent a fluorine atom or an alkyl group substituted with at least one fluorine atom. The number of carbon atoms in the alkyl group is preferably 1 to 10 and more preferably 1 to 4. The alkyl group substituted with at least one fluorine atom is preferably a perfluoroalkyl group.

$Xf_2$'s each independently represent a hydrogen atom, an alkyl group optionally having a fluorine atom as a substituent, or a fluorine atom. The number of carbon atoms in the alkyl group is preferably 1 to 10 and more preferably 1 to 4. In particular, each $Xf_2$ represents preferably a fluorine atom or an alkyl group substituted with at least one fluorine atom and more preferably a fluorine atom or a perfluoroalkyl group.

In particular, $Xf_1$'s and $Xf_2$'s each independently represent preferably a fluorine atom or a perfluoroalkyl group having 1 to 4 carbon atoms and more preferably a fluorine atom or $CF_3$. In particular, it is still more preferable that $Xf_1$'s and $Xf_2$'s are each a fluorine atom.

[0461] * represents a bonding position.

When $L_{11}$ in formula (Ia-1) represents a divalent linking group represented by formula (L1), it is preferable that the direct bond (*) on the $L_{111}$ side in formula (L1) is bonded to $A_{12}^-$in formula (Ia-1).

- Compounds represented be formulas (Ia-2) to (Ia-4) -

Next, compounds represented by formulas (Ia-2) to (Ia-4) will be described.

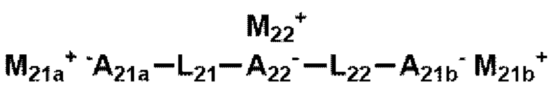
(Ia-2)

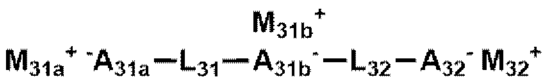
(Ia-3)

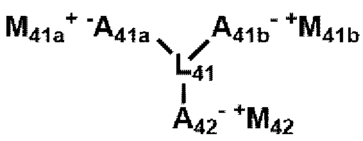
(Ia-4)

In formula (Ia-2), $A_{21a}^-$ and $A_{21b}^-$ each independently represent a monovalent anionic functional group. Each of the monovalent anionic functional groups represented by $A_{21a}^-$ and $A_{21b}^-$ means a monovalent group including the above-described anionic moiety $A_1^-$. No particular limitation is imposed on the monovalent anionic functional groups represented by $A_{21a}^-$ and $A_{21b}^-$, but $A_{21a}^-$ and $A_{21b}^-$ are each, for example, a monovalent anionic functional group selected from the group consisting of formulas (AX-1) to (AX-3) described above.

$A_{22}^-$ represents a divalent anionic functional group. The divalent anionic functional group represented by $A_{22}^-$ means a divalent groups including the anionic moiety $A_2^-$ described above. Examples of the divalent anionic functional group represented by $A_{22}^-$ include divalent anionic functional groups represented by the following formulas (BX-8) to (BX-11).

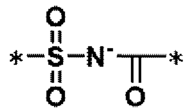 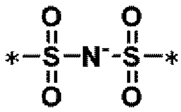 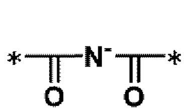 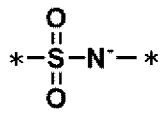

BX-8 BX-9 BX-10 BX-11

$M_{21a}^+$, $M_{21b}^+$, and $M_{22}^+$ each independently represent an organic cation. The definitions of the organic cations represented by $M_{21a}^+$, $M_{21b}^+$, and $M_{22}^+$ are the same as that of $M_1^+$ described above, and their preferred modes are also the same as those of $M_1^+$.

$L_{21}$ and $L_{22}$ each independently represent a divalent organic group.

In a compound PIa-2 formed by replacing each of the organic cations represented by $M_{21a}^+$, $M_{21b}^+$, and $M_{22}^+$ in formula (Ia-2) with $H^+$, the acid dissociation constant a2 derived from an acidic moiety represented by $A_{22}H$ is larger than the acid dissociation constant a1-1 derived from $A_{21a}H$ and the acid dissociation constant a1-2 derived from an acidic moiety represented by $A_{21b}H$. The acid dissociation constant a1-1 and the acid dissociation constant a1-2 each correspond to the acid dissociation constant a1 described above.

$A_{21a}^-$ and $A_{21b}^-$ may be the same or different. $M_{21a}^+$, $M_{21b}^+$, and $M_{22}^+$ may be the same or different.

At least one of $M_{21a}^+$, $M_{21b}^+$, $M_{22}^+$, $A_{21a}^-$, $A_{21b}^-$, $L_{21}$, or $L_{22}$ may have an acid-decomposable group as a substituent.

In formula (Ia-3), $A_{31a}^-$ and $A_{32}^-$ each independently represent a monovalent anionic functional group. The definition of the monovalent anionic functional group represented by $A_{31a}^-$ is the same as those of $A_{21a}^-$ and $A_{21b}^-$ in formula (Ia-2), and its preferred mode is also the same as those of $A_{21a}^-$ and $A_{21b}^-$.

The monovalent anionic functional group represented by $A_{32}^-$ means a monovalent group including the anionic moiety $A_2^-$ described above. No particular limitation is imposed on the monovalent anionic functional group represented by $A_{32}^-$, and $A_{32}^-$ is, for example, a monovalent anionic functional group selected from the group consisting of formulas (BX-1) to (BX-7) described above.

$A_{31b}^-$ represents a divalent anionic functional group. The divalent anionic functional group represented by $A_{31b}^-$ means a divalent group including the anionic moiety $A_1^-$ described above. Examples of the divalent anionic functional group represented by $A_{31b}^-$ include a divalent anionic functional group represented by the following formula (AX-4).

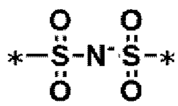

AX-4

$M_{31a}^+$, $M_{31b}^+$, and $M_{32}^+$ each independently represent a monovalent organic cation. The definitions of the organic cations represented by $M_{31a}^+$, $M_{31b}^+$, and $M_{32}^+$ are the same are that of $M_1^+$ described above, and their preferred modes are also the same as that of $M_1^+$.

$L_{31}$ and $L_{32}$ each independently represent a divalent organic group.

In a compound PIa-3 formed by replacing each of the organic cations represented by $M_{31a}^+$, $M_{31b}^+$, and $M_{32}^+$ in formula (Ia-3) with $H^+$, the acid dissociation constant a2 derived from an acidic moiety represented by $A_{32}H$ is larger than the acid dissociation constant a1-3 derived from an acidic moiety represented by $A_{31a}H$ and the acid dissociation constant a1-4 derived from an acidic moiety represented by $A_{31b}H$. The acid dissociation constant a1-3 and the acid dissociation constant a1-4 each correspond to the acid dissociation constant a1 described above.

$A_{31a}^-$ and $A_{32}^-$ may be the same or different. $M_{31a}^+$, $M_{31b}^+$, and $M_{32}^+$ may be the same or different.

At least one of $M_{31a}^+$, $M_{31b}^+$, $M_{32}^+$, $A_{31a}^-$, $A_{32}^-$, $L_{31}$, or $L_{32}$ may have an acid-decomposable group as a substituent.

In formula (Ia-4), $A_{41a}^-$, $A_{41b}^-$, and $A_{42}^-$ each independently represent a monovalent anionic functional group. The definitions of the monovalent anionic functional groups represented by $A_{41a}^-$ and $A_{41b}^-$ are the same as the definitions of $A_{21a}^-$ and $A_{21b}^-$ in formula (Ia-2) described above. The definition of the monovalent anionic functional group represented by $A_{42}^-$ is the same as that of $A_{32}^-$ in formula (Ia-3) described above, and its preferred mode is also the same as that of $A_{32}^-$.

$M_{41a}^+$, $M_{41b}^+$, and $M_{42}^+$ each independently represent an organic cation.

$L_{41}$ represents a trivalent organic group.

In a compound PIa-4 formed by replacing each of the organic cations represented by $M_{41a}^+$, $M_{41b}^+$, and $M_{42}^+$ in formula (Ia-4) with $H^+$, the acid dissociation constant a2 derived from an acidic moiety represented by $A_{42}H$ is larger than the acid dissociation constant a1-5 derived from an acidic moiety represented by $A_{41a}H$ and the acid dissociation constant a1-6 derived from an acidic moiety represented by $A_{41b}H$. The acid dissociation constant a1-5 and the acid

dissociation constant a1-6 each correspond to the acid dissociation constant a1 described above.

$A_{41a}^-$, $A_{41b}^-$, and $A_{42}^-$ may be the same or different. $M_{41a}^+$, $M_{41b}^+$, and $M_{42}^+$ may be the same or different.

At least one of $M_{41a}^+$, $M_{41b}^+$, $M_{42}^+$, $A_{41a}^-$, $A_{41b}^-$, $A_{42}^-$, or $L_{41}$ may have an acid-decomposable group as a substituent.

No particular limitation is imposed on the divalent organic groups represented by $L_{21}$ and $L_{22}$ in In formula (Ia-2) and $L_{31}$ and $L_{32}$ in formula (Ia-3), and examples thereof include - CO-, -NR-, -O-, -S-, -SO-, $-SO_2-$, alkylene groups (which may be linear or branched and have preferably 1 to 6 carbon atoms), cycloalkylene groups (having preferably 3 to 15 carbon atoms), alkenylene groups (having preferably 2 to 6 carbon atoms), divalent aliphatic heterocyclic groups (preferably 5- to 10-membered rings, more preferably 5- to 7-membered rings, and still more preferably 5- to 6-membered rings, each of which has at least one N atom, O atom, S atom, or Se atom in the ring structure), divalent aromatic heterocyclic groups (preferably 5- to 10-membered rings, more preferably 5- to 7-membered rings, and still more preferably 5- to 6-membered rings, each of which has at least one N atom, O atom, S atom, or Se atom in the ring structure), divalent aromatic hydrocarbon ring groups (preferably 6- to 10-membered rings and more preferably 6-membered rings), and divalent organic groups formed by combining a plurality of groups selected from the above groups. Examples of R include a hydrogen atom and monovalent organic groups. No particular limitation is imposed on the monovalent organic group, but the monovalent organic group is, for example, preferably an alkyl group (having preferably 1 to 6 carbon atoms).

The alkylene, cycloalkylene, alkenylene, divalent aliphatic heterocyclic, divalent aromatic heterocyclic, and divalent aromatic hydrocarbon ring groups described above may each have a substituent. Examples of the substituent include halogen atoms (preferably a fluorine atom).

Preferably, the divalent organic groups represented by $L_{21}$ and $L_{22}$ in formula (Ia-2) and $L_{31}$ and $L_{32}$ in formula (Ia-3) are each, for example, a divalent organic group represented by formula (L2) below.

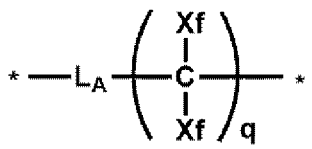

(L2)

In formula (L2), q represents an integer of from 1 to 3. * represents a bonding position.

Xfs each independently represent a fluorine atom or an alkyl group substituted with at least one fluorine atom. The number of carbon atoms in the alkyl group is preferably 1 to 10 and more preferably 1 to 4. The alkyl group substituted with at least one fluorine atom is preferably a perfluoroalkyl group.

Xf's are each preferably a fluorine atom or a perfluoroalkyl group having 1 to 4 carbon atoms and more preferably a fluorine atom or $CF_3$. In particular, it is more preferable that both Xf's are fluorine atoms.

$L_A$ represents a single bond or a divalent linking group.

No particular limitation is imposed on the divalent linking group represented by $L_A$, and examples thereof include -CO-, -O-, -SO-, $-SO_2-$, alkylene groups (which may be linear or branched and have preferably 1 to 6 carbon atoms), cycloalkylene groups (having preferably 3 to 15 carbon atoms), divalent aromatic hydrocarbon ring groups (preferably 6- to 10-membered rings and more preferably 6-membered rings), and divalent linking groups formed by combining a plurality of groups selected from the above groups.

The alkylene, cycloalkylene, and divalent aromatic hydrocarbon ring groups described above may each have a substituent. Examples of the substituent include halogen atoms (preferably a fluorine atom).

Examples of the divalent organic group represented by formula (L2) include $*-CF_2-*$, $*-CF_2-CF_2-*$, $*-CF_2-CF_2-CF_2-*$, $*-Ph-O-SO_2-CF_2-*$, $*-Ph-O-SO_2-CF_2-CF_2-*$, $*-Ph-O-SO_2-CF_2-CF_2-CF_2-*$, and $*-Ph-OCO-CF_2-*$. Ph is a phenylene group optionally having a substituent and is preferably a 1,4-phenylene group. No particular limitation is imposed on the substituent, but the substituent is preferably an alkyl group (for example, having preferably 1 to 10 carbon atoms and more preferably 1 to 6 carbon atoms), an alkoxy group (for example, having preferably 1 to 10 carbon atoms and more preferably 1 to 6 carbon atoms), or an alkoxycarbonyl group (for example, having preferably 2 to 10 carbon atoms and more preferably 2 to 6 carbon atoms).

When $L_{21}$ and $L_{22}$ in formula (Ia-2) each represent the divalent organic group represented by formula (L2), it is preferable that the direct bond (*) on the $L_A$ side in formula (L2) is bonded to $A_{21a}^-$ or $A_{21b}^-$ in formula (Ia-2).

When $L_{31}$ and $L_{32}$ in formula (Ia-3) each represent the divalent organic group represented by formula (L2), it is preferable that the direct bond (*) on the $L_A$ side in formula (L2) is bonded to $A_{31a}^-$ or $A_{32}^-$ in formula (Ia-3).

- Compound represented by formula (Ia-5) -

Next, formula (Ia-5) will be described.

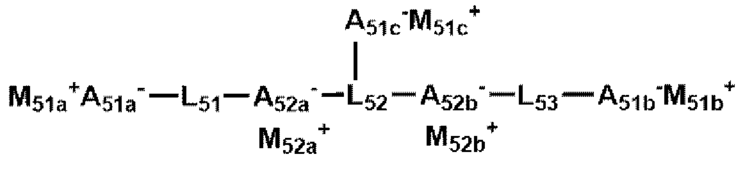

(Ia-5)

In formula (Ia-5), $A_{51a}^-$, $A_{51b}^-$, and $A_{51c}^-$ each independently represent a monovalent anionic functional group. Each of the monovalent anionic functional groups represented by $A_{51a}^-$, $A_{51b}^-$, and $A_{51c}^-$ means a monovalent group including the anionic moiety $A_1^-$ described above. No particular limitation is imposed on the monovalent anionic functional groups represented by $A_{51a}^-$, $A_{51b}^-$, and $A_{51c}^-$, but each of these monovalent anionic functional groups is, for example, a monovalent anionic functional group selected from the group consisting of formulas (AX-1) to (AX-3) described above.

$A_{52a}^-$ and $A_{52b}^-$ each represent a divalent anionic functional group. Each of the divalent anionic functional groups represented by $A_{52a}^-$ and $A_{52b}^-$ means a divalent group including the anionic moiety $A_2^-$ described above. The divalent anionic functional group represented by $A_{22}^-$ is, for example, a divalent anionic functional groups selected from the group consisting of formulas (BX-8) to (BX-11) described above.

$M_{51a}^+$, $M_{51b}^+$, $M_{51c}^+$, $M_{52a}^+$, and $M_{52b}^+$ each independently represent an organic cation. The definitions of the organic cations represented by $M_{51a}^+$, $M_{51b}^+$, $M_{51c}^+$, $M_{52a}^+$, and $M_{52b}^+$ are the same as the definition of $M_1^+$ described above, and their preferred modes are also the same as that of $M_1^+$.

$L_{51}$ and $L_{53}$ each independently represent a divalent organic group. The definitions of the divalent organic groups represented by $L_{51}$ and $L_{53}$ are the same as those of $L_{21}$ and $L_{22}$ in formula (Ia-2) described above, and their preferred modes are also the same as those of $L_{21}$ and $L_{22}$.

$L_{52}$ represents a trivalent organic group. The definition of the trivalent organic group represented by $L_{52}$ is the same as that of $L_{41}$ in formula (Ia-4) described above, and its preferred mode is also the same as that of $L_{41}$.

In a compound PIa-5 formed by replacing each of the organic cations represented by $M_{51a}^+$, $M_{51b}^+$, $M_{51c}^+$, $M_{52a}^+$, and $M_{52b}^+$ in formula (Ia-5) with $H^+$, the acid dissociation constant a2-1 derived from an acidic moiety represented by $A_{52a}H$ and the acid dissociation constant a2-2 derived from an acidic moiety represented by $A_{52b}H$ are larger than the acid dissociation constant a1-1 derived from $A_{51a}H$, the acid dissociation constant a1-2 derived from an acidic moiety represented by $A_{51b}H$, and the acid dissociation constant a1-3 derived from an acidic moiety represented by $A_{51c}H$. The acid dissociation constants a1-1 to a1-3 each correspond to the acid dissociation constant a1 described above, and the acid dissociation constants a2-1 and a2-2 each correspond to the acid dissociation constant a2 described above.

$A_{51a}^-$, $A_{51b}^-$, and $A_{51c}^-$ may be the same or different. $A_{52a}^-$ and $A_{52b}^-$ may be the same or different. $M_{51a}^+$, $M_{51b}^+$, $M_{51c}^+$, $M_{52a}^+$, and $M_{52b}^+$ may be the same or different.

At least one of $M_{51b}^+$, $M_{51c}^+$, $M_{52a}^+$, $M_{52b}^+$, $A_{51a}^-$, $A_{51b}^-$, $A_{51c}^-$, $L_{51}$, $L_{52}$, or $L_{53}$ may have an acid-decomposable group as a substituent.

(Compound (II))

The compound (II) is a compound that has two or more structural moieties X described above and at least one structural moiety Z described below and is a compound that generates an acid including two or more first acidic moieties derived from the structural moieties X and the structural moiety Z when irradiated with actinic rays or radiation.

Structural moiety Z: Non-ionic moiety capable of neutralizing acid

The definition of the structural moiety X in the compound (II) and the definitions of $A_1^-$ and $M_1^+$ are the same as that of the structural moiety X in the compound (I) described above and those of $A_1^-$ and $M_1^+$ in the structural moiety X in the compound (I) described above, and their preferred modes are also the same as those of the compound (I).

In a compound PII formed by replacing each of the cationic moieties $M_1^+$ in the structural moieties X in the compound (II) with $H^+$, a preferred range of the acid dissociation constant a1 derived from an acidic moiety represented by $HA_1$ formed by replacing the cationic moiety $M_1^+$ in one of the structural moieties X with $H^+$ is the same as that of the acid dissociation constant a1 in the compound PI.

When the compound (II) is, for example, a compound that generates an acid having two first acidic moieties derived from the structural moieties X and the structural moiety Z, the compound PII corresponds to a "compound having two $HA_1$s." When the acid dissociation constants of the compound PII are determined, the acid dissociation constant when the compound PII becomes a "compound having one $A_1^-$ and one $HA_1$" and the dissociation constant when the "compound having one $A_1^-$ and one $HA_1$" becomes a "compound having two $A_1^-$'s" each correspond to the acid dissociation constant a1.

The acid dissociation constant a1 is determined by the acid dissociation constant measurement method described above.

The compound PII corresponds to an acid generated when the compound (II) is irradiated with actinic rays or radiation.

The two or more structural moieties X may be the same or different. The two or more $A_1^-$'s may be the same or different, and the two or more $M_1^+$'s may be the same or different.

No particular limitation is imposed on the non-ionic moiety that is in the structural moiety Z and capable of neutralizing an acid, and the non-ionic moiety is, for example, preferably a moiety including a functional group having an electron or a group capable of electrostatically interacting with a proton.

Examples of the functional group having an electron or a group capable of electrostatically interacting with a proton include a functional group having a macrocyclic structure such as a cyclic polyether and a functional group having a nitrogen atom having an unshared electron pair not contributing to π-conjugation. The nitrogen atom having an unshared electron pair not contributing to π-conjugation is, for example, a nitrogen atom having a partial structure represented by any of the following formulas.

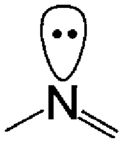 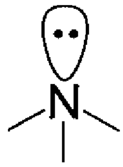 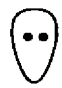

Unshared electron pair

Examples of the partial structure of the functional group having an electron or a group capable of electrostatically interacting with a proton include crown ether structures, azacrown ether structures, primary to tertiary amine structures, a pyridine structure, an imidazole structure, and a pyrazine structure. Of these, primary to tertiary amine structures are preferred.

No particular limitation is imposed on the compound (II), and examples thereof include compounds represented by the following formulas (IIa-1) and (IIa-2).

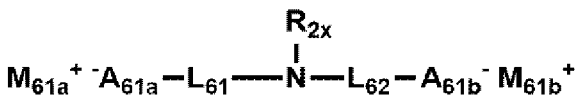

(IIa-1)

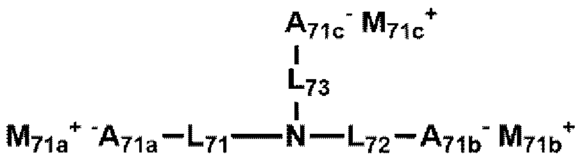

(IIa-2)

In formula (IIa-1), the definitions of $A_{61a}^-$ and $A_{61b}^-$ are each the same as that of $A_{11}^-$in formula (Ia-1) above, and their preferred modes are also the same as that of $A_{11}^-$. The definitions of $M_{61a}^+$ and $M_{61b}^+$ are each the same as that of $M_{11}^+$ in formula (Ia-1), and their preferred modes are also the same as that of $M_{11}^+$.

In formula (IIa-1), the definitions of $L_{61}$ and $L_{62}$ are each the same as that of $L_1$ in formula (Ia-1) above, and their preferred modes are also the same as that of $L_1$.

In formula (IIa-1), $R_{2X}$ represents a monovalent organic group. No particular limitation is imposed on the monovalent organic group represented by $R_{2X}$, and examples thereof include alkyl groups (which have preferably 1 to 10 carbon atoms and may be linear or branched), cycloalkyl groups (having preferably 3 to 15 carbon atoms), and alkenyl groups (having preferably 2 to 6 carbon atoms). In these groups, $-CH_2-$ may be replaced with one or a combination of two or more selected from the group consisting of -CO-, -NH-, -O-, -S-, -SO-, and $-SO_2-$.

The above alkylene, cycloalkylene, and alkenylene groups may each have a substituent. No particular limitation is imposed on the substituent, and examples thereof include halogen atoms (preferably a fluorine atom).

In a compound PIIa-1 formed by replacing each of the organic cations represented by $M_{61a}^+$ and $M_{61b}^+$ in formula (IIa-1) with $H^+$, the acid dissociation constant a1-7 derived from an acidic moiety represented by $A_{61a}H$ and the acid dissociation constant a1-8 derived from an acidic moiety represented by $A_{61b}H$ each correspond to the acid dissociation constant a1 described above.

The compound PIIa-1 formed by replacing each of the cationic moieties $M_{61a}^+$ and $M_{61b}^+$ in the structural moieties X in the compound (IIa-1) corresponds to $HA_{61a}$-$L_{61}$-$N(R_{2X})$-$L_{62}$-$A_{61b}H$. The compound PIIa-1 is the same as the acid generated from the compound represented by formula (IIa-1) upon irradiation with actinic rays or radiation.

At least one of $M_{61a}^+$, $M_{61b}^+$, $A_{61a}^-$, $A_{61b}^-$, $L_{61}$, $L_{62}$, or $R_{2X}$ may have an acid-decomposable group as a substituent.

The definitions of $A_{71a}^-$, $A_{71b}^-$, and $A_{71c}^-$ in formula (IIa-2) are each the same as that of $A_{11}^-$ in formula (Ia-1) above, and their preferred modes are also the same as that of $A_{11}^-$. The definitions of $M_{71a}^+$, $M_{71b}^+$, and $M_{71c}^+$ are each the same as that of $M_{11}^+$ in formula (Ia-1), and their preferred modes are also the same as that of $M_{11}^+$.

The definitions of $L_{71}$, $L_{72}$, and $L_{73}$ in formula (IIa-2) are each the same as that of $L_1$ in formula (Ia-1) above, and their preferred modes are also the same as that of $L_1$.

In a compound PIIa-2 formed by replacing each of the organic cations represented by $M_{71a}^+$, $M_{71b}^+$, and $M_{71c}^+$ in formula (IIa-2) with $H^+$, the acid dissociation constant a1-9 derived from an acidic moiety represented by $A_{71a}H$, the acid dissociation constant a1-10 derived from an acidic moiety represented by $A_{71b}H$, and the acid dissociation constant a1-11 derived from an acidic moiety represented by $A_{71c}H$ each correspond to the acid dissociation constant a1 described above.

The compound PIIa-2 formed by replacing each of the cationic moieties $M_{71a}^+$, $M_{71b}^+$, and $M_{71c}^+$ in the structural moieties X in the compound (IIa-1) with $H^+$ corresponds to $HA_{71a}$-$L_{71}$-$N(L_{73}$-$A_{71c}H)$-$L_{72}$-$A_{71b}H$. The compound PIIa-2 is the same as the acid generated from the compound represented by formula (IIa-2) upon irradiation with actinic rays or radiation.

At least one of $M_{71a}^+$, $M_{71b}^+$, $M_{71c}^+$, $A_{71a}^-$, $A_{71b}^-$, $A_{71c}^-$, $L_{71}$, $L_{72}$, or $L_{73}$ may have an acid-decomposable group as a substituent.

Examples of moieties that the compounds (I) to (II) can have and that differ from the cations are shown below.

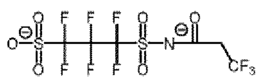
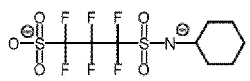
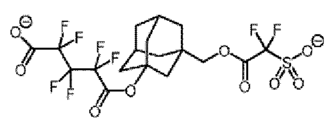
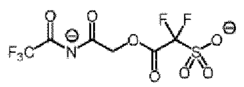
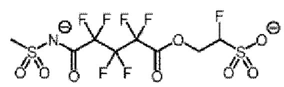
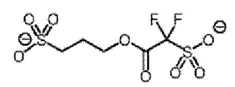
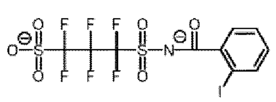
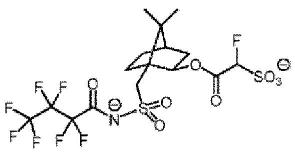
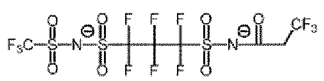
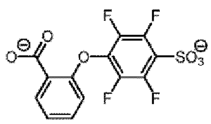
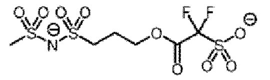
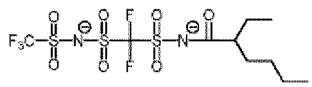
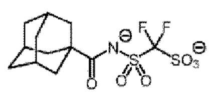
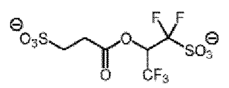
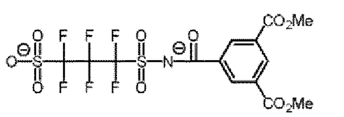
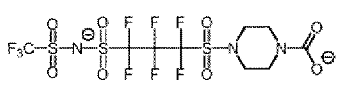
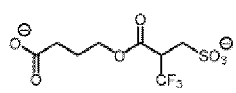
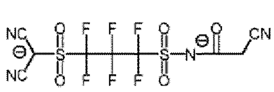
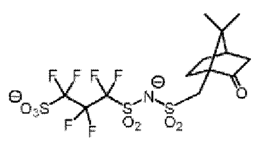
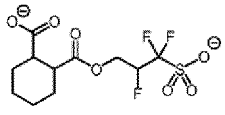
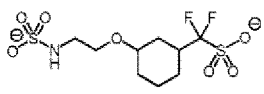

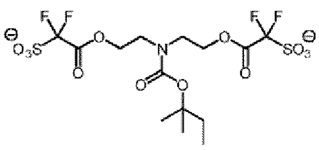
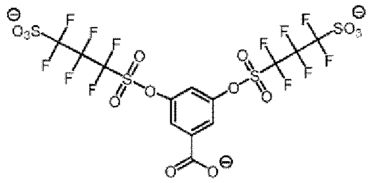

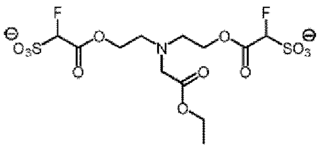
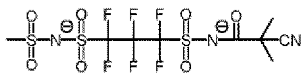

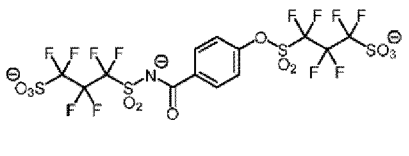
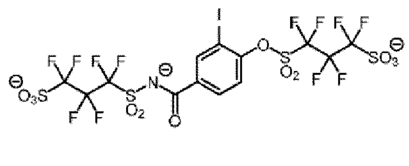

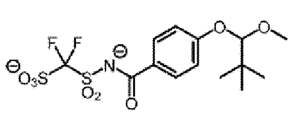
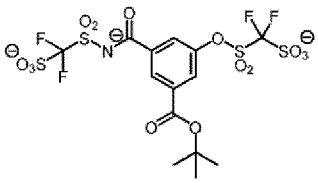
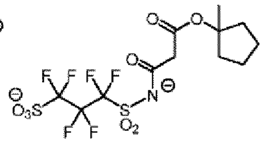

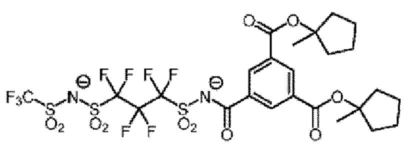
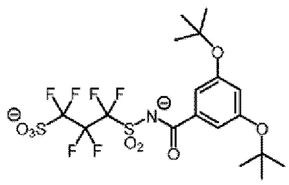

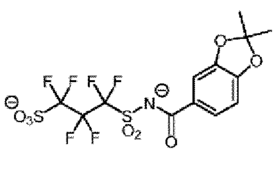
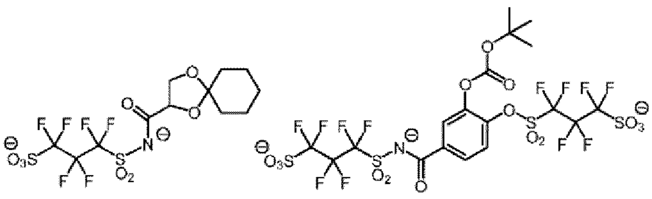

Specific examples of the photoacid generator (B) are shown below, but the photoacid generator (B) is not limited thereto.

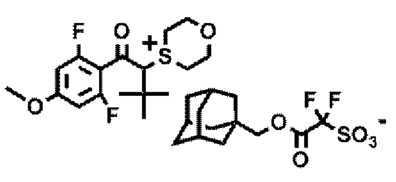

X-1

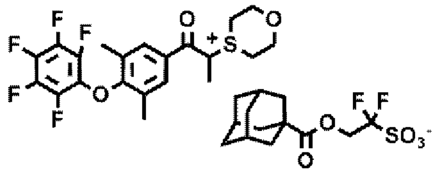

X-2

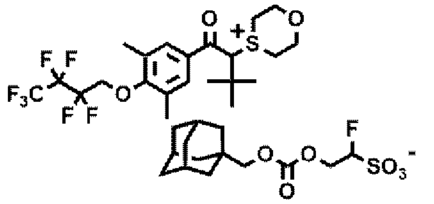

X-3

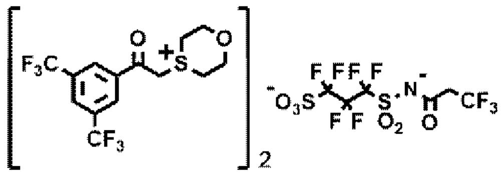

X-4

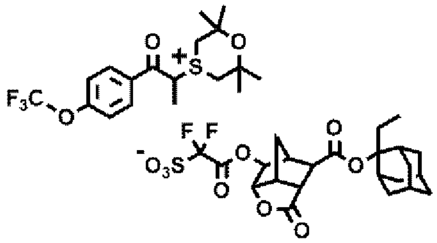

X-5

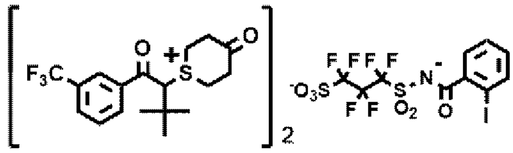

X-6

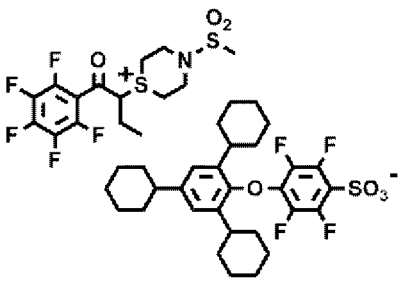

X-7

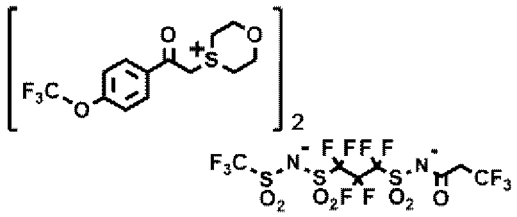

X-8

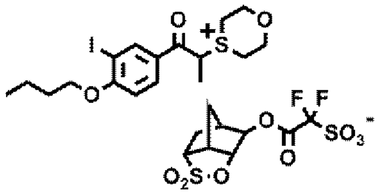

X-9

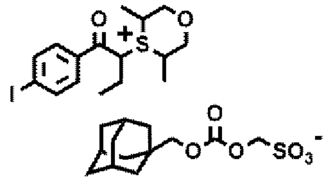

X-10

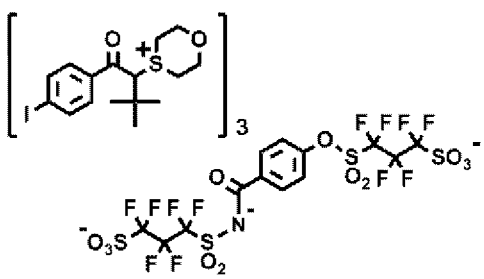

X-11

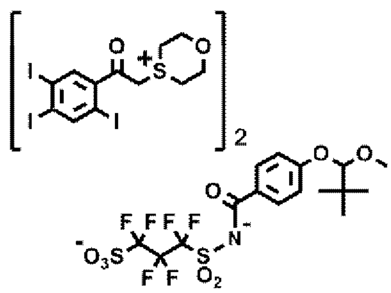

X-12

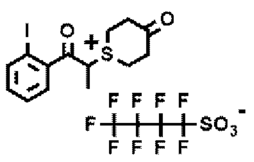

X-13

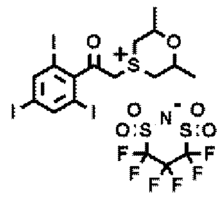

X-14

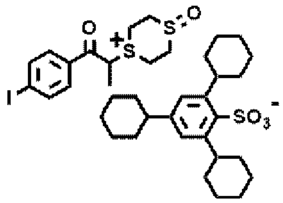

X-15

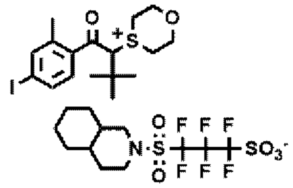

X-16

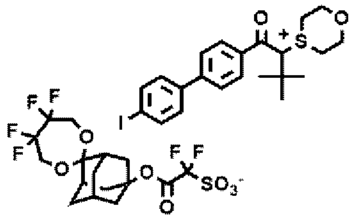

X-17

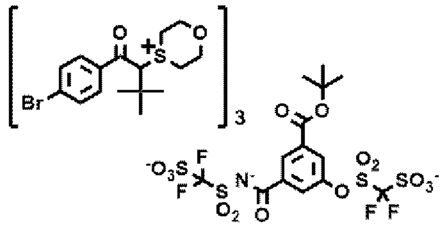

X-18

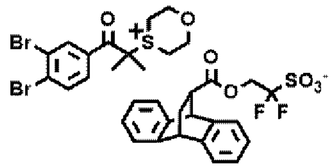

X-19

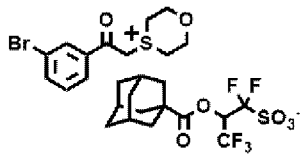

X-20

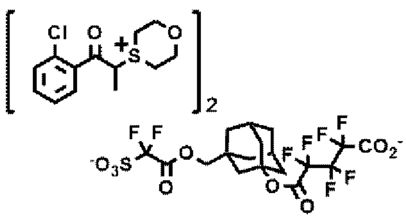

X-21

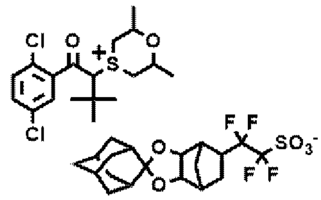

X-22

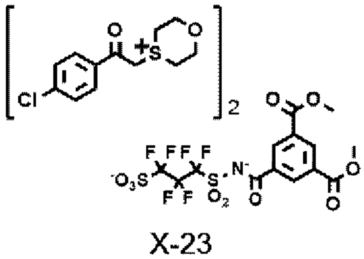

X-23

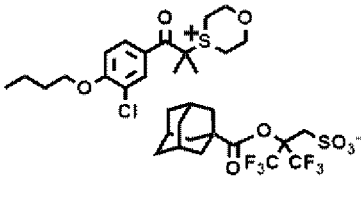

X-24

The composition of the invention may or may not include the photoacid generator (B). When the composition of the invention includes the photoacid generator (B), the content of the photoacid generator (B) with respect to the total mass of the solids in the composition of the invention is preferably 0.5% by mass or more and more preferably 1.0% by mass or more. When the composition of the invention includes the photoacid generator (B), the content of the photoacid generator (B) with respect to the total mass of the solids in the composition of the invention is preferably 50.0% by mass or less, more preferably 30.0% by mass or less, and still more preferably 25.0% by mass or less.
One photoacid generator (B) may be used alone, or two or more photoacid generators (B) may be used.

<Acid diffusion control agent>

The composition of the invention may include an acid diffusion control agent.
The acid diffusion control agent functions as a quencher that traps the acid generated from the photoacid generator etc. during exposure to light to thereby suppress the reaction of the acid decomposable resin with an excess portion of the generated acid in unexposed portions.
No particular limitation is imposed on the acid diffusion control agent, and examples thereof include a basic compound (CA), a low-molecular weight compound (CB) having a nitrogen atom and having a group that leaves by the action of an acid, and a compound (CC) whose acid diffusion control ability decreases or disappears when the compound (CC) is irradiated with actinic rays or radiation.
Examples of the compound (CC) include an onium salt compound (CD) that serves as a weak acid weaker than the photoacid generator and a basic compound (CE) whose basicity decreases or disappears upon irradiation with actinic rays or radiation.
Specific examples of the basic compound (CA) include those described in paragraphs [0132] to [0136] of WO2020/066824A, and specific examples of the basic compound (CE) whose basicity decreases or disappears upon irradiation with actinic rays or radiation include those described in paragraphs [0137] to [0155] of WO2020/066824A. Specific examples of the low-molecular weight compound (CB) having a nitrogen atom and having a group that leaves by the action of an acid include those described in paragraphs [0156] to [0163] of WO2020/066824A, and specific examples of an onium salt compound (CE) that has a nitrogen atom in its cationic moiety include those described in paragraph [0164] of WO2020/066824A.
Specific examples of the onium salt compound (CD) that serves as a weak acid weaker than the photoacid generator include those described in paragraphs [0305] to [0314] of WO2020/158337A.
In addition to the compounds described above, for example, known compounds disclosed in paragraphs [0627] to [0664] of US2016/0070167A, paragraphs [0095] to [0187] of US2015/0004544A, paragraphs [0403] to [0423] of US2016/0237190A, and paragraphs [0259] to [0328] of US2016/0274458A can be preferably used as the acid diffusion control agent.
When the composition of the invention includes the acid diffusion control agent, the content of the acid diffusion control agent (the total content when a plurality of acid diffusion control agents are present) with respect to the total amount of the solids in the composition of the invention is preferably 0.1 to 30.0% by mass, more preferably 0.1 to 15.0% by mass, and still more preferably 1.0 to 15.0% by mass.
In the composition of the invention, one acid diffusion control agent may be used alone, or a combination of two or more acid diffusion control agents may be used.

<Hydrophobic resin>

The composition of the invention may further include a hydrophobic resin different from the resin (A).
Preferably, the hydrophobic resin is designed so as to segregate on the surface of a resist film to be formed using the composition of the invention. However, it is not always necessary that, unlike a surfactant, the hydrophobic resin have a hydrophilic group in its molecule and contribute to uniform mixing of polar and nonpolar substances.
The effects of the addition of the hydrophobic resin include control of the static and dynamic contact angles of water on the surface of the resist film and reduction of outgassing.
From the viewpoint of segregation of the hydrophobic resin in a surface layer of the film, the hydrophobic resin has preferably at least one of a fluorine atom, a silicon atom, or a $CH_3$ partial structure included in a side chain portion of the resin and has more preferably two or more of them. Preferably, the hydrophobic resin has a hydrocarbon group having 5 or more carbon atoms. Each of these groups may be present as a substituent in the main chain of the resin or its side chain.
Examples of the hydrophobic resin include compounds described in paragraphs [0275] to [0279] of WO2020/004306A.
When the composition of the invention includes the hydrophobic resin, the content of the hydrophobic resin with respect to the total amount of the solids in the composition of the invention is preferably 0.01 to 20.0% by mass

and more preferably 0.1 to 15.0% by mass.

<Surfactant>

The composition of the invention may include a surfactant. When the surfactant is included, the compound has better adhesiveness, and a pattern with less development defects can be formed.

The surfactant is preferably a fluorine-based surfactant and/or a silicon-based surfactant.

Examples of the fluorine-based surfactant and/or the silicon-based surfactant include surfactants disclosed in paragraphs [0218] and [0219] of WO2018/19395A.

One of these surfactants may be used alone, or two or more of them may be used.

When the composition of the invention includes the surfactant, the content of the surfactant with respect to the total amount of the solids in the composition of the invention is preferably 0.0001 to 2.0% by mass, more preferably 0.0005 to 1.0% by mass, still more preferably 0.01 to 1.0% by mass, and particularly preferably 0.1 to 1.0% by mass.

<Solvent>

Preferably, the composition of the invention includes a solvent.

Preferably, the solvent includes at least one of (M1) propylene glycol monoalkyl ether carboxylate or (M2) at least one selected from the group consisting of propylene glycol monoalkyl ethers, lactates, acetates, alkoxypropionates, chain ketones, cyclic ketones, lactones, and alkylene carbonates. The solvent may further include a component other than the components (M1) and (M2).

The details of the components (M1) and (M2) are described in paragraphs [0218] to [0226] of WO2020/004306A, the contents of which are incorporated herein.

When the solvent further includes a component other than the components (M1) and (M2), the content of the component other than the components (M1) and (M2) with respect to the total amount of the solvent is preferably 5 to 30% by mass.

The content of the solvent in the composition of the invention is determined such that the concentration of the solid contents in the composition of the invention is preferably 0.5 to 30% by mass and more preferably 1 to 20% by mass. In this case, the coatability of the composition of the invention can be further improved.

<Additional additives>

The composition of the invention may further include a dissolution inhibiting compound, a dye, a plasticizer, a photosensitizer, a light absorber, and/or a compound capable of increasing the solubility in a developer (such as a phenol compound having a molecular weight of 1000 or less or an alicyclic or aliphatic compound including a carboxy group).

The composition of the invention may further include a dissolution inhibiting compound. The "dissolution inhibiting compound" is a compound that has a molecular weight of 3000 or less and is decomposed by the action of an acid to cause the degree of solubility of the composition of the invention in an organic-based developer to decrease.

The composition of the invention is preferably used as a photosensitive composition for EUV light.

The wavelength of the EUV light is 13.5 nm and is shorter than the wavelength of ArF light (wavelength: 193 nm) etc., and the number of incident photons when light exposure is performed at the same sensitivity is smaller. Therefore, the influence of "photon shot noise," i.e., stochastic variations in the number of photons, is large, and this causes an increase in LER and bridge defects. One method to reduce the photon shot noise is to increase the exposure value to increase the number of incident photons, but there is a trade-off with a demand for higher sensitivity.

When the value of A determined by formula (1) is large, the efficiency of absorption of EUV light and electron beams by a resist film formed by the composition of the invention is high, and this is effective in reducing the photon shot noise. The value of A means the efficiency of absorption of EUV light and electron beams by a resist film having a specific mass ratio.

$$\text{Formula (1): } A = ([H] \times 0.04 + [C] \times 1.0 + [N] \times 2.1 + [O] \times 3.6 + [F] \times 5.6 + [S] \times 1.5 + [I] \times 39.5) / ([H] \times 1 + [C] \times 12 + [N] \times 14 + [O] \times 16 + [F] \times 19 + [S] \times 32 + [I] \times 127)$$

The value of A is preferably 0.120 or more. No particular limitation is imposed on the upper limit of the value of A. However, if the value of A is excessively large, the EUV light and electron beam transmittance of the resist film decreases, and the profile of an optical image in the resist film deteriorates, so that a good pattern shape is unlikely to be obtained. Therefore, the value of A is preferably 0.240 or less and more preferably 0.220 or less.

In formula (1), [H] represents the molar ratio of hydrogen atoms derived from the total solids in the actinic ray-sensitive or radiation-sensitive resin composition with respect to all the atoms in the total solids, and [C] represents the molar ratio of carbon atoms derived from the total solids in the actinic ray-sensitive or radiation-sensitive resin composition with respect to all the atoms in the total solids. [N] represents the molar ratio of nitrogen atoms derived from the total solids in the actinic ray-sensitive or radiation-sensitive resin composition with respect to all the atoms in the total solids, and [O] represents the molar ratio of oxygen atoms derived from the total solids in the actinic ray-sensitive or radiation-sensitive resin composition with respect to all the atoms in the total solids. [F] represents the molar ratio of fluorine atoms derived from the total solids in the actinic ray-sensitive or radiation-sensitive resin composition with respect to all the atoms in the total solids, and [S] represents the molar ratio of sulfur atoms derived from the total solids in the actinic ray-sensitive or radiation-sensitive resin composition with respect to all the atoms in the total solids. [I] represents the molar ratio of iodine atoms derived from the total solids in the actinic ray-sensitive or radiation-sensitive resin composition with respect to all the atoms in the total solids.
For example, when the resist composition includes the acid decomposable resin, the photoacid generator, the acid diffusion control agent, and the solvent, the acid decomposable resin, the photoacid generator, and the acid diffusion control agent correspond to the solids. Specifically, all the atoms in the total solids correspond to the sum of all the atoms derived from the resin, all the atoms derived from the photoacid generator, and all the atoms derived from the acid diffusion control agent.
For example, [H] represents the molar ratio of hydrogen atoms derived from the total solids with respect to all the atoms in the total solids. In the above example, [H] represents the total molar ratio of hydrogen atoms derived from the acid decomposable resin, hydrogen atoms derived from the photoacid generator, and hydrogen atoms derived from the acid diffusion control agent with respect to the sum of all the atoms derived from the acid decomposable resin, all the atoms derived from the photoacid generator, and all the atoms derived from the acid diffusion control agent.
When the structures of the constituent components of the total solids in the composition of the invention and their contents are known, the value of A can be computed by computing the ratio of the numbers of atoms included in the composition. Even when the constituent components are unknown, the ratio of the numbers of constituent atoms can be computed by subjecting a film obtained by evaporating the solvent component in the composition to an analytical method such as elemental analysis.

<Resist film and pattern forming method>

The present invention also relates to a resist film formed using the composition of the invention.
The present invention also relates to a pattern forming method using the resist film formed using the composition of the invention.
No particular limitation is imposed on the procedure of the pattern forming method of the invention, but it is preferable that the method include the following steps.
Step 1: The step of forming a resist film on a substrate using the composition of the invention.
Step 2: The step of exposing the resist film to light.
Step 3: The step of developing the exposed resist film using a developer.
The procedure of each of the steps will next be described in detail.

(Step 1: resist film forming step)

Step 1 is the step of forming a resist film on a substrate using the composition of the invention.
The composition of the invention is as described above.
Examples of the method for forming a resist film on a substrate using the composition of the invention include a method in which the composition of the invention is applied to the substrate.
Preferably, the composition of the invention is filtrated through a filter before the application as needed. The pore size of the filter is preferably 0.1 μm or less, more preferably 0.05 μm or less, and still more preferably 0.03 μm or less. The filer is preferably a polytetrafluoroethylene-made filter, a polyethylene-made filter, or a nylon-made filter.
The composition of the invention can be applied to a substrate (e.g., a silicon substrate or a silicon dioxide coating) used for production of an integrated circuit element using an appropriate application method using a spinner, a coater, etc. The application method is preferably spin coating using a spinner. The number or revolutions when the spin coating using a spinner is performed is preferably 1000 to 3000 rpm.
After the application of the composition of the invention, the substrate may be dried to thereby form the resist film. If necessary, an undercoat film (an inorganic film, an organic film, or an antireflection film) may be formed as an underlayer of the resist film.
Examples of the drying method include a method in which the substrate is heated and dried. The heating may be performed using heating means included in an ordinary exposing device and/or an ordinary developing device or

may be performed using a hot plate etc. The heating temperature is preferably 80 to 150°C, more preferably 80 to 140°C, and still more preferably 80 to 130°C. The heating time is preferably 30 to 1000 seconds, more preferably 60 to 800 seconds, and still more preferably 60 to 600 seconds.

No particular limitation is imposed on the film thickness of the resist film, but the film thickness is preferably 10 to 120 nm because a finer pattern can be formed with higher accuracy. In particular, when the resist film is exposed to EUV light, the film thickness of the resist film is more preferably 10 to 65 nm and still more preferably 15 to 50 nm. When ArF liquid immersion exposure is performed, the film thickness of the resist film is more preferably 10 to 120 nm and still more preferably 15 to 90 nm.

A topcoat may be formed on the resist film using a topcoat composition.

It is preferable that the topcoat composition is immiscible with the resist film and can be uniformly applied to the upper surface of the resist film. No particular limitation is imposed on the topcoat, and a well-known topcoat can be formed using a well-known method. For example, the topcoat can be formed using a method described in paragraphs [0072] to [0082] of JP2014-059543A.

It is preferable, for example, that a topcoat including a basic compound described in JP2013-61648A is formed on the resist film. Specific examples of the basic compound that can be included in the topcoat include basic compounds that can be included in the resist composition.

It is also preferable that the topcoat includes a compound including at least one group or bond selected from the group consisting of an ether bond, a thioether bond, a hydroxy group, a thiol group, a carbonyl bond, and an ester bond.

(Step 2: Exposure step)

Step 2 is the step of exposing the resist film to light.

Examples of the light exposure method include a method in which the resist film formed is irradiated with actinic rays or radiation through a prescribed mask.

Examples of the actinic rays or radiation include infrared rays, visible rays, ultraviolet rays, far-ultraviolet rays, extreme ultraviolet rays, X rays, and electron beams. Far-ultraviolet rays having a wavelength of preferably 250 nm or shorter, more preferably 220 nm or shorter, and particularly preferably 1 to 200 nm are preferred, and specific examples include KrF excimer laser light (248 nm), ArF excimer laser light (193 nm), $F_2$ excimer laser light (157 nm), EUV light (13 nm), X rays, and electron beams.

It is preferable to perform baking (heating) after the light exposure but before development. The baking facilitates the reaction in the exposed portions, and the sensitivity and the pattern shape are further improved.

The heating temperature is preferably 80 to 150°C, more preferably 80 to 140°C, and still more preferably 80 to 130°C.

The heating time is preferably 10 to 1000 seconds, more preferably 10 to 180 seconds, and still more preferably 30 to 120 seconds.

The heating may be performed using heating means included in an ordinary exposing device and/or an ordinary developing device or may be performed using a hot plate etc.

This step is referred to as post-exposure baking.

(Step 3: Developing step)

Step 3 is the step of developing the exposed resist film with a developer to form a pattern.

The developer may be an alkali developer or may be a developer including an organic solvent (hereinafter referred to as an organic-based developer).

Examples of the developing method include: a method in which the substrate is dipped into a bath filled with the developer for a prescribed time (a dipping method); a method in which the developer is placed on the surface of the substrate so as to bulge due to surface tension and left to stand for a prescribed time to develop the resist film (a puddle method); a method in which the developer is sprayed onto the surface of the substrate (a spraying method); and a method in which the developer is continuously discharged from a developer discharging nozzle onto the substrate rotating at a constant speed while the developer discharging nozzle is scanned at a constant speed (a dynamic dispensing method).

The step of replacing the solvent with another solvent to stop the development may be performed after the developing step.

No particular limitation is imposed on the developing time so long as the resin in unexposed portions is dissolved sufficiently, and the developing time is preferably 10 to 300 seconds and more preferably 20 to 120 seconds.

The temperature of the developer is preferably 0 to 50°C and more preferably 15 to 35°C.

The alkali developer used is preferably an aqueous alkali solution including an alkali. No particular limitation is imposed on the type of aqueous alkali solution. Examples of the aqueous alkali solution include aqueous alkali solutions

including quaternary ammonium salts typified by tetramethylammonium hydroxide, inorganic alkalis, primary amines, secondary amines, tertiary amines, alcohol amines, cyclic amines, etc. In particular, the alkali developer is preferably an aqueous solution of a quaternary ammonium salt typified by tetramethylammonium hydroxide (TMAH). An appropriate amount of an alcohol, a surfactant, etc. may be added to the alkali developer. The alkali concentration of the alkali developer is generally 0.1% to 20% by mass. The pH of the alkali developer is generally 10.0 to 15.0.

The organic-based developer is preferably a developer including at least one organic solvent selected from the group consisting of ketone-based solvents, ester-based solvents, alcohol-based solvents, amide-based solvents, ether-based solvents, and hydrocarbon-based solvents.

A mixture of a plurality of solvents selected from the above solvents may be used, or the organic-based developer may be mixed with water or a solvent other that the above solvents. The content of water with respect to the total mass of the developer is preferably less than 50% by mass, more preferably less than 20% by mass, and still more preferably less than 10% by mass, and it is particularly preferable that the developer includes substantially no water.

The content of the organic solvent with respect to the total mass of the organic-based developer is preferably from 50% by mass to 100% by mass inclusive, more preferably from 80% by mass to 100% by mass inclusive, still more preferably from 90% by mass to 100% by mass inclusive, and particularly preferably from 95% by mass to 100% by mass inclusive.

(Additional steps)

Preferably, the above pattern forming method further includes the step of, after step 3, washing with a rinsing solution.

Examples of the rinsing solution used in the rinsing step after the step of developing using the alkali developer include pure water. An appropriate amount of a surfactant may be added to the pure water.

An appropriate amount of a surfactant may be added to the rinsing solution.

No particular limitation is imposed on the rinsing solution used for the rinsing step after the step of developing using the alkali developer so long as the rinsing solution does not dissolve the pattern, and a solution including a general-purpose organic solvent can be used. Preferably, the rinsing solution used includes at least one organic solvent selected from the group consisting of hydrocarbon-based solvents, ketone-based solvents, ester-based solvents, alcohol-based solvents, amide-based solvents, and ether-based solvents.

No particular limitation is imposed on the method for the rinsing step, and examples thereof include: a method in which the rinsing solution is continuously discharged onto the substrate rotating at a constant speed (a spin coating method); a method in which the substrate is dipped into a bath filled with the rinsing solution for a prescribed time (a dipping method); and a method in which the rinsing solution is sprayed onto the surface of the substrate (a spraying method).

The pattern forming method of the invention may further include a heating (post-baking) step after the rinsing step. Through this step, the developer and the rinsing solution remaining between traces of the pattern and inside the pattern are removed by baking. Through this step, the resist pattern is annealed, and the effect of improving surface roughness of the pattern is obtained. The heating step after the rinsing step is performed at generally 40 to 250°C (preferably 90 to 200°C) for generally 10 seconds to 3 minutes (preferably 30 seconds to 120 seconds).

The pattern formed may be used as a mask to perform etching treatment on the substrate. Specifically, the pattern formed in step 3 may be used as a mask to process the substrate (or the underlayer film and the substrate) to thereby form a pattern on the substrate.

No particular limitation is imposed on the method for processing the substrate (or the underlayer film and the substrate). It is preferable that the pattern formed in step 3 is used as a mask and the substrate (or the underlayer film and the substrate) is dry-etched to form a pattern on the substrate. The dry etching is preferably oxygen plasma etching.

Preferably, the composition of the invention and various materials (such as the solvent, the developer, the rinsing solution, a composition for forming an antireflection film, and the composition for forming the topcoat) used in the pattern forming method of the invention include no impurities such as metals. The content of the impurities included in each of these materials is preferably 1 ppm by mass or less, more preferably 10 ppb by mass or less, still more preferably 100 ppt by mass or less, particularly preferably 10 ppt by mass or less, and most preferably 1 ppt by mass or less. No particular limitation is imposed on the lower limit of the content of the impurities, and the content is preferably 0 ppt by mass or more. Examples of the metal impurities include Na, K, Ca, Fe, Cu, Mg, Al, Li, Cr, Ni, Sn, Ag, As, Au, Ba, Cd, Co, Pb, Ti, V, W, and Zn.

Examples of a method for removing impurities such as metals from the above materials include filtration using a filter. The details of the filtration using a filer are described in paragraph [0321] of WO2020/004306A.

Examples of a method for reducing the amount of impurities such as metals included in the above materials include: a method in which raw materials including smaller amounts of metals are used as the raw materials forming the above materials; a method in which the raw materials forming the above materials are filtrated through a filter; and a

method in which distillation is performed under the condition that contamination is reduced as much as possible, for example, by coating the inside of the device used with Teflon (registered trademark).

Besides the filtration using a filter, an adsorbent may be used to remove impurities. The filtration using a filter and the absorbent may be used in combination. The adsorbent used may be a well-known adsorbent, and examples of the adsorbent that can be used include inorganic-based adsorbents such as silica gel and zeolite and organic-based adsorbents such as activated carbon. To reduce the amount of impurities such as metals included in the above materials, it is necessary to prevent the metal impurities from mixing in the production process. Whether the metal impurities have been sufficiently removed from the production device can be checked by measuring the content of metal components included in a washing solution used to clean the production device. The content of the metal components included in the washing solution after use is preferably 100 ppt (parts per trillion) by mass or less, more preferably 10 ppt by mass or less, and still more preferably 1 ppt by mass or less. No particular limitation is imposed on the lower limit of the content, and the content is preferably 0 ppt by mass or more.

An electrically conductive compound may be added to an organic treatment solution such as the rinsing solution in order to prevent failure of chemical solution pipes and various parts (such as filters, O-rings, and tubes) due to electrostatic charges and subsequent electrostatic discharge. No particular limitation is imposed on the electrically conductive compound, and examples thereof include methanol. No particular limitation is imposed on the amount of the electrically conductive compound added. From the viewpoint of maintaining preferred development characteristics or rinsing characteristics, the amount of the electrically conductive compound is preferably 10% by mass or less and more preferably 5% by mass or less. No particular limitation is imposed on the lower limit, and the amount of the electrically conductive compound is preferably 0.01% by mass or more.

The chemical solution pipes used may be, for example, SUS (stainless steel) pipes or pipes coated with antistatic-treated polyethylene, antistatic-treated polypropylene, or an antistatic-treated fluorocarbon resin (such as polytetrafluoroethylene or a perfluoroalkoxy resin). Similarly, antistatic-treated polyethylene, antistatic-treated polypropylene, or an antistatic-treated fluorocarbon resin (such as polytetrafluoroethylene or a perfluoroalkoxy resin) may be used for the filters and the O-rings.

<Method for manufacturing electronic device>

The present invention also relates to a method for manufacturing an electronic device including the pattern forming method described above and to an electronic device manufactured by the manufacturing method.

In preferred modes of the electronic device of the invention, the device is installed in electric and electronic devices (such as household electrical appliances and OA (Office Automation) devices, media-related devices, optical devices, and telecommunication devices).

The present invention also relates to the above-described compound represented by general formula (S1). The compound represented by general formula (S1) is as described above. A method for synthesizing the compound represented by general formula (S1) is exemplified in Examples below.

[EXAMPLES]

The present invention will be further described in detail by way of Examples. Materials, amounts used, ratios, treatment details, treatment procedures shown in the following Examples can be appropriately changed so long as they do not depart from the gist of the invention. Therefore, the scope of the present invention should not be construed as limited to the following Examples.

<Synthesis Example 1: Synthesis of compound C-1>

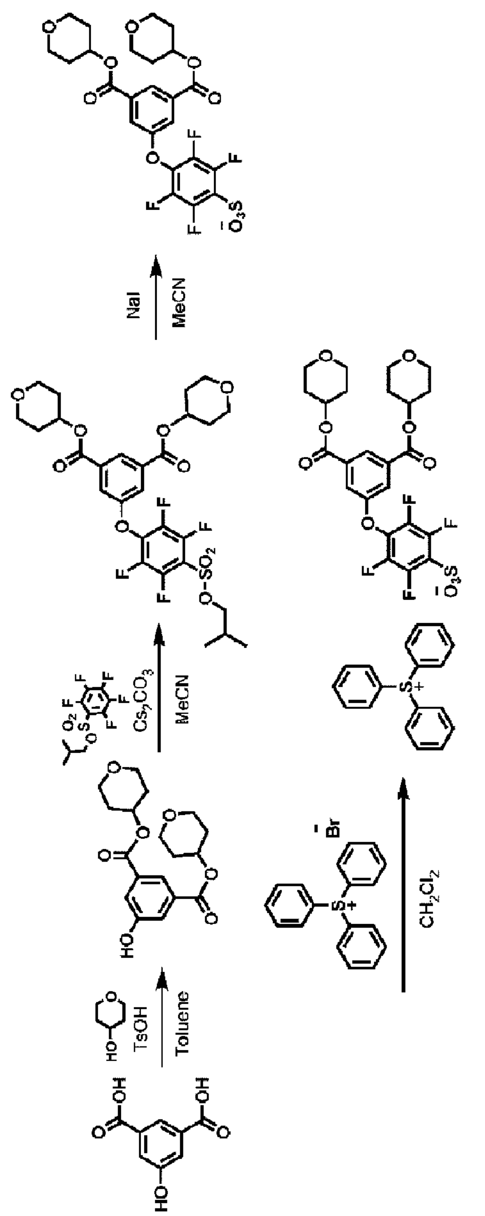


C-1

66 g of 5-hydroxyisophthalic acid, 148 g of 4-hydroxytetrahydropyran, 34.5 g of p-toluenesulfonic acid (TsOH), and 150 g of toluene were mixed, and the mixture was heated at reflux for 4 hours using a Dean-Stark apparatus and then cooled to room temperature. The obtained reaction solution was transferred to a separatory funnel. 400 g of ethyl acetate and 400 g of a saturated sodium bicarbonate solution were added, and the organic layer was separated. The obtained organic layer was washed twice with 400 g of ion exchanged water, and the solvent was removed by evaporation to thereby obtain 115 g of a white solid.

90 g of the obtained solid, 78 g of isobutyl pentafluorobenzenesulfonate, 126 g of cesium carbonate, and 987 g of acetonitrile (MeCN) were mixed, stirred at 60°C for 1 hour, and then cooled to room temperature. 500 g of ethyl acetate was added to the reaction solution, and the mixture was transferred to a separatory funnel. Then the organic layer was washed three times with 500 g of ion exchanged water, and the solvent was removed by evaporation to thereby obtain a brown oil. The oil was dissolved in 480 g of acetonitrile. Then 42.3 g of sodium iodide was added, and the mixture was stirred at 60°C for 2 hours (then a white solid was gradually precipitated). The reaction solution was cooled to room temperature, and the precipitated solid was separated by filtration. The solid residue was washed with acetonitrile to thereby obtain 104 g of a white solid.

2.95 g of the obtained solid, 35.4 g of methylene chloride, and 35.4 g of ion exchanged water were mixed. Then 1.79 g of triphenylsulfonium bromide was added, and the resulting mixture was stirred at room temperature for 30 minutes. The reaction solution was transferred to a separatory funnel, and the organic layer was washed twice with 70 g of 0.01 mol/L hydrochloric acid and twice with 70 g of ion exchanged water. The solvent was removed by evaporation,

and 3.5 g of the target compound (C-1) was obtained as a white solid. The compound was identified by NMR (Nuclear Magnetic Resonance).

$^{1}$HNMR (300 MHz, MeOD) $\delta$ = 8.46 (t,1H), 7.86-7.93 (m,5H), 7.78-7.83 (m,12H), 5.24 (m,2H), 3.98 (m,4H), 3.65 (m,4H), 2.08 (m,4H), 1.84 (m,4H).

$^{19}$FNMR (300 MHz, MeOD) $\delta$ = -139.81 (m,2F), -156.46 (m,2F).

Compounds C-2 to C-24 described later were synthesized in the same manner as above.

Components used for resist compositions in Examples and Comparative Examples are shown below.

[Acid decomposable resins (resins (A))]

Acid decomposable resins A-1 to A-27 each include repeating units shown in Table 1 below in contents (% by mole) shown in Table 1. Each repeating unit is shown using the structure of the corresponding monomer.

The weight average molecular weight (Mw), number average molecular weight (Mn), and dispersity (Mw/Mn) of each resin were measured by GPC (carrier: tetrahydrofuran (THF)) as described above. The compositional ratio (the contents of repeating units) of each resin was measured by $^{13}$C-NMR.

[Table 1]

| Resin (A) | Repeating unit 1 | | Repeating unit 2 | | Repeating unit 3 | | Repeating unit 4 | | Mw | Mw/Mn |
|---|---|---|---|---|---|---|---|---|---|---|
| | Type | Content (% by mole) | Type | Content (% by mole) | Type | Content (% by mole) | Type | Content (% by mole) | | |
| A-1 | MB-10 | 50 | MA-16 | 50 | - | - | - | - | 8500 | 1.60 |
| A-2 | MB-15 | 40 | MA-7 | 60 | - | - | - | - | 9000 | 1.70 |
| A-3 | MB-7 | 30 | MB-14 | 10 | MA-6 | 60 | - | - | 7000 | 1.55 |
| A-4 | MB-5 | 50 | MB-12 | 10 | MA-15 | 40 | - | - | 7500 | 1.55 |
| A-5 | MB-20 | 40 | MB-3 | 20 | MA-4 | 40 | - | - | 7000 | 1.60 |
| A-6 | MB-6 | 30 | MB-19 | 10 | MA-3 | 60 | - | - | 9500 | 1.45 |
| A-7 | MB-9 | 30 | MB-20 | 10 | MA-13 | 60 | - | - | 12000 | 1.65 |
| A-8 | MB-19 | 20 | MB-4 | 20 | MA-2 | 60 | - | - | 6000 | 1.55 |
| A-9 | MB-16 | 30 | MA-17 | 70 | - | - | - | - | 8000 | 1.40 |
| A-10 | MB-31 | 30 | MB-4 | 30 | MA-4 | 40 | - | - | 6500 | 1.65 |
| A-11 | MB-30 | 20 | MA-8 | 80 | - | - | - | - | 5500 | 1.65 |
| A-12 | MB-13 | 50 | MA-10 | 50 | - | - | - | - | 15000 | 1.75 |
| A-13 | MB-8 | 30 | MA-20 | 70 | - | - | - | - | 9000 | 1.60 |
| A-14 | MB-18 | 30 | MB-29 | 30 | MA-14 | 40 | - | - | 8000 | 1.55 |

(continued)

| Resin (A) | Repeating unit 1 | | Repeating unit 2 | | Repeating unit 3 | | Repeating unit 4 | | Mw | Mw/Mn |
|---|---|---|---|---|---|---|---|---|---|---|
| | Type | Content (% by mole) | Type | Content (% by mole) | Type | Content (% by mole) | Type | Content (% by mole) | | |
| A-15 | MB-20 | 20 | MB-3 | 30 | MA-2 | 50 | - | - | 7500 | 1.70 |
| A-16 | MB-1 | 30 | MB-26 | 30 | MA-11 | 40 | - | - | 18000 | 1.80 |
| A-17 | MB-27 | 60 | MA-5 | 40 | - | - | - | - | 7500 | 1.65 |
| A-18 | MB-17 | 30 | MB-24 | 10 | MA-19 | 60 | - | - | 8000 | 1.70 |
| A-19 | MB-11 | 30 | MB-28 | 40 | MA-9 | 30 | - | - | 9500 | 1.80 |
| A-20 | MB-20 | 10 | MB-3 | 20 | MB-32 | 10 | MA-2 | 60 | 10000 | 1.70 |
| A-21 | MB-2 | 60 | MA-1 | 40 | - | - | - | - | 11000 | 1.65 |
| A-22 | MB-21 | 50 | MA-12 | 50 | - | - | - | - | 6500 | 1.60 |
| A-23 | MB-23 | 40 | MA-10 | 60 | - | - | - | - | 8000 | 1.55 |
| A-24 | MB-19 | 20 | MB-1 | 20 | MA-2 | 60 | - | - | 8000 | 1.55 |
| A-25 | MB-25 | 30 | MA-2 | 70 | - | - | - | - | 7500 | 1.60 |
| A-26 | MB-3 | 40 | MA-3 | 60 | - | - | - | - | 9500 | 1.60 |
| A-27 | MB-22 | 40 | MA-4 | 60 | - | - | - | - | 10000 | 1.70 |

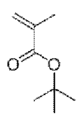
MA-1

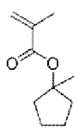
MA-2

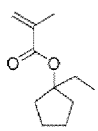
MA-3

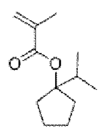
MA-4

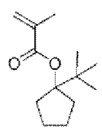
MA-5

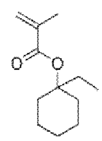
MA-6

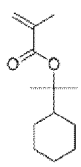
MA-7

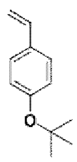
MA-8

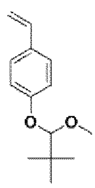
MA-9

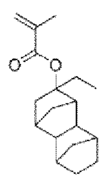
MA-10

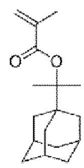
MA-11

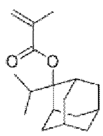
MA-12

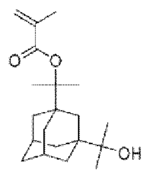
MA-13

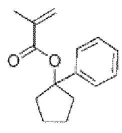
MA-14

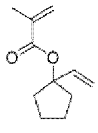
MA-15

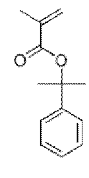
MA-16

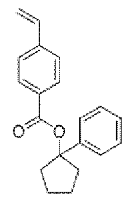
MA-17

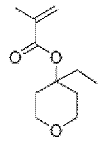
MA-18

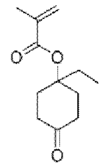
MA-19

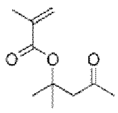
MA-20

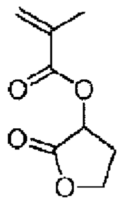
MB-1

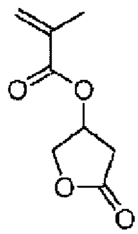
MB-2

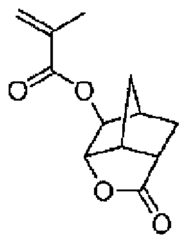
MB-3

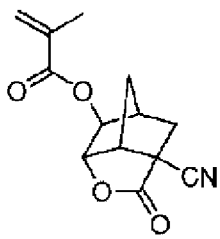
MB-4

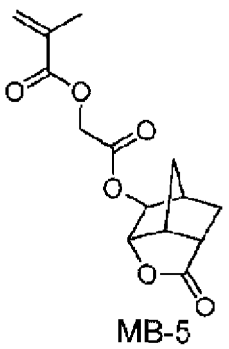
MB-5

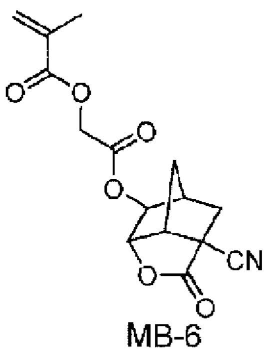
MB-6

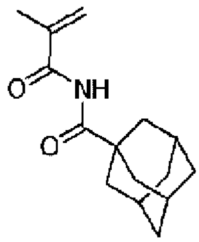
MB-7

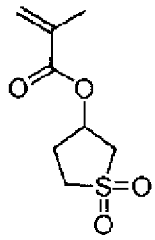
MB-8

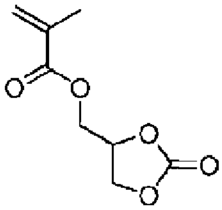
MB-9

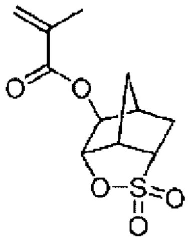
MB-10

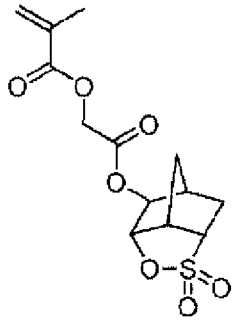
MB-11

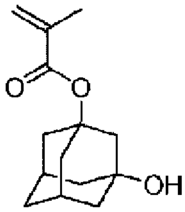
MB-12

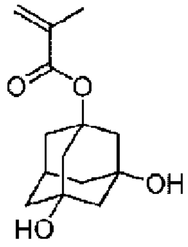
MB-13

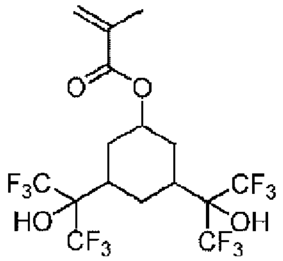
MB-14

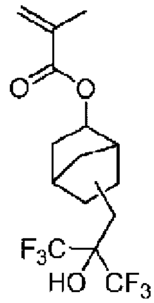
MB-15

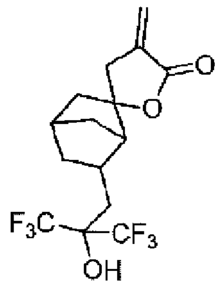
MB-16

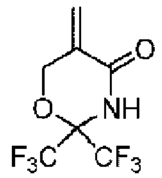
MB-17

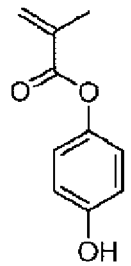
MB-18

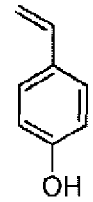
MB-19

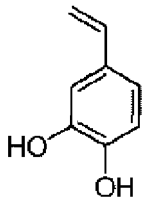
MB-20

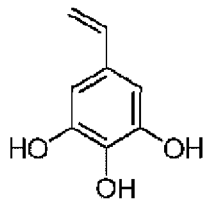
MB-21

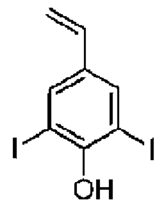
MB-22

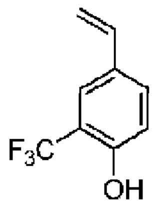
MB-23

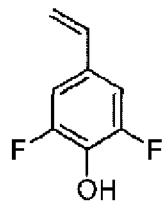
MB-24

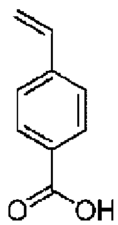
MB-25

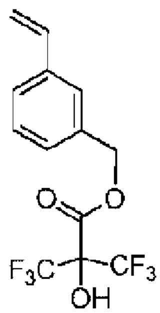
MB-26

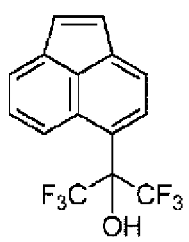
MB-27

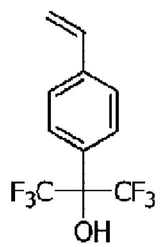
MB-28

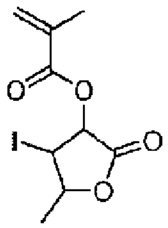

MB-29

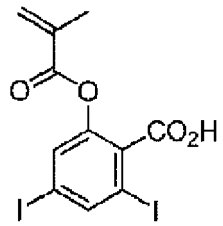

MB-30

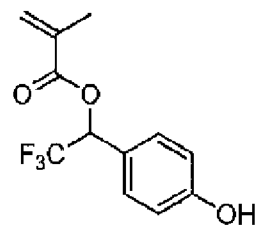

MB-31

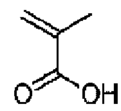

MB-32

[Photoacid generators]

The structures of compounds used as photoacid generators are shown below.

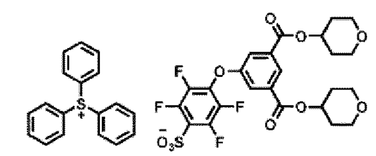

C-1

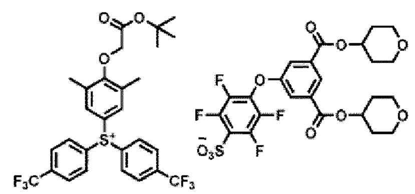

C-2

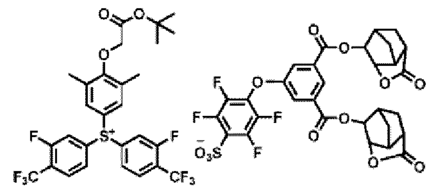

C-3

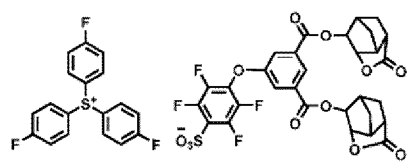

C-4

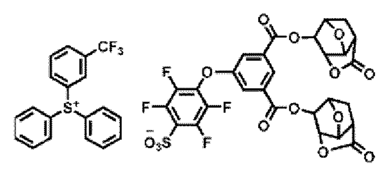

C-5

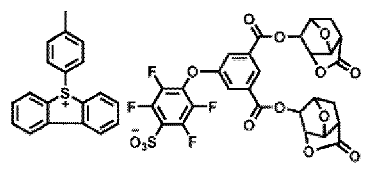

C-6

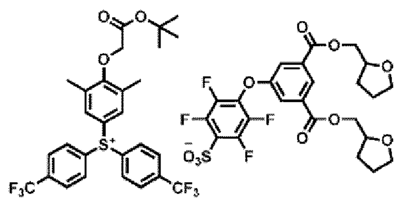

C-7

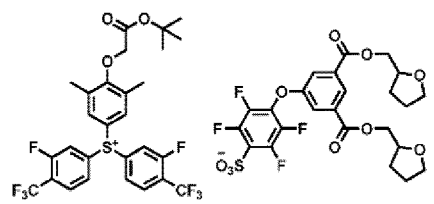

C-8

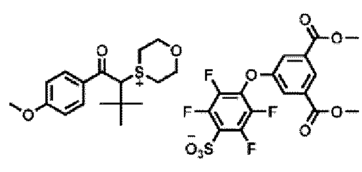

C-9

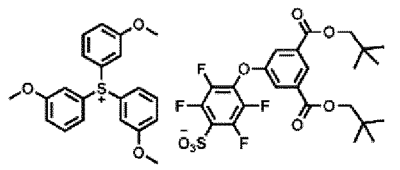

C-10

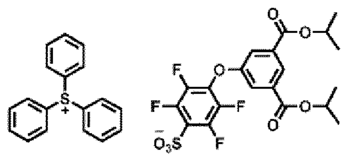

C-11

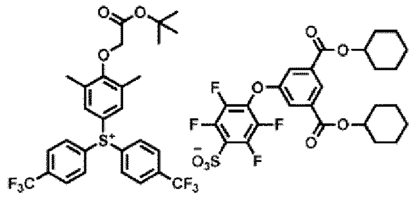

C-12

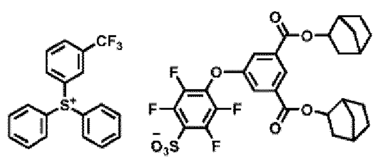

C-13

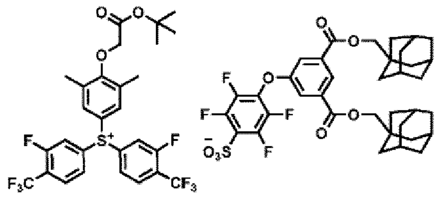

C-14

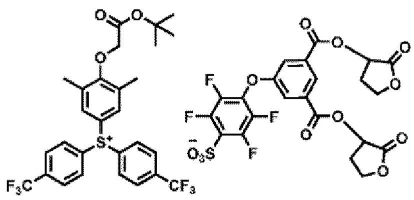

C-15

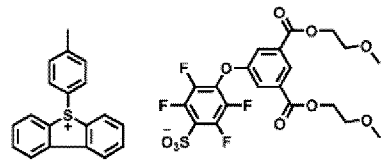

C-16

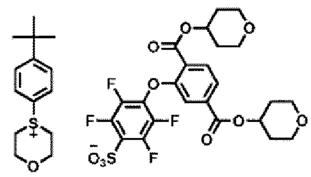

C-17

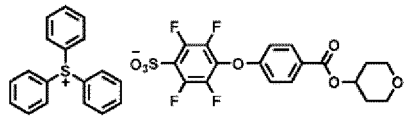

C-18

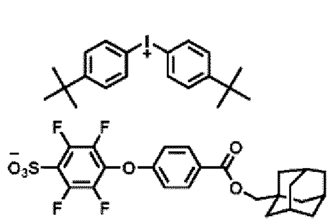

C-19

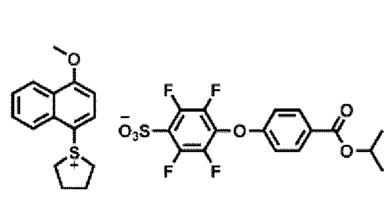

C-20

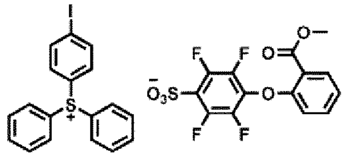

C-21

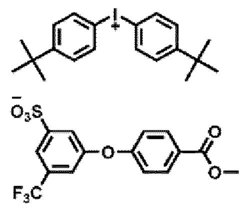

C-22

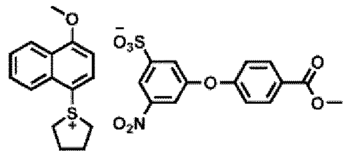

C-23

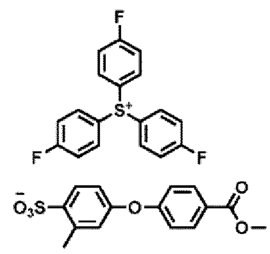

C-24

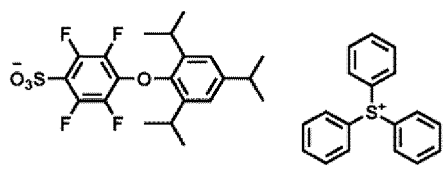

Z-1

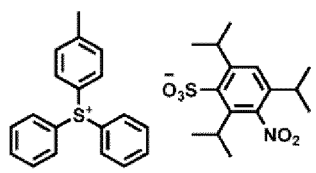

Z-2

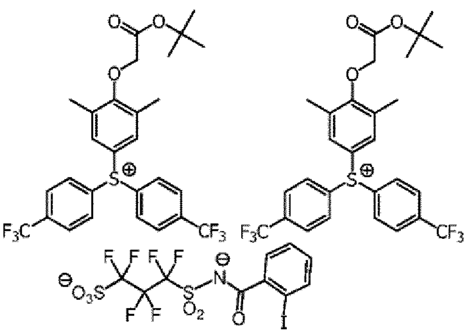

B-1

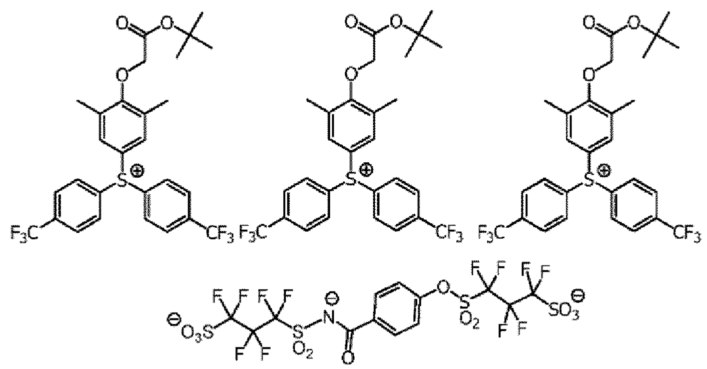

B-2

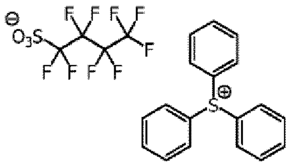

**B-3**

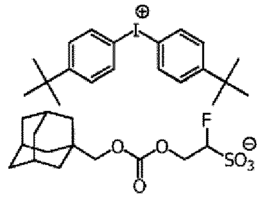

**B-4**

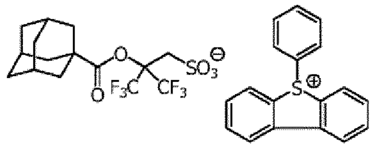

**B-5**

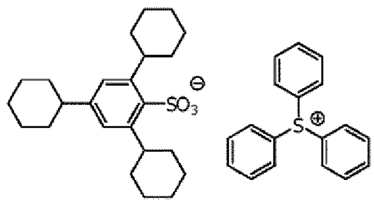

**B-6**

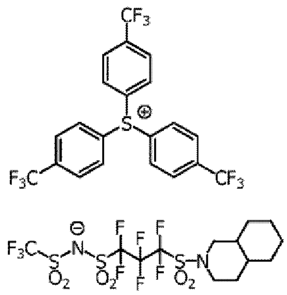

**B-7**

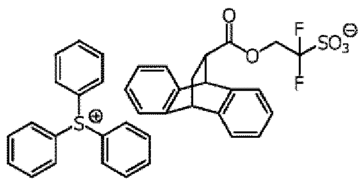

**B-8**

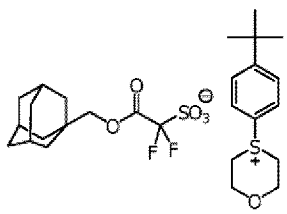

**B-9**

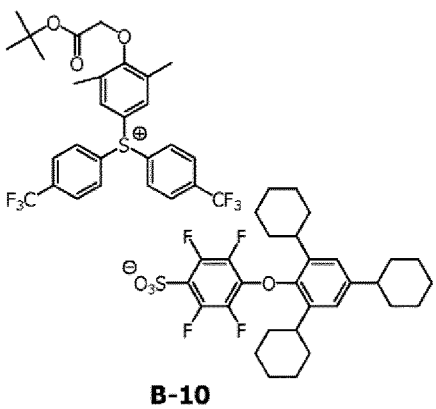

**B-10**

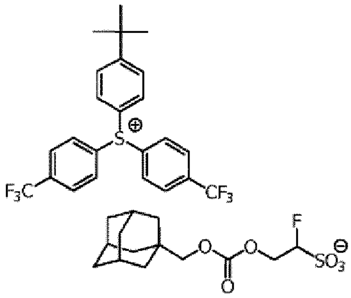

**B-11**

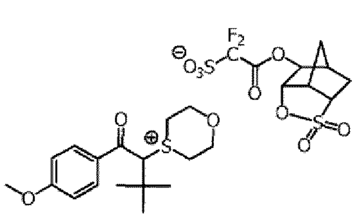

**B-12**

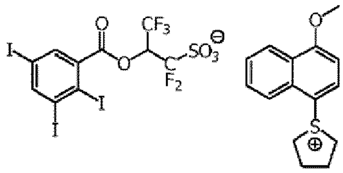

B-13

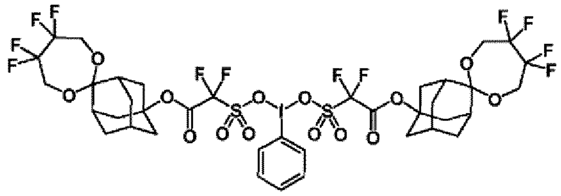

B-14

[Acid diffusion control agents]

The structure of compounds used as acid diffusion control agents are shown below.

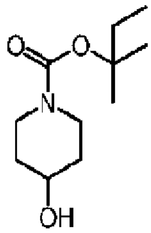

D-1

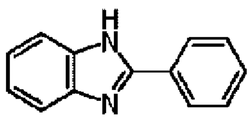

D-2

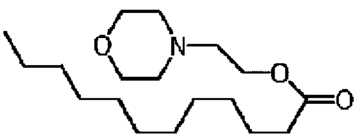

D-3

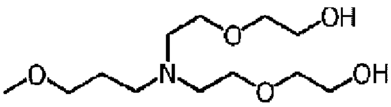

D-4

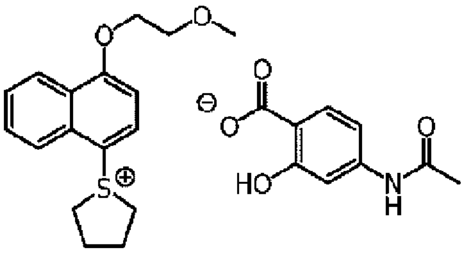

D-5

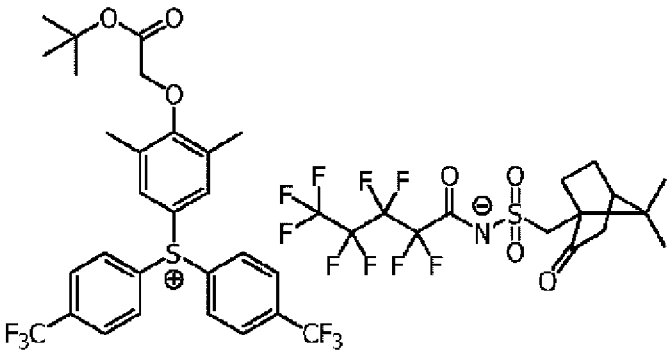

**D-6**

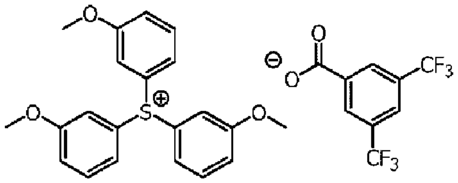

**D-7**

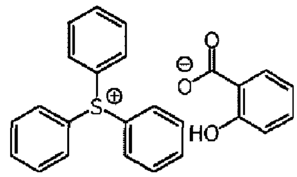

**D-8**

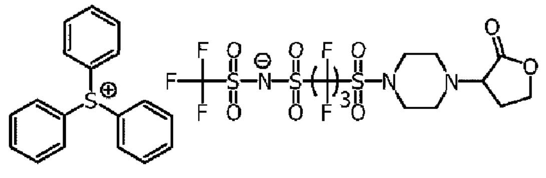

**D-9**

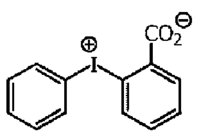

**D-10**

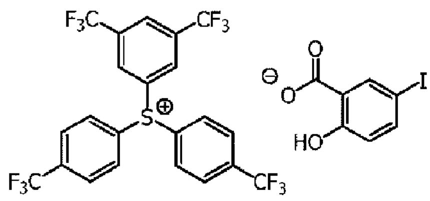

**D-11**

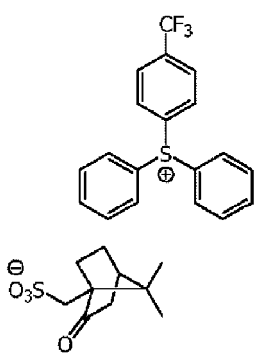

**D-12**

[Hydrophobic resins]

Hydrophobic resins E-1 to E-12 each include repeating units shown in Table 2 below in contents (% by mole) shown in Table 2. Each repeating unit is shown using the structure of the corresponding monomer.

[Table 2]

| Hydrophobic resin | Repeating unit 1 | | Repeating unit 2 | | Repeating unit 3 | | Repeating unit 4 | | Mw | Mw/Mn |
|---|---|---|---|---|---|---|---|---|---|---|
| | Type | Content (% by mole) | Type | Content (% by mole) | Type | Content (% by mole) | Type | Content (% by mole) | | |
| E-1 | ME-3 | 60 | ME-4 | 40 | - | - | - | - | 10000 | 1.40 |
| E-2 | ME-15 | 50 | ME-1 | 50 | - | - | - | - | 12000 | 1.50 |
| E-3 | ME-2 | 40 | ME-13 | 50 | ME-9 | 5 | ME-20 | 5 | 6000 | 1.30 |
| E-4 | ME-19 | 50 | ME-14 | 50 | - | - | - | - | 9000 | 1.50 |
| E-5 | ME-10 | 50 | ME-2 | 50 | - | - | - | - | 15000 | 1.50 |
| E-6 | ME-17 | 50 | ME-15 | 50 | - | - | - | - | 10000 | 1.50 |
| E-7 | ME-7 | 100 | - | - | - | - | - | - | 23000 | 1.70 |
| E-8 | ME-5 | 100 | - | - | - | - | - | - | 13000 | 1.50 |
| E-9 | ME-6 | 50 | ME-16 | 50 | - | - | - | - | 10000 | 1.70 |
| E-10 | ME-13 | 10 | ME-18 | 85 | ME-9 | 5 | - | - | 11000 | 1.40 |
| E-11 | ME-8 | 80 | ME-11 | 20 | - | - | - | - | 13000 | 1.40 |
| E-12 | ME-15 | 50 | ME-21 | 50 | - | - | - | - | 6500 | 1.65 |

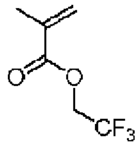
ME-1

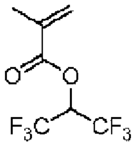
ME-2

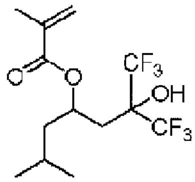
ME-3

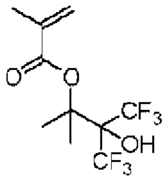
ME-4

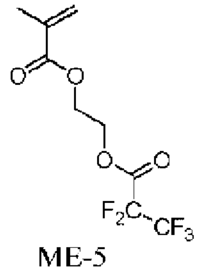
ME-5

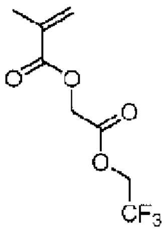
ME-6

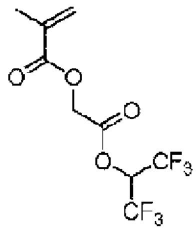
ME-7

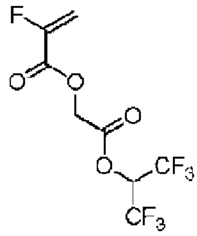
ME-8

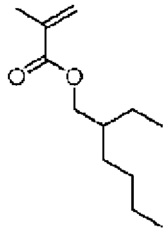
ME-9

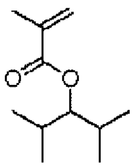
ME-10

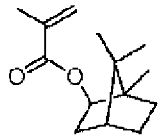
ME-11

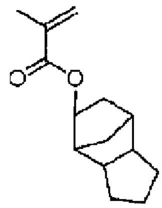
ME-12

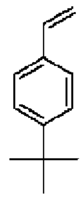
ME-13

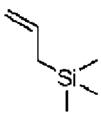
ME-14

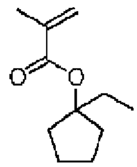
ME-15

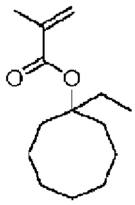
ME-16

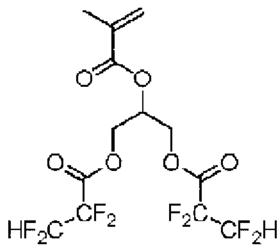
ME-17

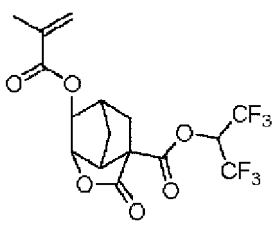
ME-18

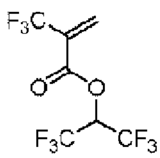
ME-19

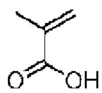
ME-20

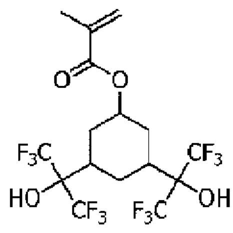
ME-21

[Surfactants]

The following H-1 to H-3 were used as surfactants.

H-1: MEGAFACE F176 (manufactured by DIC Corporation, fluorine-based surfactant)
H-2: MEGAFACE R08 (manufactured by DIC Corporation, fluorine and silicon-based surfactant)

H-3: PF656 (manufactured by OMNOVA, fluorine-based surfactant)

[Solvents]

The following F-1 to F-9 were used as solvents.

F-1: Propylene glycol monomethyl ether acetate (PGMEA)
F-2: Propylene glycol monomethyl ether (PGME)
F-3: Propylene glycol monoethyl ether (PGEE)
F-4: Cyclohexanone
F-5: Cyclopentanone
F-6: 2-Heptanone
F-7: Ethyl lactate
F-8: γ-Butyrolactone
F-9: Propylene carbonate

[Preparation of resist compositions]

Components shown in Tables 3 and 4 in amounts shown in Tables 3 and 4 were dissolved in solvents shown in Tables 3 and 4 to prepare solutions. The solutions were filtered through polyethylene filters having a pore size of 0.02 μm to prepare resist compositions Re-1 to Re-50. The concentration of solid contents in each resist composition was set to 2% by mass. The content of each component other than the solvents is the ratio (% by mass) with respect to the total solids in the resist composition. When two or more compounds were used as solvents, the mixing ratio (mass ratio) of the compounds is shown in Tables 3 and 4.

[Table 3]

| Resist composition | Photoacid generator 1 | | Acid decomposable resin | | Photoacid generator 2 | | Acid diffusion control agent | | Hydrophobic resin | | Surfactant | | Solvent | |
|---|---|---|---|---|---|---|---|---|---|---|---|---|---|---|
| | Type | Content (% by mass) | Type | Content (% by mass) | Type | Content (% by mass) | Type | Content (% by mass) | Type | Content (% by mass) | Type | Content (% by mass) | Type | Mixing ratio |
| Re-1 | C-1 | 45.3 | A-22 | 49.3 | | | D-3 | 5.4 | | | | | F-1/F-8 | 85/15 |
| Re-2 | C-2 | 35.2 | A-26 | 53.5 | | | D-11 | 10.2 | E-9 | 1.1 | | | F-1/F-2 | 70/30 |
| Re-3 | C-3 | 30.1 | A-2 | 67.8 | | | D-4 | 2.1 | | | | | F-1/F-7 | 80/20 |
| Re-4 | C-4 | 21.7 | A-18 | 73.1 | | | D-2 | 2.9 | E-3 | 2.3 | | | F-4 | 100 |
| Re-5 | C-5 | 38.3 | A-10 | 56.9 | | | D-2 | 4.8 | | | | | F-1/F-9 | 90/10 |
| Re-6 | C-6 | 25.5 | A-3 | 68.4 | | | D-8 | 4.1 | E-6 | 2 | | | F-1/F-6 | 40/60 |
| Re-7 | C-7 | 41.3 | A-25 | 52.2 | | | D-5 | 6.4 | | | H-3 | 0.1 | F-1/F-5 | 50/50 |
| Re-8 | C-8 | 23.2 | A-7 | 74.9 | | | D-3 | 1.9 | | | | | F-1 | 100 |
| Re-9 | C-9 | 29.1 | A-22 | 61.2 | | | D-12 | 9.7 | | | | | F-1/F-2/F-8 | 70/25/5 |
| Re-10 | C-10 | 33.4 | A-15 | 57.7 | | | D-4 | 8.9 | | | | | F-1 | 100 |
| Re-11 | C-11 | 59.8 | A-1 | 11.7 | | | D-6 | 25.1 | E-8 | 3.4 | | | F-7 | 100 |
| Re-12 | C-12 | 28.1 | A-10 | 67.7 | | | D-9 | 4.2 | | | | | F-1/F-2 | 70/30 |
| Re-13 | C-13 | 26.3 | A-15 | 70.3 | | | D-1 | 2.3 | E-5 | 1.1 | | | F-1/F-3 | 90/10 |
| Re-14 | C-14 | 19.7 | A-6 | 77.4 | | | D-8 | 2.9 | | | | | F-1/F-7 | 80/20 |

(continued)

| Resist composition | Photoacid generator 1 | | Acid decomposable resin | | Photoacid generator 2 | | Acid diffusion control agent | | Hydrophobic resin | | Surfactant | | Solvent | |
|---|---|---|---|---|---|---|---|---|---|---|---|---|---|---|
| | Type | Content (% by mass) | Type | Content (% by mass) | Type | Content (% by mass) | Type | Content (% by mass) | Type | Content (% by mass) | Type | Content (% by mass) | Type | Mixing ratio |
| Re-15 | C-15 | 35.6 | A-15 | 58.9 | | | D-10 | 5.5 | | | | | F-1/F-8 | 85/15 |
| Re-16 | C-16 | 27.7 | A-4 | 65.8 | | | D-7 | 6.5 | | | | | F-4 | 100 |
| Re-17 | C-17 | 20.3 | A-17 | 68.7 | | | D-10 | 7.9 | E-10 | 2.9 | H-1/H-2 | 0.1/0.1 | F-1/F-5 | 50/50 |
| Re-18 | C-18 | 37.9 | A-13/A-18 | 33.4/25 | | | D-2 | 3.7 | | | | | F-1 | 100 |
| Re-19 | C-19 | 34.4 | A-23 | 52.9 | | | D-6 | 12.7 | | | | | F-1/F-2/F-8 | 70/25/5 |
| Re-20 | C-20 | 36.5 | A-13 | 49.6 | | | D-7 | 13.9 | | | | | F-1/F-6 | 40/60 |
| Re-21 | C-21 | 45.5 | A-20 | 38.6 | | | D-9 | 14.4 | E-2 | 1.5 | | | F-4 | 100 |
| Re-22 | C-22 | 24.9 | A-18 | 67.9 | | | D-8 | 7.2 | | | | | F-1 | 100 |
| Re-23 | C-23 | 36.9 | A-21 | 51.8 | | | D-12 | 11.3 | | | | | F-1 | 100 |
| Re-24 | C-24 | 39.7 | A-14 | 50.6 | | | D-1 | 7.8 | E-12 | 1.9 | | | F-1/F-3 | 90/10 |
| Re-25 | C-19 | 42.1 | A-9 | 40.8 | B-7 | 7.1 | D-3 | 9.9 | | | H-2 | 0.1 | F-1/F-2 | 70/30 |

[Table 4]

| Resist composition | Photoacid generator 1 | | Acid decomposable resin | | Photoacid generator 2 | | Acid diffusion control agent | | Hydrophobic resin | | Surfactant | | Solvent | |
|---|---|---|---|---|---|---|---|---|---|---|---|---|---|---|
| | Type | Content (% by mass) | Type | Content (% by mass) | Type | Content (% by mass) | Type | Content (% by mass) | Type | Content (% by mass) | Type | Content (% by mass) | Type | Mixing ratio |
| Re-26 | C-1 | 10.7 | A-5 | 59.1 | B-2 | 30.2 | | | | | | | F-4 | 100 |
| Re-27 | C-20 | 35.7 | A-3 | 39.8 | B-6 | 5.6 | D-7 | 16.7 | E-1 | 2.2 | | | F-1/F-9 | 90/10 |
| Re-28 | C-13 | 22.3 | A-12 | 59.9 | B-10 | 4.5 | D-6 | 13.3 | | | | | F-1/F-2 | 70/30 |
| Re-29 | C-5 | 10.5 | A-20 | 57.9 | B-1 | 31.6 | | | | | | | F-1/F-8 | 85/15 |
| Re-30 | C-14 | 5.3 | A-10 | 65.2 | B-8 | 22 | D-4 | 6 | E-11 | 1.5 | | | F-7 | 100 |
| Re-31 | C-10 | 7.8 | A-8 | 54 | B-14 | 29 | D-8 | 9.2 | | | | | F-1/F-8 | 85/15 |
| Re-32 | C-22 | 6.5 | A-2 | 64.1 | B-13 | 25.9 | D-2 | 3.5 | | | | | F-1/F-2 | 70/30 |
| Re-33 | C-23 | 20.8 | A-24 | 53.5 | B-3 | 16.2 | D-10 | 7.7 | E-4 | 1.8 | | | F-1/F-7 | 80/20 |
| Re-34 | C-3 | 32 | A-16 | 52.2 | B-9 | 10.9 | D-3 | 4.9 | | | | | F-4 | 100 |
| Re-35 | C-17 | 17.3 | A-11 | 56 | B-5 | 12.6 | D-9 | 14 | | | H-1 | 0.1 | F-1/F-9 | 90/10 |
| Re-36 | C-11 | 15.3 | A-13 | 55.2 | B-11 | 19 | D-12 | 10.5 | | | | | F-1/F-6 | 40/60 |
| Re-37 | C-2 | 10.3 | A-27 | 54.8 | B-4 | 21.8 | D-8 | 11.1 | E-7 | 2 | | | F-1/F-5 | 50/50 |
| Re-38 | C-7 | 29.7 | A-19 | 61.8 | B-12 | 5.1 | D-1 | 3.4 | | | | | F-1/F-3 | 70/30 |
| Re-39 | C-1 | 13.8 | A-5/A-9 | 44/35.5 | | | D-7 | 6.7 | | | | | F-1/F-8 | 85/15 |

(continued)

| Resist composition | Photoacid generator 1 | | Acid decomposable resin | | Photoacid generator 2 | | Acid diffusion control agent | | Hydrophobic resin | | Surfactant | | Solvent | |
|---|---|---|---|---|---|---|---|---|---|---|---|---|---|---|
| | Type | Content (% by mass) | Type | Content (% by mass) | Type | Content (% by mass) | Type | Content (% by mass) | Type | Content (% by mass) | Type | Content (% by mass) | Type | Mixing ratio |
| Re-40 | C-2/C-4 | 8.6/32 | A-14 | 43.9 | | | D-11 | 15.5 | | | | | F-1/F-6 | 40/60 |
| Re-41 | C-12/C-20 | 11.5/12.8 | A-5 | 71.3 | | | D-2 | 3.3 | E-5 | 1.1 | | | F-1/F-2/F-8 | 70/25/5 |
| Re-42 | C-7/C-13 | 31.2/7.5 | A-12 | 52.5 | | | D-5 | 8.8 | | | | | F-1/F-6 | 40/60 |
| Re-43 | C-21 | 3.5 | A-26 | 74.6 | B-2/B-3 | 19.3/2.6 | | | | | | | F-1 | 100 |
| Re-44 | C-3 | 10.1 | A-15 | 76.1 | B-1/B-2 | 10.5/3.3 | | | | | | | F-4 | 100 |
| Re-45 | C-5 | 5.3 | A-11 | 78.1 | B-1/B-12 | 11.3/5.3 | | | | | | | F-1/F-7 | 80/20 |
| Re-46 | C-15 | 28.3 | A-18 | 64.8 | | | D-6/D-9 | 1.3/5.6 | | | | | F-1/F-6 | 40/60 |
| Re-47 | C-19 | 6 | A-2 | 74.9 | B-1 | 18.2 | D-3 | 0.9 | | | | | F-4 | 100 |
| Re-48 | C-11 | 3.5 | A-8 | 73.7 | B-2 | 20.5 | D-8 | 2.3 | | | | | F-1 | 100 |
| Re-49 | Z-1 | 25.5 | A-3 | 68.1 | | | D-7 | 6.4 | | | | | F-1/F-8 | 85/15 |
| Re-50 | Z-2 | 21.4 | A-10 | 70.9 | | | D-10 | 7.7 | | | | | F-1/F-6 | 40/60 |

[Pattern formation (1): Exposure to EUV light, development with aqueous alkali solution]

A composition AL412 (manufactured by Brewer Science) for formation of an underlayer film was applied to a silicon wafer and baked at 205°C for 60 seconds to form an underlayer film having a film thickness of 20 nm. A resist composition shown in Table 5 below was applied to the underlayer film and baked at 100°C for 60 seconds to form a resist film having a film thickness of 30 nm.

An EUV exposure device (Micro Exposure Tool manufactured by Exitech, NA 0.3, Quadrupol, outer sigma: 0.68, inner sigma: 0.36) was used to subject the obtained silicon wafer having the resist film to pattern irradiation. The reticle used was a mask with a line size of 20 nm and a line: space ratio of 1:1.

The resist film exposed to light was baked at 90°C for 60 seconds and developed with an aqueous tetramethylammonium hydroxide solution (2.38% by mass) for 30 seconds and rinsed with pure water for 30 second. The resulting resist film was spin-dried to thereby obtain a positive-type pattern.

<Evaluation of pattern shape: Exposure to EUV light, development with aqueous alkali solution>

The cross-sectional shape of a line pattern having an average line width of 20 nm was observed, and a critical dimension scanning electron microscope (SEM, S-9380II, Hitachi, Ltd.) was used to measure the pattern line width Lb at the bottom of the resist pattern and the pattern line width La in an upper portion of the resist pattern. When $1.00 < (La / Lb) \leq 1.03$, the line pattern was rated "A." When $1.03 < (La / Lb) \leq 1.06$, the line pattern was rated "B." When $1.06 < (La / Lb) \leq 1.10$, the line pattern was rated "C." When $1.10 < (La / Lb) \leq 1.13$, the line pattern was rated "D." When $1.13 < (La / Lb)$, the line pattern was rated "E."

The evaluation results are shown in Table 5.

[Table 5]

| Development with aqueous alkali solution | Resist composition | Pattern shape |
|---|---|---|
| Example 1-1 | Re-1 | A |
| Example 1-2 | Re-2 | A |
| Example 1-3 | Re-3 | A |
| Example 1-4 | Re-4 | A |
| Example 1-5 | Re-5 | A |
| Example 1-6 | Re-6 | A |
| Example 1-7 | Re-7 | A |
| Example 1-8 | Re-8 | A |
| Example 1-9 | Re-9 | A |
| Example 1-10 | Re-10 | A |
| Example 1-11 | Re-11 | A |
| Example 1-12 | Re-12 | A |
| Example 1-13 | Re-13 | A |
| Example 1-14 | Re-14 | A |
| Example 1-15 | Re-15 | A |
| Example 1-16 | Re-16 | A |
| Example 1-17 | Re-17 | A |
| Example 1-18 | Re-18 | B |
| Example 1-19 | Re-19 | B |
| Example 1-20 | Re-20 | B |
| Example 1-21 | Re-21 | B |
| Example 1-22 | Re-22 | B |

(continued)

| Development with aqueous alkali solution | Resist composition | Pattern shape |
|---|---|---|
| Example 1-23 | Re-23 | C |
| Example 1-24 | Re-24 | C |
| Example 1-25 | Re-25 | B |
| Example 1-26 | Re-26 | A |
| Example 1-27 | Re-27 | B |
| Example 1-28 | Re-28 | A |
| Example 1-29 | Re-29 | A |
| Example 1-30 | Re-30 | A |
| Example 1-31 | Re-31 | A |
| Example 1-32 | Re-32 | B |
| Example 1-33 | Re-33 | C |
| Example 1-34 | Re-34 | A |
| Example 1-35 | Re-35 | A |
| Example 1-36 | Re-36 | A |
| Example 1-37 | Re-37 | A |
| Example 1-38 | Re-38 | A |
| Example 1-39 | Re-39 | A |
| Example 1-40 | Re-40 | A |
| Example 1-41 | Re-41 | A |
| Example 1-42 | Re-42 | A |
| Example 1-43 | Re-43 | B |
| Example 1-44 | Re-44 | A |
| Example 1-45 | Re-45 | A |
| Example 1-46 | Re-46 | A |
| Example 1-47 | Re-47 | B |
| Example 1-48 | Re-48 | A |
| Comparative Example 1-1 | Re-49 | D |
| Comparative Example 1-2 | Re-50 | E |

[Pattern formation (2): Exposure to EUV light, development with organic solvent]

A composition AL412 (manufactured by Brewer Science) for formation of an underlayer film was applied to a silicon wafer and baked at 205°C for 60 seconds to form an underlayer film having a film thickness of 20 nm. A resist composition shown in Table 6 below was applied to the underlayer film and baked at 100°C for 60 seconds to form a resist film having a film thickness of 30 nm.

An EUV exposure device (Micro Exposure Tool manufactured by Exitech, NA 0.3, Quadrupol, outer sigma: 0.68, inner sigma: 0.36) was used to subject the obtained silicon wafer having the resist film to pattern irradiation. The reticle used was a mask with a line size of 20 nm and a line: space ratio of 1:1.

The resist film exposed to light was baked at 90°C for 60 seconds and developed with n-butyl acetate for 30 seconds and spin-dried to thereby obtain a negative-type pattern.

<Evaluation of pattern shape: Exposure to EUV light, development with organic solvent>

The cross-sectional shape of a line pattern having an average line width of 20 nm was observed, and a critical dimension scanning electron microscope (SEM, S-9380II, Hitachi, Ltd.) was used to measure the pattern line width Lb at the bottom of the resist pattern and the pattern line width La in an upper portion of the resist pattern. When $1.00 < (Lb / La) \leq 1.03$, the line pattern was rated "A." When $1.03 < (Lb / La) \leq 1.06$, the line pattern was rated "B." When $1.06 < (Lb / La) \leq 1.10$, the line pattern was rated "C." When $1.10 < (Lb / La) \leq 1.13$, the line pattern was rated "D." When $1.13 < (Lb / La)$, the line pattern was rated "E."

The evaluation results are shown in Table 6.

[Table 6]

| Development with organic solvent | Resist composition | Pattern shape |
|---|---|---|
| Example 2-1 | Re-1 | A |
| Example 2-2 | Re-2 | A |
| Example 2-3 | Re-3 | A |
| Example 2-4 | Re-4 | A |
| Example 2-5 | Re-5 | A |
| Example 2-6 | Re-6 | A |
| Example 2-7 | Re-7 | A |
| Example 2-8 | Re-8 | A |
| Example 2-9 | Re-9 | A |
| Example 2-10 | Re-10 | A |
| Example 2-11 | Re-11 | A |
| Example 2-12 | Re-12 | A |
| Example 2-13 | Re-13 | A |
| Example 2-14 | Re-14 | A |
| Example 2-15 | Re-15 | A |
| Example 2-16 | Re-16 | A |
| Example 2-17 | Re-17 | A |
| Example 2-18 | Re-18 | B |
| Example 2-19 | Re-19 | B |
| Example 2-20 | Re-20 | B |
| Example 2-21 | Re-21 | B |
| Example 2-22 | Re-22 | B |
| Example 2-23 | Re-23 | C |
| Example 2-24 | Re-24 | C |
| Example 2-25 | Re-25 | B |
| Example 2-26 | Re-26 | A |
| Example 2-27 | Re-27 | B |
| Example 2-28 | Re-28 | A |
| Example 2-29 | Re-29 | A |
| Example 2-30 | Re-30 | A |
| Example 2-31 | Re-31 | A |

(continued)

| Development with organic solvent | Resist composition | Pattern shape |
|---|---|---|
| Example 2-32 | Re-32 | B |
| Example 2-33 | Re-33 | C |
| Example 2-34 | Re-34 | A |
| Example 2-35 | Re-35 | A |
| Example 2-36 | Re-36 | A |
| Example 2-37 | Re-37 | A |
| Example 2-38 | Re-38 | A |
| Example 2-39 | Re-39 | A |
| Example 2-40 | Re-40 | A |
| Example 2-41 | Re-41 | A |
| Example 2-42 | Re-42 | A |
| Example 2-43 | Re-43 | B |
| Example 2-44 | Re-44 | A |
| Example 2-45 | Re-45 | A |
| Example 2-46 | Re-46 | A |
| Example 2-47 | Re-47 | B |
| Example 2-48 | Re-48 | A |
| Comparative Example 2-1 | Re-49 | D |
| Comparative Example 2-2 | Re-50 | E |

As can be seen from the evaluation results in Tables 5 and 6, when the resist compositions Re-1 to Re-48 in the Examples each including the compound represented by general formula (S1) and an acid decomposable resin were used to form an ultrafine pattern having a line width of 20 nm or less, a pattern having a better shape could be formed irrespective of whether the development was performed using the aqueous alkali solution or the organic solvent. Specifically, in the Examples, the difference between the line width in the upper portion of the resist pattern and the line width in the lower portion was smaller (i.e., the cross section of the pattern had a sharper rectangular shape) than that when the resist compositions Re-49 to Re-50 in the Comparative Examples each including a photoacid generator other than the compound represented by general formula (S1) and an acid decomposable resin were used.

The present invention can provide the actinic ray-sensitive or radiation-sensitive resin composition that can be used for pattern formation to form a pattern having a good shape, the resist film formed using the actinic ray-sensitive or radiation-sensitive resin composition, the pattern forming method, the electronic device manufacturing method, and the compound usable for the actinic ray-sensitive or radiation-sensitive resin composition.

While the invention has been described in detail with reference to the specific embodiments, it will be apparent to those skilled in the art that various changes and modifications can be made without departing from the spirit and scope of the invention.

This application is based on Japanese patent application filed on April 16, 2021 (JP2021-070036) , the entire content of which are incorporated herein by reference.

## Claims

1. An actinic ray-sensitive or radiation-sensitive resin composition comprising: a compound represented by the following general formula (S1); and an acid decomposable resin:

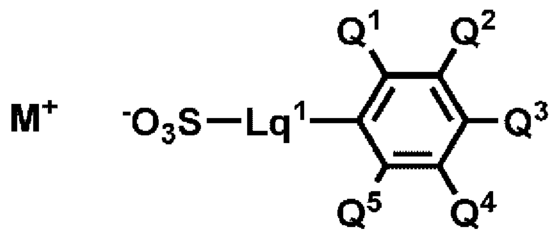

(S1)

wherein, in the general formula (S1), $Q^1$, $Q^2$, $Q^3$, $Q^4$, and $Q^5$ each independently represent a hydrogen atom or a substituent, provided that at least one of $Q^1$, $Q^2$, $Q^3$, $Q^4$, or $Q^5$ represents a substituent including an aryloxy group represented by general formula (QR1) below; $Lq^1$ represents a single bond or a divalent linking group; and $M^+$ represents an organic cation:

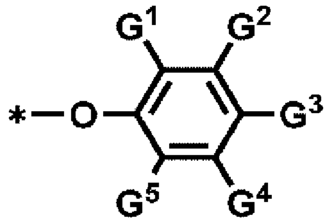

(QR1)

wherein, in the general formula (QR1), $G^1$, $G^2$, $G^3$, $G^4$, and $G^5$ each independently represent a hydrogen atom or a substituent, provided that at least one of $G^1$, $G^2$, $G^3$, $G^4$, or $G^5$ represents a substituent including an ester group; and * represents a bonding position.

**2.** The actinic ray-sensitive or radiation-sensitive resin composition according to claim 1, wherein at least one of $Q^1$, $Q^2$, $Q^3$, $Q^4$, or $Q^5$ in the general formula (S1) represents an electron-withdrawing group

**3.** The actinic ray-sensitive or radiation-sensitive resin composition according to claim 1 or 2, wherein at least one of $Q^1$, $Q^2$, $Q^3$, $Q^4$, or $Q^5$ in the general formula (S1) represents a fluorine atom or a monovalent fluorinated hydrocarbon group.

**4.** The actinic ray-sensitive or radiation-sensitive resin composition according to any one of claims 1 to 3, wherein $Lq^1$ in the general formula (S1) represents a single bond.

**5.** The actinic ray-sensitive or radiation-sensitive resin composition according to any one of claims 1 to 4, wherein at least one of $Q^1$, $Q^2$, $Q^3$, $Q^4$, or $Q^5$ in the general formula (S1) above represents the aryloxy group represented by the general formula (QR1).

**6.** The actinic ray-sensitive or radiation-sensitive resin composition according to claim 5, wherein $Q^3$ in the general formula (S1) represents the aryloxy group represented by the general formula (QR1).

**7.** The actinic ray-sensitive or radiation-sensitive resin composition according to any one of claims 1 to 6, wherein at least two selected from the group consisting of $G^1$, $G^2$, $G^3$, $G^4$, and $G^5$ in the general formula (QR1) each represent the substituent including an ester group.

**8.** The actinic ray-sensitive or radiation-sensitive resin composition according to any one of claims 1 to 7, wherein the substituent including an ester group is a group represented by general formula (GR1) or (GR2) below:

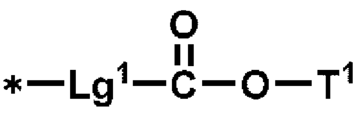

(GR1)

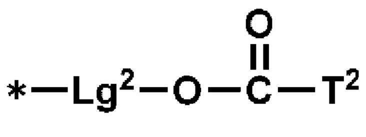

(GR2)

wherein, in the general formulas (GR1) and (GR2), $Lg^1$ and $Lg^2$ each independently represent a single bond or a divalent linking group; $T^1$ and $T^2$ each independently represent an organic group; and * represents a bonding position to a benzene ring in the general formula (QR1).

**9.** The actinic ray-sensitive or radiation-sensitive resin composition according to claim 8, wherein $Lg^1$ in the general formula (GR1) and $Lg^2$ in the general formula (GR2) each represent a single bond.

**10.** The actinic ray-sensitive or radiation-sensitive resin composition according to claim 8 or 9, wherein $T^1$ in the general formula (GR1) and $T^2$ in the general formula (GR2) each independently represent an organic group having 1 to 20 carbon atoms.

**11.** The actinic ray-sensitive or radiation-sensitive resin composition according to any one of claims 8 to 10, wherein $T^1$ in the general formula (GR1) and $T^2$ in the general formula (GR2) each independently represent a chain aliphatic group optionally including a heteroatom or a cyclic aliphatic group optionally including a heteroatom.

**12.** A resist film formed using the actinic ray-sensitive or radiation-sensitive resin composition according to any one of claims 1 to 11.

**13.** A pattern forming method using the resist film according to claim 12.

**14.** A method for manufacturing an electronic device, the method comprising the pattern forming method according to claim 13.

**15.** A compound represented by the following general formula (S1):

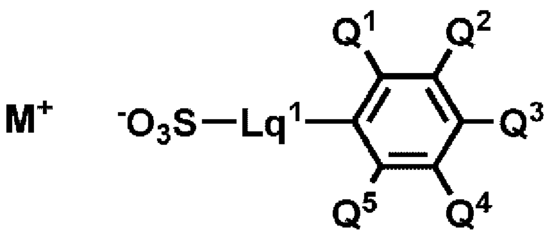

(S1)

wherein, in the general formula (S1), $Q^1$, $Q^2$, $Q^3$, $Q^4$, and $Q^5$ each independently represent a hydrogen atom or a substituent, provided that at least one of $Q^1$, $Q^2$, $Q^3$, $Q^4$, or $Q^5$ represents a substituent including an aryloxy group represented by general formula (QR1) below; $Lq^1$ represents a single bond or a divalent linking group; and $M^+$ represents an organic cation:

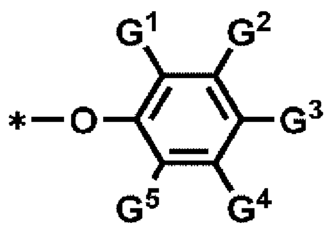

(QR1)

wherein, in the general formula (QR1), $G^1$, $G^2$, $G^3$, $G^4$, and $G^5$ each independently represent a hydrogen atom or a substituent, provided that at least one of $G^1$, $G^2$, $G^3$, $G^4$, or $G^5$ represents a substituent including an ester group; and * represents a bonding position.

**INTERNATIONAL SEARCH REPORT**

International application No.
**PCT/JP2022/017419**

**A. CLASSIFICATION OF SUBJECT MATTER**

***C07C 309/06***(2006.01)i; ***C07C 309/12***(2006.01)i; ***C07C 309/17***(2006.01)i; ***C07C 309/40***(2006.01)i; ***C07C 309/42***(2006.01)i; ***C07C 311/53***(2006.01)i; ***C07C 381/12***(2006.01)i; ***C07D 309/12***(2006.01)i; ***C09K 3/00***(2006.01)i; ***C07D 493/18***(2006.01)i; ***G03F 7/004***(2006.01)i; ***G03F 7/038***(2006.01)i; ***G03F 7/039***(2006.01)i; ***G03F 7/20***(2006.01)i; ***C07D 307/00***(2006.01)i; ***C07D 307/12***(2006.01)i

FI: G03F7/004 503A; G03F7/039 601; G03F7/038 601; C07C309/40; C07C311/53; C07C309/12; C07C309/17; C07C381/12; C07C309/06; C09K3/00 K; G03F7/20 501; G03F7/20 521; C07D309/12; C07D307/00; C07D493/18; C07D307/12; C07C309/42 CSP

According to International Patent Classification (IPC) or to both national classification and IPC

**B. FIELDS SEARCHED**

Minimum documentation searched (classification system followed by classification symbols)

C07C309/06; C07C309/12; C07C309/17; C07C309/40; C07C309/42; C07C311/53; C07C381/12; C07D309/12; C09K3/00; C07D493/18; G03F7/004; G03F7/038; G03F7/039; G03F7/20; C07D307/00; C07D307/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2014-199358 A (FUJIFILM CORP.) 23 October 2014 (2014-10-23) claims, examples | 1-15 |
| X | JP 2014-029481 A (FUJIFILM CORP.) 13 February 2014 (2014-02-13) claims, examples | 15 |
| X | JP 2014-199357 A (FUJIFILM CORP.) 23 October 2014 (2014-10-23) claims, examples | 15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **03 June 2022** | **21 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/017419**

**Box No. III Observations where unity of invention is lacking (Continuation of item 3 of first sheet)**

This International Searching Authority found multiple inventions in this international application, as follows:

(Invention 1) Claims 1-14
The invention in claims 1-14 lacks novelty in light of document 1 and thus does not have special technical features.
Therefore, claims 1-14 are classified as invention 1.

(Invention 2) Claim 15
Claim 15 cannot be said to share the same or corresponding features with claims 1-14 classified as invention 1.
Furthermore, claim 15 does not depend from claim 1 classified as invention 1.
Additionally, claim 15 is not substantially identical to or similarly closely related to any of the claims classified as invention 1.
Therefore, claim 15 cannot be classified as invention 1.
Claim 15 is classified as invention 2.

Document 1: JP 2014-199358 A (FUJIFILM CORP.) 23 October 2014 (2014-10-23), claims, examples
& TW 201447482 A

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2022/017419**

| Patent document cited in search report | Publication date (day/month/year) | Patent family member(s) | Publication date (day/month/year) |
|---|---|---|---|
| JP 2014-199358 A | 23 October 2014 | TW 201447482 A | |
| JP 2014-029481 A | 13 February 2014 | US 2015/0118628 A1<br>claims, examples<br>TW 201410714 A<br>KR 10-2015-0029673 A | |
| JP 2014-199357 A | 23 October 2014 | US 2016/0024005 A1<br>claims, examples<br>TW 201446715 A<br>KR 10-2015-0123896 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013160955 A
- JP 2004109976 A
- JP 2014041327 A
- WO 2018193954 A
- JP 2014098921 A
- WO 2020066824 A
- WO 2020158337 A
- US 20160070167 A
- US 20150004544 A
- US 20160237190 A
- US 20160274458 A
- WO 2020004306 A
- WO 201819395 A
- JP 2014059543 A
- JP 2013061648 A
- JP 2021070036 A


### Non-patent literature cited in the description


- *Materials Letters,* 2008, vol. 62, 3152
- Prediction of polymer properties. Marcel Dekker Inc, 1993